Europäisches Patentamt
European Patent Office
Office européen des brevets

(19) (11) Publication number: **0 367 124 A1**

(12) **EUROPEAN PATENT APPLICATION**

(21) Application number: **89119941.6**

(51) Int. Cl.5: **C07D 499/84 , A61K 31/43**

(22) Date of filing: **27.10.89**

Claims for the following Contracting States: ES + GR

(30) Priority: **31.10.88 US 265161**
**02.10.89 US 416186**

(43) Date of publication of application:
**09.05.90 Bulletin 90/19**

(84) Designated Contracting States:
**AT BE CH DE ES FR GB GR IT LI LU NL SE**

(71) Applicant: **F. HOFFMANN-LA ROCHE AG**
**Postfach 3255**
**CH-4002 Basel(CH)**

(72) Inventor: **Bartkovitz, David Joseph**
**21 Povershon Road**
**Nutley, N.J. 07110(US)**
Inventor: **Keith, Dennis Dalton**
**8 Mendl Terrace**
**Montclair, N.J. 07042(US)**
Inventor: **Mook, Robert**
**Laurel Ridge Apartments No. 84 Highway 54 Pass**
**Chapel Hill, N.C. 27514(US)**
Inventor: **Wei, Chung Chen**
**244 Malapardis Road**
**Cedar Knolls, N.J. 07927(US)**

(74) Representative: **Lederer, Franz, Dr. et al**
**Van der Werth, Lederer & Riederer**
**Patentanwälte Lucile-Grahn-Strasse 22**
**D-8000 München 80(DE)**

(54) **Penicillanic acid derivatives.**

(57) The compounds of the general formula

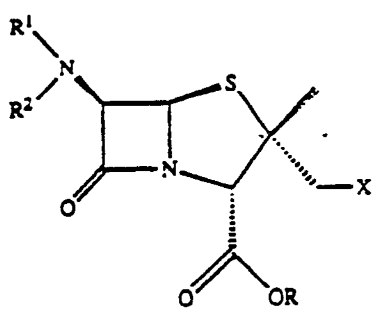

I

in which R is hydrogen, a readily hydrolyzable ester group or a protecting group, $R^1$ is hydrogen and $R^2$ is hydrogen or an acyl group, or $R^1$ and $R^2$ taken together are the group

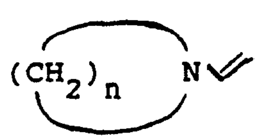

in which n is 5, 6 or 7,

EP 0 367 124 A1

X is fluoro, thiocyanato, azido or the group $R^{3a}CO_2$-

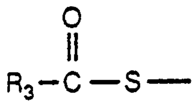 , 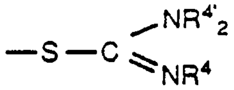 , $-SR^3$ , $-OR^3$

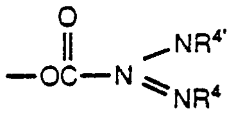 , 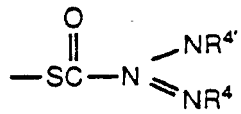 , 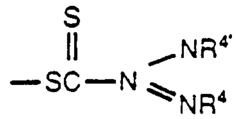 ,

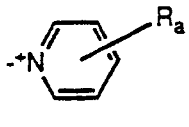 or 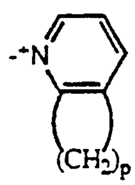 ,

$R^{3a}$ is $C_2$- to $C_{16}$-alkyl, aryl, a heterocycle, -$(CH_2)_m$-aryl, -$(CH_2)_m$-heterocycle, a heterocycle-lower alkenyl or aryl-lower alkenyl, $R^3$ is $C_1$- to $C_{16}$-alkyl, aryl, a heterocycle, -$(CH_2)_m$-aryl, a -$(CH_2)_m$-heterocycle, a heterocycle-lower alkenyl or aryl-lower alkenyl, $R^4$ and $R^{4'}$ independently are hydrogen, lower alkyl, lower alkenyl or lower alkynyl, $R_a$ is hydrogen, halogen, amino, amino-lower alkyl, carboxy, carbamoyl, carboxy-lower alkyl, carbamoyl-lower alkyl or lower carbalkoxy-lower alkyl, m is from 1 to 4 and p is 3, 4 or 5, provided that X is other than fluoro when R is a protecting group, a hydrate, a readily hydrolyzable ester or salt thereof, or a hydrate of the ester or the salt, when R is hydrogen are useful as anti-bacterial agents for the prevention and treatment of bacterial infections in mammals, including humans.

## Penicillanic Acid Derivatives

The present invention is concerned with compounds of the general formula

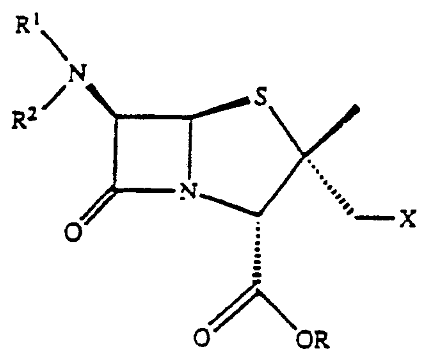

I

in which R is hydrogen, a readily hydrolyzable ester group or a protecting group, $R^1$ is hydrogen and $R^2$ is hydrogen or an acyl group, or $R^1$ and $R^2$ taken together are the group

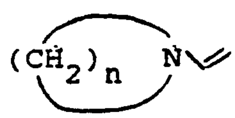

in which n is 5, 6 or 7,
X is fluoro, thiocyanato, azido or the group $R^{3a}CO_2$-,

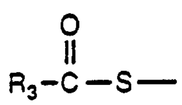 , 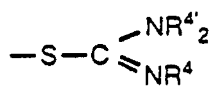 , $-SR^3$ , $-OR^3$

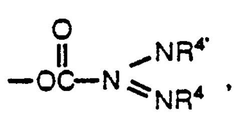 , 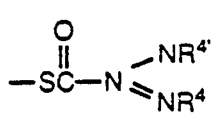 , 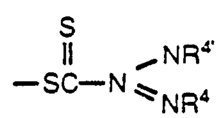 ,

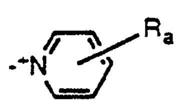 or 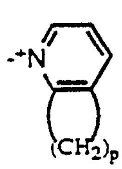 ,

$R^{3a}$ is $C_2$- to $C_{16}$-alkyl, aryl, a heterocycle, -$(CH_2)_m$-aryl, a-$(CH_2)_m$-heterocycle, a hetero cycle-lower alkenyl or aryl-lower alkenyl, $R^3$ is $C_1$- to $C_{16}$-alkyl, aryl, a heterocycle, -$(CH_2)_m$-aryl, a -$(CH_2)_m$-heterocycle, a heterocycle-lower alkenyl or aryl-lower alkenyl, $R^4$ and $R^{4'}$ independently are hydrogen, lower alkyl, lower alkenyl or lower alkynyl, $R_a$ is hydrogen, halogen, amino, amino-lower alkyl, carboxy, carbamoyl, carboxy-lower alkyl, carbamoyl-lower alkyl or lower carbalkoxy-lower alkyl, m is from 1 to 4 and p is 3, 4 or 5, provided that X is other than fluoro when R is a protecting group. Also included, when R is hydrogen, are hydrates, salts of these compounds, as well as hydrates of the salts.

These compounds are useful as anti-bacterial agents for the prevention and treatment of bacterial infections in mammals, including humans.

In the depiction of the compounds of formula I and of the compounds throughout this description, a thickened tapered line ( ◀ ) indicates a substituent in the beta-orientation (that is, above the plane of the molecule or page), and a broken line ( ııı ) indicates a substituent in the alpha-orientation (that is, below the plane of the molecule or page).

As used in this disclosure, the term "lower alkyl" refers to a straight or branched chain saturated hydrocarbon group having from 1 to 8, preferably 1 to 4, carbon atoms, for instance, methyl, ethyl, propyl, isopropyl, tertiary butyl, and the like.

The term "lower alkoxy" refers to a straight or branched chain hydrocarbonoxy group in which the "lower alkyl" portion is a lower alkyl group as defined above, for example, methoxy, ethoxy, propoxy and the like.

The terms "halo", "halogen" and "Hal" are used to represent the four forms chloro, bromo, iodo and fluoro unless specified otherwise.

By the term "aryl" is meant a substituted or unsubstituted aromatic moiety, such as, phenyl, naphthyl and the like, which may have 1 to 3 suitable substituents, such as halo (fluoro, chloro, bromo, etc.), hydroxy, amino, nitro, cyano, carboxy, carbamoyl, lower carbalkoxy, carboxy-lower alkyl, carbamoyl-lower alkyl, lower carbalkoxy-lower alkyl, amino-lower alkyl, lower alkanoyloxy and so forth.

By the term "substituted phenyl" is meant phenyl mono-or di-substituted with halo (chloro, bromo, fluoro, etc.), lower alkyl, amino, nitro or trifluoromethyl.

By the term "substituted alkyl" is meant an alkyl moiety substituted with, for example, halo (chloro, fluoro, bromo, etc.), trifluoromethyl, amino, cyano, and so forth.

By the term "lower alkenyl" is meant a straight or branched chain hydrocarbon group which contains an olefinic double bond having 2 to 8 carbon atoms, for example, allyl, vinyl, and so forth.

By the term "lower cycloalkyl" is meant a 3-8 membered saturated carbocyclic moiety, for example, cyclopropyl, cyclobutyl, cyclohexyl, and so forth.

The term "heterocycle" means rings containing more than one type of atom. Suitable heterocyclic groups are, for example, unsaturated 5 to 8-membered heterocycles containing 1 to 4 nitrogen atoms(s) in the ring (e.g, pyrrole, pyrazole, imidazole, dihydroimidazole, pyridine and tetrazole), unsaturated condensed heterocycles containing 1 to 3 nitrogen atom(s) in the ring (e.g. indole), unsaturated 5 to 8-membered heterocyles containing a sulfur atom in the ring (e.g., thiophene), unsaturated condensed heterocycles containing 1 to 3 sulfur atoms in the ring (e.g. thianthrene), unsaturated 5 to 8-membered heterocycles containing a sulfur atom and 1 to 2 nitrogen atoms in the ring (e.g. thiazole, dihydrothiazole, thiadiazole), unsaturated condensed heterocycles containing a sulfur atom and 1 to 2 nitrogen atoms in the ring (e.g. benzothiazole). unsaturated 5 to 8-membered heterocycles containing an oxygen atom in the ring (e.g. furan), unsaturated condensed heterocycles containing an oxygen atom and 1 to 2 nitrogen atoms in the ring (e.g. benzoxazole), unsaturated 5 to 8-membered heterocyles containing an oxygen atom and 1 to 2 nitrogen atoms in the ring (e.g. oxadiazole), and so forth.

These heterocyclic groups may be unsubstituted or mono-, di- or trisubstituted with lower alkyl, lower carbalkoxy, lower alkoxy, lower alkylthio, hydroxy, mercapto and oxo.

The term "acyl" as used in conjunction with $R^2$ in this disclosure means and includes all organic radicals derived from a carboxylic acid by removal of the hydroxyl group. Although the group $R^2$ may be any one of many acyl radicals, certain acyl groups are preferred.

Exemplary are those acyl groups which have been used in the past to acylate $\beta$-lactam antibiotics, including 6-aminopenicillanic acid and derivatives and 7-aminocephalosporanic acid and derivatives: see, for example, Cephalosporins and Penicillins, edited by Flynn, Academic Press (1972), Belgian patent 866,038, published October 17, 1978, Belgian patent 867,994, published December 11, 1978, U.S. Patent 4,152,432, issued May 1, 1979, U.S. Patent 3,971,778, issued July 27, 1976, and U.S. Patent 4,173,199, issued October 23. 1979. The portions of these references describing various acyl groups are incorporated herein by reference. The following list of acyl groups is presented to further illustrate the term "acyl", but it should not be regarded as all-inclusive. Exemplary acyl groups are:

(a) Aliphatic acyl groups having the formula

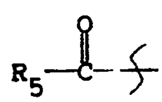

wherein $R_5$ is hydrogen. lower alkyl, lower cycloalkyl, lower alkoxy, lower alkenyl. lower cycloalkenyl. cyclohexadienyl; or lower alkyl or lower alkenyl substituted with one or more halogen, cyano, nitro, amino, mercapto, alkylthio or cyanomethylthio groups.

(b) Aromatic acyl groups having the formula

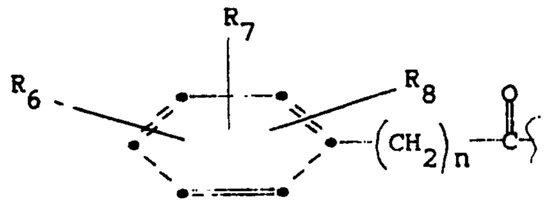

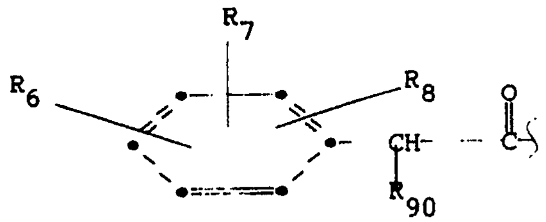

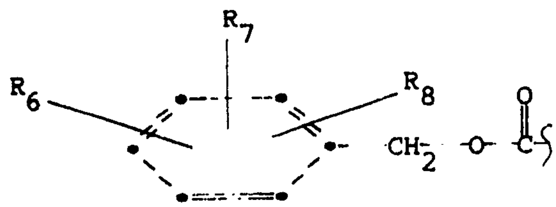

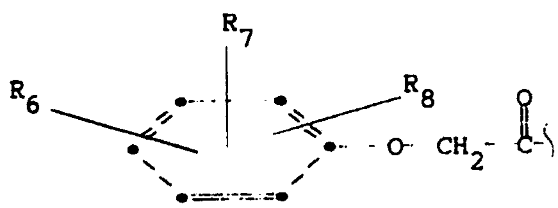

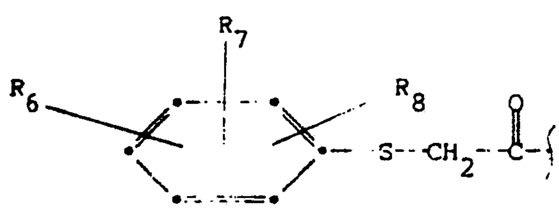

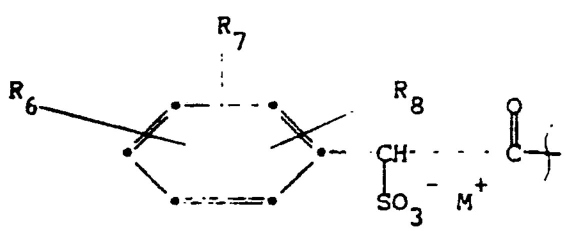

and

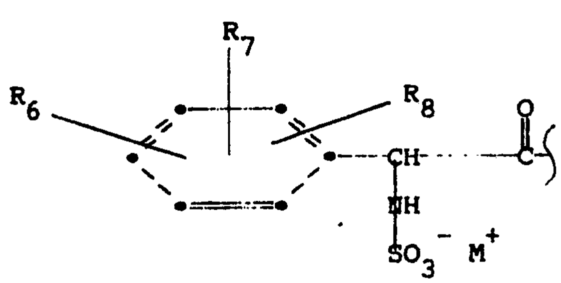

whèrein n is 0, 1, 2 or 3; $R_6$, $R_7$ and $R_8$ each is independently hydrogen, halogen, hydroxy, nitro, amino, cyano, trifluoromethyl, alkyl of 1 to 4 carbon atoms, alkoxy of 1 to 4 carbon atoms or aminomethyl; and $R_{90}$ is amino, acylamino, hydroxy. a carboxy or sulfo salt, protected carboxy such as benzyloxycarbonyl, formyloxy or azido.

Preferred aromatic acyl groups include those having the formula

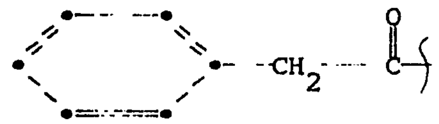

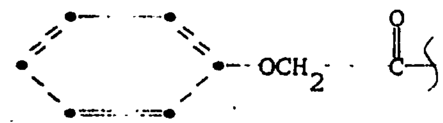

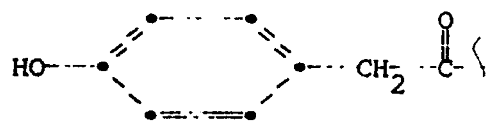

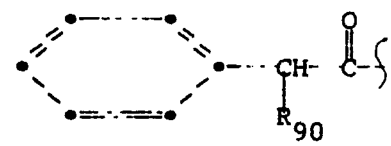

and

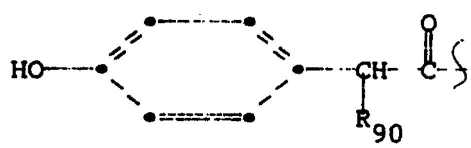

$R_{90}$ is preferably an amino group, a hydroxy group, or a carboxy or sulfo salt.

Examples of other aromatic acyl groups suitable for the purposes of the present invention are sulfophenylacetyl, hydroxysulfonyloxyphenylacetyl, sulfamoylphenylacetyl, (phenoxycarbonyl)phenylacetyl, (p-tolyloxycarbonyl)phenylacetyl, formyloxyphenylacetyl, carboxyphenylacetyl, formylaminophenylacetyl benzyloxycarbonylphenylacetyl, 2-(N,N-dimethylsulfamoyl)-2-phenylacetyl, etc.

(c) Heteroaromatic acyl groups having the formula

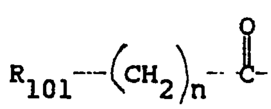

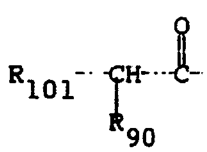

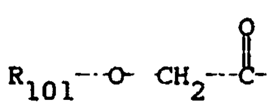

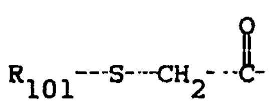

or

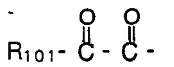

wherein n is 0, 1, 2 or 3; $R_{90}$ is as defined above: and $R_{101}$ is a substituted or unsubstituted 5-, 6- or 7-membered heterocyclic ring containing 1, 2, 3 or 4 (preferably 1 or 2) hetero atoms selected from among nitrogen, oxygen and sulfur. Exemplary heterocyclic rings are thienyl, furyl, pyrrolyl, pyridinyl, pyrazinyl, thiazolyl, pyrimidinyl and tetrazolyl. Exemplary substituents are halogen, hydroxy, nitro, amino, cyano, trifluoromethyl, alkyl of 1 to 4 carbon atoms or alkoxy of 1 to 4 carbon atoms.

Preferred heteroaromatic acyl groups include those groups of the above formulas wherein $R_{101}$ is 2-amino-4-thiazolyl, 2-amino-5-halo-4-thiazolyl, 4-aminopyridin-2-yl, 2-amino-1,3,4-thiadiazol-5-yl, 2-thienyl, 2-furanyl, 4-pyridinyl or 2,6-dichloro-4-pyridinyl.

(d) [[(4-Substituted-2,3-dioxo-1-piperazinyl)carbonyl] amino]acetyl groups having the formula

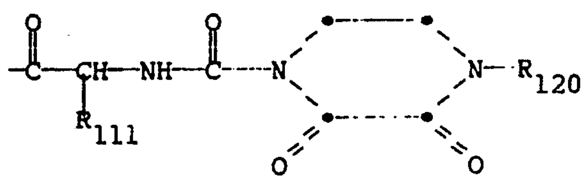

wherein $R_{111}$ is lower alkyl, lower hydroxyalkyl or an aromatic heterocyclic or carbocylic group, such as those of the formula

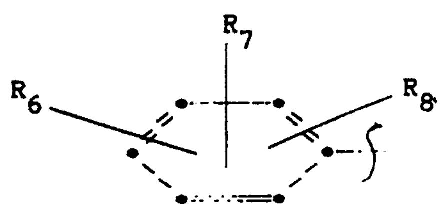

wherein $R_6$, $R_7$ and $R_8$ are as previously defined or the heteroaromatic groups as included within the definition of $R_{101}$; and $R_{120}$ is lower alkyl, substituted lower alkyl (wherein the alkyl group is substituted with one or more halogen, cyano, nitro amino or mercapto groups), e.g., 4-lower alkyl (preferably ethyl or methyl)-2,3-dioxo-1-piperazinecarbonyl-D-phenylglycyl.

(e) (Substituted oxyimino) arylacetyl groups having the formula

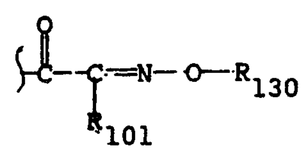

wherein $R_{101}$ is as defined above and $R_{130}$ is hydrogen, lower alkyl or $C_3$-$C_7$-cycloalkyl or substituted lower alkyl [wherein the alkyl group is substituted with one or more halogen, cyano, nitro, amino, mercapto, lower alkylthio, aromatic groups (as defined by $R_{111}$), carboxy (including salts thereof), amido, carbamoyl, lower alkoxycarbonyl, phenylmethoxycarbonyl, diphenylmethoxycarbonyl, hydroxyalkoxyphosphinyl, dihydroxyphosphinyl, hydroxy(phenylmethoxy)phosphinyl or di(lower alkoxy)phosphinyl], or carboxy-$C_3$-$C_7$-cycloalkyl.

Examples of the

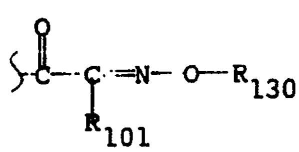

grouping are [2-[(chloroacetyl)amino]-4-thiazolyl](methoxyamino)acetyl, (2-amino-4-thiazolyl)(1-methylethoxyimino) acetyl, (2-amino-4-thiazolyl)(methoxyimino)acetyl, (2-furyl)(methoxyimino)acetyl, (4-hydroxyphenyl)-

(methoxyimino)acetyl, (methoxyimino)(phenyl)acetyl, (hydroxyimino)(phenyl)acetyl, (hydroxyimino)(2-thienyl)acetyl, [[(dichloroacetyl)oxy]imino](2-thienyl)acetyl, [5-chloro-2-[(chloroacetyl)amino]-4-thiazolyl]-(methoxyimino)acetyl, (2-amino-5-chloro-4-thiazolyl)(methoxyimino)acetyl, [[[1-(1,1-dimethylethoxy)-carbonyl]-1-methylethoxy]imino]-2-sulfoamino-4-thiazolyl)acetyl, [[[1-(1,1-dimethylethoxycarbonyl]-1-methylethoxy]imino]-[[2-(triphenylmethyl)amino]-4-thiazolyl]acetyl, (methoxyimino)(2-sulfoamino-4-thiazolyl)-acetyl, [(1-methylethoxy)imino]-[2-[(methylsulfonyl)amino]-4-thiazolyl]acetyl, [(3-methylsulfonyl)-2[3H]-thiazolimin-4-yl]-[1-(methylethoxy)imino]acetyl, [[2-(chloracetyl)amino]-4-thiazolyl] [[[[(4-nitrophenyl)-methoxy]carbonyl]methoxylimino]acetyl, (2-amino-4-thiazolyl)[(carboxymethoxy)imino]acetyl, (2-amino-4-thiazolyl)[1-carboxy-(1-methylethoxy)imino]acetyl, (2-amino-4-thiazolyl)[[(aminocarbonyl)methoxy]imino]-acetyl.

(f) (Acylamino) acetyl groups having the formula

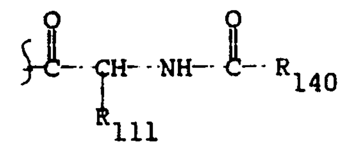

wherein $R_{111}$ is as defined above and $R_{140}$ is an unsaturated heterocycle, a group of the formula

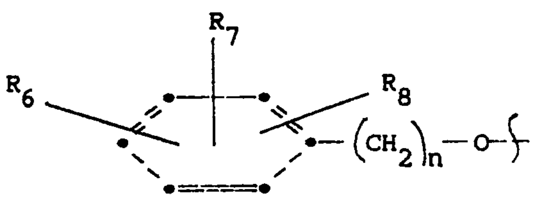

or

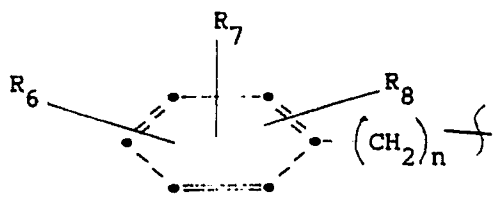

(wherein $R_6$, $R_7$, $R_8$ and n are as previously defined), hydrogen, lower alkyl, substituted lower alkyl, amino, lower alkylamino, di(lower alkyl)amino, (cyano lower alkyl)amino, hydrazino, lower alkyl-hydrazino, aryl-hydrazino or acyl-hydrazino.

Preferred (acylamino)acetyl groups of the above formula include those groups wherein $R_{140}$ is amino or acylamino. Also preferred are those groups wherein $R_{111}$ is phenyl or 2-thienyl.

(g) Substituted oxyimino acetyl groups having the formula

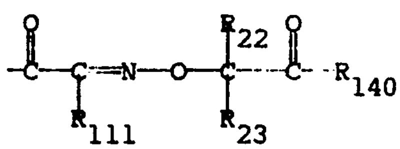

wherein $R_{111}$ and $R_{140}$ are as defined above, and $R_{22}$ and $R_{23}$ are independently selected from the group consisting of hydrogen and lower alkyl, or $R_{22}$ and $R_{23}$ taken together with the carbon atom to which they are attached form a $C_3$-$C_7$-carbocyclic ring, for example, cyclopropyl, cyclobutyl or cyclopentyl.

Preferred substituted oxyimino acetyl groups of the above formula include those groups wherein $R_{140}$ is hydroxy or amino. Also preferred are those groups wherein $R_{111}$ is 2-amino-4-thiazolyl.

(h) [[[3-Substituted-2-oxo-1-imidazolidinyl]carbonyl]amino]acetyl groups having the formula

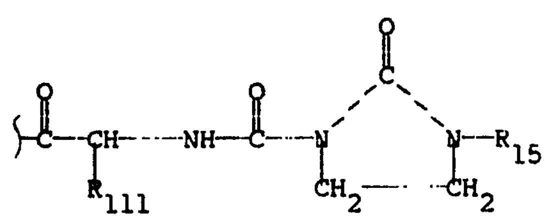

wherein $R_{111}$ is as defined above and $R_{15}$ is hydrogen, lower alkylsulfonyl, arylmethyleneamino (i.e., $-N=CHR_{111}$ where in $R_{111}$ is as defined above), $-COR_{16}$ (wherein $R_{16}$ is hydrogen, lower alkyl or halogen

substituted lower alkyl), an aromatic group (as defined by $R_{111}$ above), lower alkyl or substituted lower alkyl (wherein the lower alkyl group is substituted with one or more halogen, cyano, nitro, amino or mercapto groups).

Preferred [[[3-substituted-2-oxo-1-imidazolidinyl]carbonyl]amino]acetyl groups of the above formula include those wherein $R_{111}$ is phenyl or 2-thienyl. Also preferred are those groups wherein $R_{15}$ is hydrogen, methylsulfonyl, phenylmethyleneamino or 2-furylmethyleneamino.

Preferred compounds of formula I are those wherein X is $R^{3a}CO_2$-,

$$R^3\text{--}\overset{\overset{O}{\|}}{C}\text{--}S\text{--},\ -SR^3 \text{ and } -OR^3.$$

Especially preferred compounds are those wherein $R^{3a}$ and $R^3$ are independently aryl or a heterocycle.

As readily hydrolyzable esters of the compounds of formula I there are to be understood compounds of formula I, the carboxy group(s) of which (i.e., the 2-carboxy group) is/are present in the form of readily hydrolyzable ester groups. Examples of such esters, which can be of the conventional type, are the lower alkanoyloxyalkyl esters (e.g., the acetoxymethyl, pivaloyloxymethyl, 1-acetoxymethyl and 1-pivaloyloxyethyl ester), the lower alkoxycarbonyloxyalkyl esters (e.g., the methoxycarbonyloxymethyl, 1-ethoxycarbonyloxyethyl and 1-isopropoxycarbonyloxyethyl ester), the lactonyl esters (e.g., the phthalidyl and thiophthalidyl ester), the lower alkoxymethyl esters (e.g., the methoxymethyl ester) and the lower alkanoylaminomethyl esters (e.g., the acetamidomethyl ester). Other esters (e.g., the benzyl and cyanomethyl esters) can also be used.

Examples of salts of the compounds of formula I are alkali metal salts such as the sodium and potassium salt, the ammonium salt, alkaline earth metal salts such as the calcium salt, salts with organic bases such as salts with amines (e.g., salts with N-ethyl-piperidine, procaine, dibenzylamine, N,N′-dibenzylethylenediamine, alkylamines or dialkylamines) as well as salts with amino acids such as, for example, salts with arginine or lysine. The salts can be mono-salts, di-salts or tri-salts.

The compounds of formula I also form addition salts with organic or inorganic acids. Illustrative of such salts are hydrohalides (for example, hydrochlorides, hydrobromides and hydroiodides), as well as other mineral acid salts such as sulfates, nitrates, phosphates and the like, alkylsulfonates and monoarylsulfonates such as ethanesulfonates, toluenesulfonates, benzenesulfonates and the like, and also other organic acid salts such as acetates, tartrates, maleates, citrates, benzoates, salicylates, ascorbates and the like.

The compounds of formula I, their salts and esters and hydrates of those compounds are useful as agents to combat bacterial infections (including urinary and respiratory tract infections) in mammals, for example, dogs, cats, horses, and humans. These compounds exhibit activity against a broad range of Gram-negative bacteria.

The *in vitro* activity of compounds of the present invention as measured by the Minimum Inhibitory Concentration (MIC) in micrograms per milliliter (mcg/ml), utilizing the Agar Well Diffusion Method, against a variety of Gram-negative microorganisms is illustrated in the following Table, in which the compounds evaluated were as follows:

Compound A: [2S-[2α,3α,5α,6β(R*)]]-6-[[[[(4-Ethyl-2,3-dioxo-1-piperazinyl)carbonyl]amino]phenylacetyl]amino]-3-methyl-3-[[(1-methyl-1H-tetrazole-5-yl)thio]methyl]-7-oxo-4-thia-1-azabicyclo[3.2.0]heptane-2-carboxylic acid monosodium salt sesquihydrate

Compound B: [2S-(2α,3α,5α,6β)]-3-Methyl-3-[[(1-methyl-1H-tetrazol-5-yl)-thio]methyl-7-oxo-6-[(phenylacetyl)amino]-4-thia-1-azabicyclo[3.2.0] heptane-2-carboxylic acid monosodium salt

Compound C: [2S-[2α,3α,5α,6β(Z)]]-6-[[(2-Amino-4-thiazolyl)-(methoxyimino)-acetyl]amino]-3-methyl-3-[[(1-methyl-1H-tetrazol-5-yl)thio]methyl]-7-oxo-4-thia-1-azabicyclo[3.2.0]heptane-2-carboxylic acid monosodium salt

Compound D: [2S-(2α,3α,5α,6β)]-3-(Azidomethyl)-3-methyl-7-oxo-6-[(phenylacetyl)amino]-4-thia-1-azabicyclo-[3.2.0]heptane-2-carboxylic acid monosodium salt

Compound E: [2S-(2α,3α,5α,6β)]-1-[[2-carboxy-3-methyl-7-oxo-6-[(phenylacetyl)amino]-4-thia-1-azabicyclo-[3.2.0]hept-3-yl]methylpyridinium hydroxide inner salt

Compound F: [2S-(2α,3α,5α,6β)]-3-[[(2,5-Dihydro-6-hydroxy-2-methyl-5-oxo-1,2,4-triazine-3-yl)thio]-methyl]-3-methyl-7-oxo-6-[(phenylacetyl)amino]-4-thia-1-azabicyclo[3.2.0]heptane-2-carboxylic acid disodium salt

Compound G: [2S-(2α,3α,5α,6β)]-3-[(Acetylthio)methyl]-3-methyl-7-oxo-6-[(phenylacetyl)amino]-4-thia-1-azabicyclo[3.2.0]heptane-2-carboxylic acid monosodium salt

Compound H: [2S-(2α,3α,5α,6β)]-3-[[[3,4-bis(Acetyloxy)benzoyl]oxy]methyl]-3-methyl-7-oxo-6-[(phenylacetyl)amino]-4-thia-1-azabicyclo[3.2.0]heptane-2-carboxylic acid monosodium salt

In Vitro MIC (mcg/ml)

| Culture | Compounds A | B | C | D | E | F | G | H |
|---|---|---|---|---|---|---|---|---|
| Pseudomonas aeruginosa 56 ATCC 8079 | 15.6 | 62.5 | 62.5 | >125 | 125 | 31.5 | 125 | 31.5 |
| Pseudomonas aeruginosa 56M | 0.49 | 0.98 | 0.98 | 3.9 | 3.9 | 0.49 | 0.98 | 1.95 |
| Pseudomonas maltophilia 28978 ATCC 17445 | 250 | >125 | >500 | >125 | | | >500 | 31.5 |
| Proteus vulgaris 101N ATCC 6380 | 0.98 | 15.6 | 31.3 | 15.6 | 125 | 7.8 | 31.3 | 62.5 |
| Proteus vulgaris 100 | 7.8 | >125 | 250 | >125 | >500 | 250 | >500 | >500 |
| Escherichia coli 1269B | 62.5 | >125 | >500 | >125 | 500 | 500 | | |
| Escherichia coli 2721B | 3.9 | 62.5 | 31.3 | 62.5 | 31.3 | 31.3 | 62.5 | 500 |
| Escherichia coli 2722B | 0.12 | 31.3 | 1.95 | 15.6 | 31.3 | 3.9 | 7.8 | 62.5 |
| Escherichia coli 3081B | 15.6 | >125 | >125 | 125 | 125 | 125 | 500 | >500 |
| Escherichia coli 3082B | 0.24 | 31.3 | 1.95 | 7.8 | 31.3 | 0.98 | 1.95 | 15.6 |

In Vitro MIC (mcg/ml)

(cont.'d)

Compounds

| Culture | A | B | C | D | E | F | G | H |
|---|---|---|---|---|---|---|---|---|
| Streptococcus faecium ATCC 8043 | 31.3 | 7.8 | 125 | 7.8 | 125 | 62.5 | 0.98 | 7.8 |
| Staphylococcus aureus 82 ATCC 6538P | 0.98 | 0.03 | 0.49 | 0.03 | 0.49 | 0.24 | 0.03 | 0.12 |
| Staphylococcus aureus 1059B | 7.8 | 3.9 | 3.9 | 7.8 | 15.6 | 0.24 | 0.98 | 7.8 |
| Staphylococcus aureus 3037B | 500 | 125 | 250 | 125 | 62.5 | 500 | 250 | 125 |
| Staphylococcus aureus 3647B | 500 | 125 | 250 | 125 | 500 | 500 | 250 | 500 |
| Micrococcus luteus PCI ATCC 9341 | 0.49 | 0.03 | 0.24 | 0.03 | 1.95 | 0.24 | 0.06 | 0.12 |
| Bacillus megaterium 164 ATCC 8011 | 0.12 | 0.015 | 0.12 | 0.12 | 0.49 | 0.12 | | |
| Bacillus subtilus 558 NRRL 558 | 0.98 | 0.03 | 0.49 | 0.06 | 0.24 | 0.12 | 0.03 | 0.12 |

For combatting bacterial infections in mammals, a compound of this invention can be administered to a mammal in an amount from about 5 milligrams per kilogram of body weight per day (mg/mg/day) to about 100 mg/kg/day, and most preferably from about 10 mg/kg/day to about 55 mg/kg/day.

Virtually any mode of administration employed conventionally in the past to deliver penicillin and cephalosporin antibiotics to the site of infection are also contemplated for use with the compounds of this invention. Such modes of administration include intravenous, intramuscular, subcutaneous and enterally, for example, as a suppository.

It may be understood that a pharmaceutical composition including a compound of formula I contains a pharmaceutically acceptable carrier, such as those carriers known in the art.

The following Reaction Schemes set forth methods and intermediates useful in producing the end product compounds of formula I of this invention.

In these reaction sequences, where a substituent is present in the molecule which may be chemically attacked during the reaction, it should be present in a protected form utilizing a protecting group. Protecting groups suitable for this purpose may be selected from among conventional materials well known to those skilled in the art. By way of illustration, amino groups can be protected using easily removable protecting groups employed in peptide chemistry, such as an alkylcarbonyl groups, for example, formyl, acetyl, propionyl, and so forth, an alkoxycarbonyl group, for example, t-butoxycarbonyl, and so forth, an alkoxyalkylcarbonyl group, for example, methoxyacetyl, methoxypropionyl, and so forth, a substituted alkoxycarbonyl group. for example, trichloroethoxycarbonyl, and so forth, a substituted alkylcarbonyl group, for example, monochloromethylcarbonyl, monochloroethylcarbonyl, dichloromethyl carbonyl, dichloroethylcarbonyl, trichloromethylcarbonyl, trichloroethylcarbonyl, trichloropropylcarbonyl, and so forth, an aralkyloxycarbonyl group, for example, benzyloxycarbonyl, and so forth, a substituted aralkyloxycarbonyl group, for example, p-nitrobenzyloxycarbonyl, and so forth, or an amino group which is protected with a protein.

Preferred as the amino-protecting group for purposes of this invention are benzyloxycarbonyl. t-butyloxycarbonyl or a silyl protecting group. such as trimethylsilyl.

The removal of the amino-protecting group is accomplished conventionally, for instance, by acid treatment in the case of t-butoxycarbonyl, by catalytic reduction in the case of p-nitrobenzyloxycarbonyl, or by treatment with zinc and acid in the case of trichloroethoxycarbonyl.

As protecting groups for carboxylic acid groups in the molecule, one may utilize an ester group which is later readily convertible to the carboxylic acid group upon mild treatment with an acid or alkali or by reduction. Illustrative of such carboxylic acid protecting groups are beta-methylsulfonylethyl, trimethylsilyl, t-butyldimethylsilyl, benzhydryl, β-β-β-trichloroethyl, phenacyl, p-methoxybenzyl, p-nitrobenzyl, methoxymethyl, and so forth.

In these Reaction Schemes, $R'$ designates a carboxylic acid-protecting group (such as exemplified above), Ph means a phenyl group, M designates a metal cation (for example, a sodium or potassium ion), and $R^1$, $R^2$, X and Hal are as defined previously.

Scheme 1

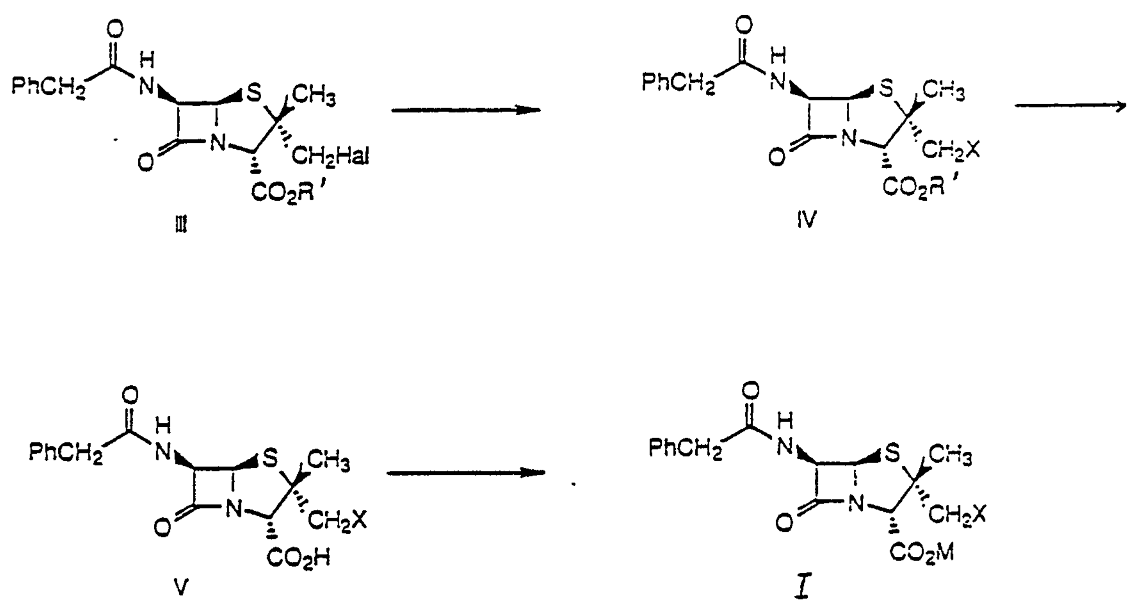

III IV V I

Scheme 1

III --------> IV

The compound of formula III, which is known and can be made by described methods, is reacted with a salt of the chosen nucleophile. The reaction is carried out in any suitable solvent. Suitable salts of the nucleophile are, for instance, sodium, potassium, cesium, silver, or tetrabutylammonium. The preferred halogen is bromine. The reaction is run at about -10° C to 80° C, with room temperature (e.g., 23-25° C) being preferred.

IV --------> V

The compound of formula IV is thereafter deprotected to obtain the desired product of formula V using reagents compatible with the ester protecting group utilized. The following reagents and corresponding compatible ester can be utilized: para-nitrobenzyl removed by hydrogenolysis with palladium on carbon or by hydrolysis in the presence of sodium sulfide at about or below 0 °C to room temperature in a solvent such as dimethylformamide (aqueous); t-butyl or diphenylmethyl ester removed by reaction with trifluoroacetic acid in the presence of anisole at about 0 °C to room temperature with or without a co-solvent such as methylene chloride; or allyl esters removed by a palladium-catalyzed transallylation reaction in the presence of the sodium or potassium salt of 2-ethyl hexanoic acid; See, for example. J. Org. Chem. 47,587 (1982).

V --------> I

The compound of formula V is converted to the salt of formula I with an alkali metal hydroxide, carbonate or bicarbonate. The preferred reagents are sodium or potassium bicarbonate. This conversion can be performed in a solvent such as water or a mixture of water and an organic solvent.

Scheme 2

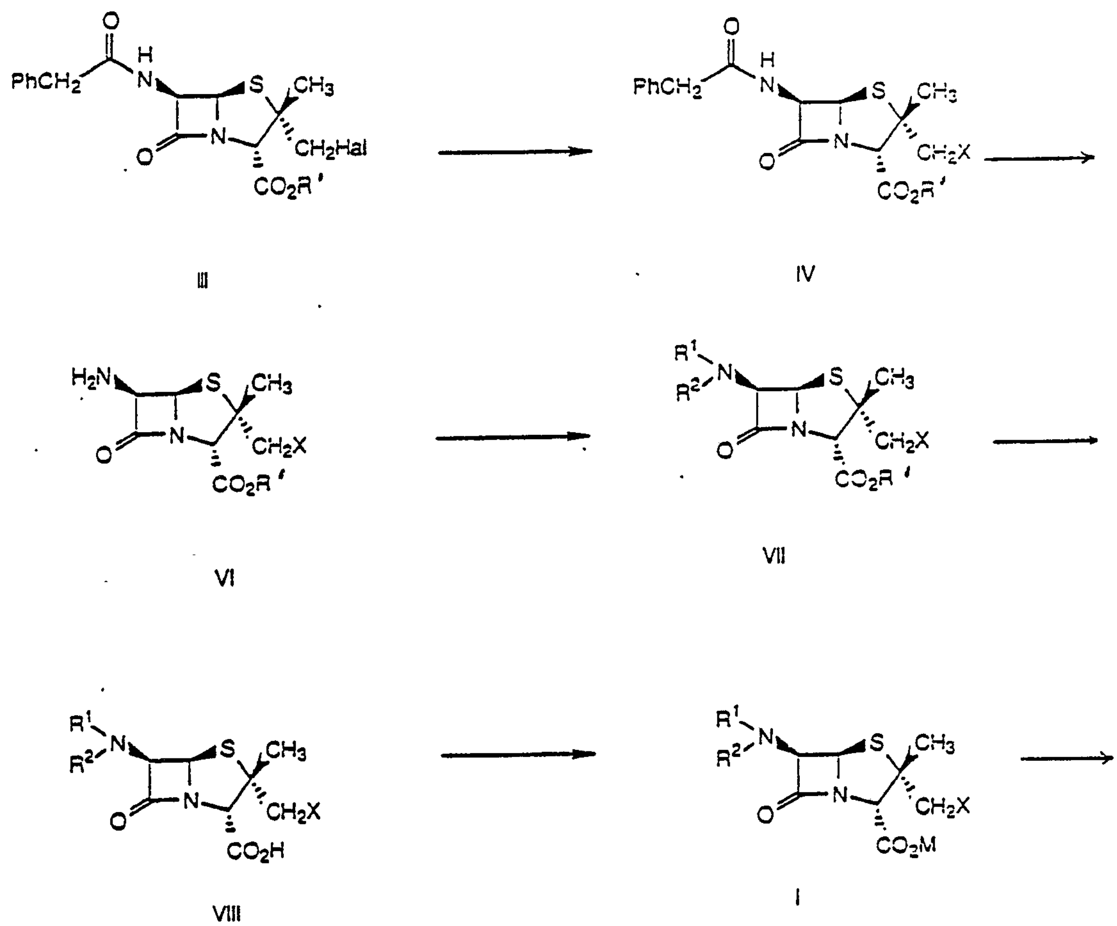

III IV

VI VII

VIII I

Scheme 2

III --------> IV

The conversion of the compound of formula III to the compound of formula IV is effected using the same conditions described for Reaction Scheme 1.

IV --------> VI

The compound of formula IV is converted to VI by reacting with $PCl_5$ or $PBr_5$ and pyridine or lutidine at a temperature of about -20° to 40° C, preferably -10° C. A chlorinated solvent is employed, such as $CH_2Cl_2$, $CHCl_3$ or $CH_2ClCH_2Cl$. Subsequent reaction with an alcohol, preferably n-propanol or isopropanol and water gives, after basification, the compound of formula VI.

VI --------> VII

The amino group in the compound of formula VI is acylated by reaction with an activated carboxylic acid, according to methods known in the art, to introduce $R^2$ ($R^1$ = H) and obtain the compound of formula VII. For example, utilizing a solvent such as methylene chloride, ethyl acetate or dimethylformamide compound VI is reacted with an acylating agent of the formula

$R_5-\overset{O}{\overset{\|}{C}}-Z$, in which Z is an acyl activating group. Preferred for activating group Z are halogen,

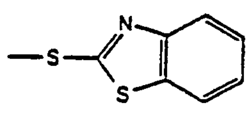

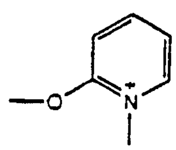

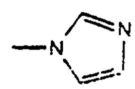

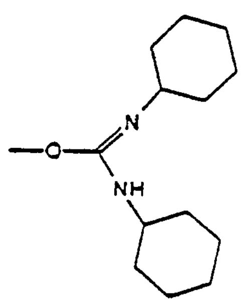

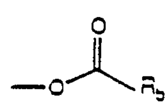

Reactions are carried out at about 0° to about 30° C, for about 2 to 24 hours. If $R^2$ itself is introduced in a form which contains protected functionalities, the protecting groups are subsequently removed by appropriate methods known in the art.

The compound of formula VII where $R^1$ and $R^2$ taken together are

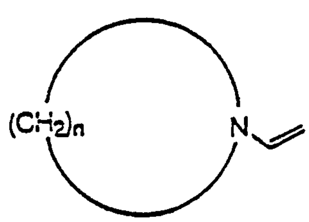

is obtained by reaction with the following:

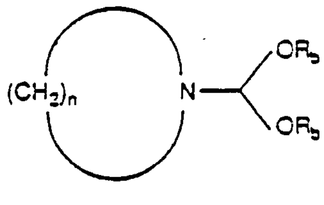

or

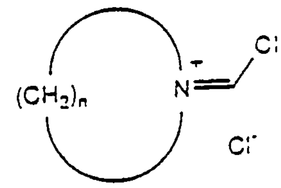

in a chlorinated hydrocarbon such as methylene chloride, at a temperature of about -20° to 40° C.

VII ---------> VIII --------> I

The conversion of the compound of formula VII to VIII and VIII to the compound of formula 1 is effected using the same conditions described in Reaction Scheme 1 for the conversion of IV to V and V to I.

Scheme 3

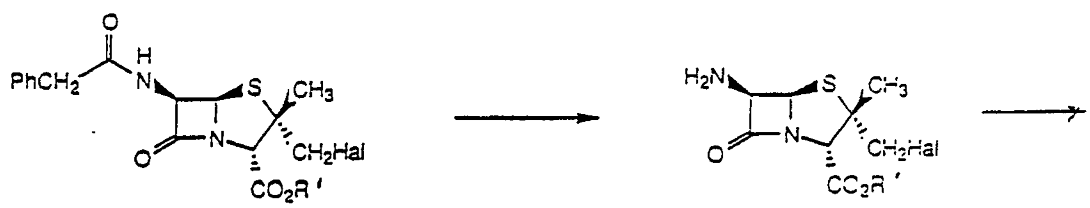

III IX

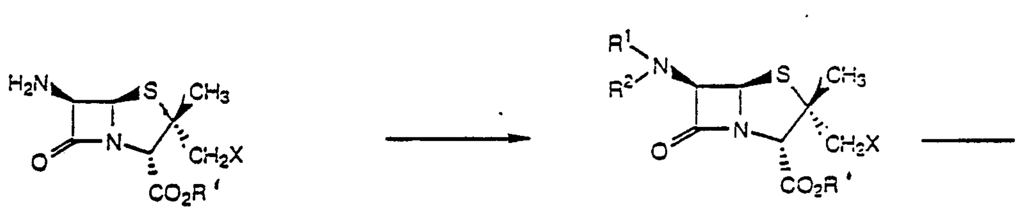

VI VII

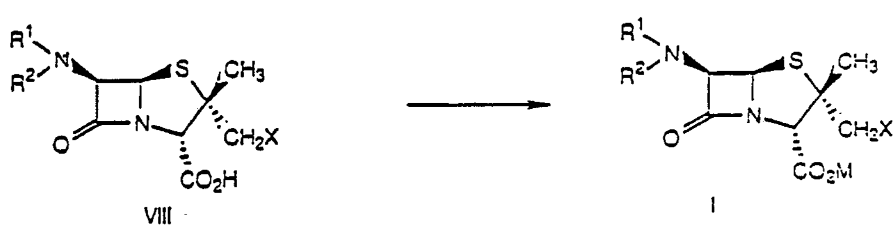

VIII I

Scheme 3

III --------> IX

The reaction conditions are the same as described in Reaction Scheme 2 for the conversion of IV to VI.

IX --------> VI

The reaction conditions are the same as described in Reaction Scheme 1 for the conversion of III to IV.

VI --------> VII

The reaction conditions are the same as described in Scheme 2 for the conversion of VI to VII.

VII ---------> VIII ----------> I

The reaction conditions are the same as described in Scheme 2 for the conversions of VII to VIII and VIII to I.

Scheme 4

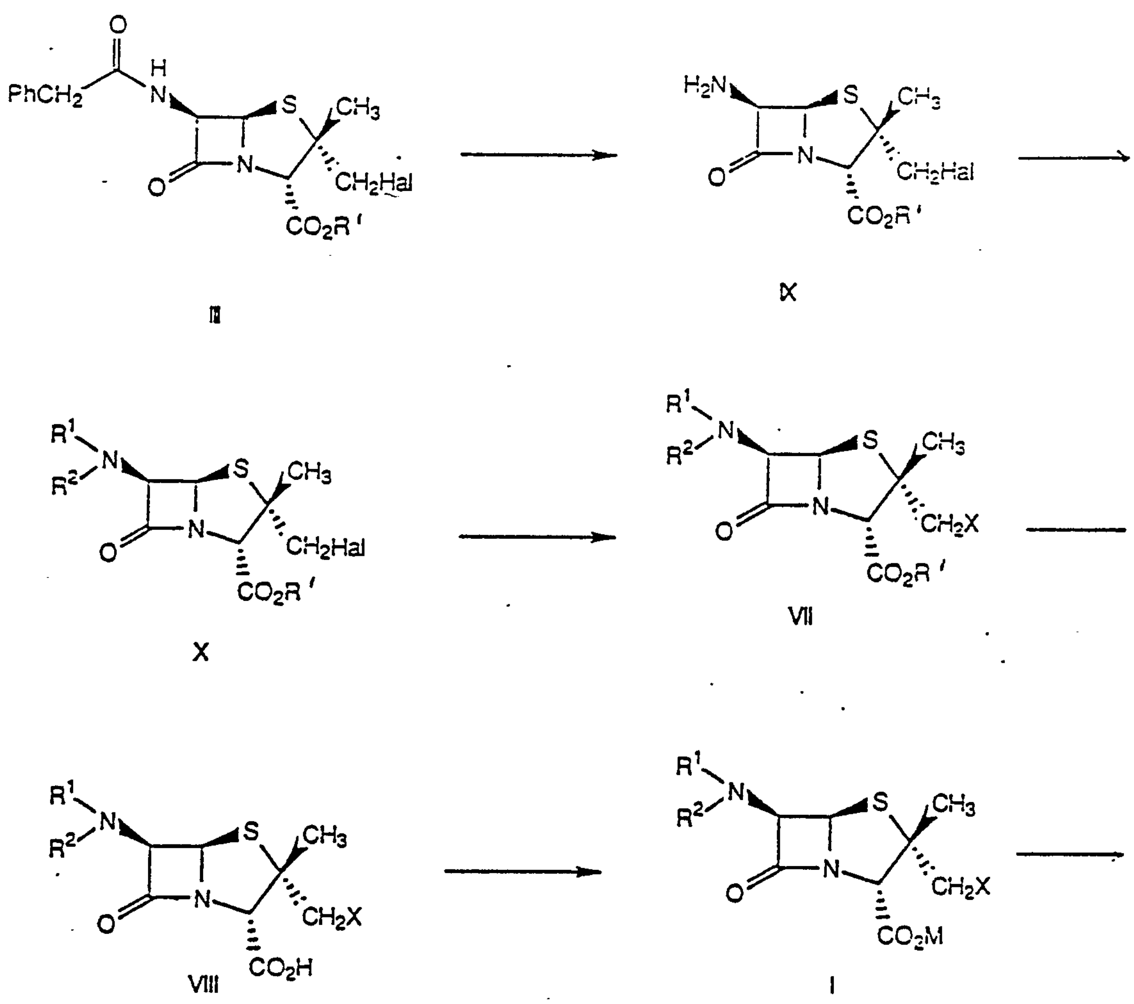

III IX X VII VIII I

Scheme 4

III --------> IX

The reaction conditions are the same as described in Reaction Scheme 2 for the conversion of IV to VI.

IX --------> X

The reaction conditions are the same as described in Reaction Scheme 2 for the conversion of VI to VII.

X --------> VII

The reaction conditions are the same as described in Reaction Scheme 1 for the conversion of III to IV.

VII ---------> VIII ----------> I

The reaction conditions are the same as described in Scheme 2 for the conversions of VII to VIII and VIII to I.

In order to prepare the easily hydrolyzable esters of the carboxylic acids of the formula I compound, the carboxylic acid is preferably reacted with an appropriate halide containing the ester group, and especially with the iodide. The reaction can be accelerated with the aid of a base, such as an alkali metal hydroxide or carbonate, or an organic amine, for example, triethylamine. The esterification reaction is usually carried out in an inert organic solvent, such as dimethylacetamide, hexamethylphosphoric acid bromide, dimethylsulfoxide or dimethylformamide, preferably the latter. The temperature is preferably in the range of about 0°-40° C.

The salts and hydrates of the compounds of formula I, or the hydrates of these salts, can be prepared in a known manner, such as by reacting the carboxylic acid of formula I with an equivalent amount of the desired base, appropriately in an aqueous or non-aqueous solvent, such as water, ethanol, methanol or acetone. The temperature of the salt formation is generally room temperature (e.g., about 23° C) but can also be above or below that, for example, in the range from 0° C to +50° C.

The formation of the hydrates usually takes place automatically during the course of the preparation process or in any event, as a result of the hydrogscopic properties of an initially anhydrous product. For the controlled preparation of a hydrate, a completely or partially anhydrous (carboxylic acid of the formula I, or ester, or salt thereof) can be subjected to a moist atmosphere at a temperature from about 10° C to +40° C.

In the following Examples, the stereochemical configurations are set forth in either alpha ("α") or beta ("β") designations in the chemical names for the compounds.

## Example 1

### [2S-(2α,3α,5α,6β)]-3-Methyl-3-[[(1-methyl-1H-tetrazol-5-yl)-thio]methyl]-7-oxo-6-[(phenylacetyl)amino]-4-thia-1-azabicyclo[3.2.0]heptane-2-carboxylic acid (4-nitrophenyl)methyl ester

[2S-(2α,3α,5α,6β)]-3-(Bromomethyl-3-methyl-7-oxo-6-[(phenylacetyl)amino]-4-thia-1-azabicyclo[3.2.0]-heptane-2-carboxylic acid (4-nitrophenyl)-methyl ester (201.1 mg, 0.367 mmol) and 5-mercapto-1-methyl tetrazole, sodium salt (79.1 mg, 0.573 mmol) were dissolved in acetonitrile (2.5 ml) and stirred at ambient temperature for 2 1/2 hours. The mixture was then concentrated and purified by flash chromatography, eluting with 40% and 50% ethyl acetate in hexane, which gave an amorphous solid product (87.8 mg, 0.150 mmol, 41.0% yield).

## Example 2

[2S-(2α,3α,5α,6β)]-3-Methyl-3-[[(1-methyl-1H-tetrazol-5-yl)-thio]methyl-7-oxo-6-[(phenylacetyl)amino]-4-thia-1-azabicyclo[3.2.0]heptane-2-carboxylic acid monosodium salt

[2S-(2α,3α,5α,6β)]-3-Methyl-3-[[(1-methyl-1H-tetrazol-5-yl)thio]methyl]-7-oxo-6-[(phenylacetyl)amino]-4-thia-1-azabicyclo[3.2.0]heptane-2-carboxylic acid (4-nitrophenyl)methyl ester (131.8 mg, 0.226 mmol) was dissolved in tetrahydrofuran (11.1 ml) containing ethanol (0.25 ml). The catalyst, 10% Pd/C (268.9 mg), was added and the mixture was stirred for 3 hours at ambient temperature under hydrogen. The reaction was filtered through celite and the filtrate was concentrated. The crude residue (131.8 mg) was dissolved in tetrahydrofuran (0.7 ml) and ethylacetate (2 ml). To this solution sodium bicarbonate (38.1 mg, 0.453 mmol) and water (4 ml) were added. The water layer was purified by HPLC, eluting with acetonitrile in water. After lyophilizing, a white amorphous solid was obtained, which weighed 26.9 mg (0.0572 mmol, 22.7% yield).

## Example 3

[2S-(2α,3α,5α,6β)]-3-[(Acetyloxy)methyl]-3-methyl-7-oxo-6-[(phenylacetyl)-amino]-4-thia-1-azabicyclo [3.2.0] heptane-2-carboxylic acid (4-nitrophenyl)methyl ester

Potassium acetate (337 mg. 3.434 mmol) was dissolved in ethanol (9.5 ml) and added to [2S-(2α,3α,5α,6β)]-3-(bromomethyl)-3-methyl-7-oxo-6-[(phenylacetyl)amino]-4-thia-1-azabicyclo[3.2.0]heptane-2-carboxylic acid 4-(nitrophenyl) methyl ester (500.4 mg. 0.912 mmol). The reaction mixture was stirred at ambient temperature. After 2 hours and 25 minutes, a large amount of orange-yellow solid was still present. The supernatant was removed by pipet, diluted with ethyl acetate (90 ml) and extracted with saturated sodium bicarbonate (100 ml) then brine (100 ml). The ethyl acetate was dried over $Na_2SO_4$. The solid was dissolved in methylene chloride (1 ml) and ethanol (9.5 ml) containing potassium acetate (337 mg, 3.434 mmol). After 1 hour and 15 minutes, ethanol was evaporated and the residue was dissolved in ethyl acetate (100 ml) and washed with saturated $NaHCO_3$, then brine (100 ml). The ethyl acetate was dried over $Na_2SO_4$. Both ethyl acetate extracts were filtered and concentrated. The first weighed 80 mg and the second weighed 360 mg. They were combined and purified by flash chromatography, total product weight 117 mg (0.222 mmol, 24.3 % yield).

## Example 4

[2S-(2α,3α,5α,6β)]-3-[[2,5-Dihydro-6-hydroxy-2-methyl-5-oxo-1,2,4-triazine-3-yl)thio]methyl]-3-methyl-7-oxo-6-[(phenylacetyl)amino]-4-thia-1-azabicyclo[3.2.0]heptane-2-carboxylic acid (4-nitrophenyl)methyl ester monosodium salt

To a solution of [2S-(2α,3α,5α,6β)]-3-(bromomethyl)-3-methyl-7-oxo-6-[(phenylacetyl)amino]-4-thia-1-azabicyclo[3.2.0]heptane-2-carboxylic acid (4-nitrophenyl)methyl ester (0.255 g, 0.47 mmol) in acetone (15 ml) and water (3 ml) was added the disodium salt of tetrahydro-6-hydroxy-2-methyl-5-oxo-3-thioxo-1,2,4-triazine (0.106 g, 0.52 mmol) at room temperature, overnight. The acetone was removed in vacuo and the aqueous layer was lyophilized. The residue was taken up in water and applied to a reverse phase C18 Sep-Pak ((Waters Assoc.) and eluted with $CH_3CN/H_2O$ (0 - 40%, product elutes with 10% $CH_3CN/H_2O$.Rf($SiO_2$) = 0.3 60:3:1 ethyl acetate/acetic acid/water). The proper fractions were combined, the acetonitrile was removed in vacuo, and the aqueous phase lyophilized to a light yellow flocculent powder (0.065 g, 21%).

## Example 5

[2S-(2α,3α,5α,6β)]-3-[[(2,5-Dihydro-6-hydroxy-2-methyl-5-oxo-1,2,4-triazine-3-yl)thio]-methyl]-3-methyl-7-oxo-6-[(phenyl-acetyl)amino]-4-thia-1-azabicyclo[3.2.0]heptane-2-carboxylic acid disodium salt

To a solution of [2S-(2α,3α,5α,6β)]-3-[[2,5-dihydro-6-hydroxy-2-methyl-5-oxo-1,2,4-triazine-3-yl)thio]-methyl]-3-methyl-7-oxo-6-[(phenylacetyl)amino]-4-thia-1-azabicyclo-[3.2.0]heptane-2-carboxylic acid(4-nitrophenyl)methyl ester monosodium salt (0.057 g, 0.088 mmol) in ethyl acetate (2 ml) and aqueous sodium bicarbonate (0.1M, 0.88 ml), was added 10% Pd/C(0.06g). The heterogeneous suspension was evacuated and charged with hydrogen five times and allowed to stir under a hydrogen atmosphere for 4 hours. The reaction mixture was filtered through celite and the celite was washed with water. The organic solvents were removed in vacuo, and the resultant aqueous solution was lyophilized. The residue was chromatographed on a reverse phase C18 Sep-Pak (Waters Assoc.) eluting with $CH_3CN/H_2O$ (0-40%). The proper fractions were combined, the acetonitrile removed in vacuo, and the aqueous solution was lyophilized. The residue was further purified by reverse phase, high pressure chromatography (Whatman M9-Partisil 10-ODS-2, linear gradient 0-100% $CH_3CN/H_2O$), to give [2S-(2α,3α,5α,6β)]-3-[[(2,5-dihydro-6-hydroxy-2-methyl-5-oxo-1,2,4-triazine-3-yl)thio]-methyl]-3-methyl-7-oxo-6-[(phenyl-acetyl)amino]-4-thia-1-azabicyclo[3.2.0]heptane-2-carboxylic acid disodium salt, as a white flocculent powder.

## Example 6

[2S-(2α,3α,5α,6β)]-3-(Azidomethyl)-3-methyl-7-oxo-6-[(phenylacetyl)amino]-4-thia-1-azabicyclo[3.2.0]-heptane-2-carboxylic acid(4-nitrophenyl)methyl ester

To a solution of [2S-(2α,3α,5α,6β)]-3-(bromomethyl)-3-methyl-7-oxo-6-[(phenylacetyl)amino]-4-thia-1-azabicyclo[3.2.0]-heptane-2-carboxylic acid (4-nitrophenyl)methyl ester (2.13 g, 3.89 mmol) in acetone (40 ml) and water (18 ml) at room temperature was added sodium azide (0.78g, 11.66 mmole). After 4 hours, the acetone was removed in vacuo, and the resultant mixture was partitioned with ethyl acetate (100 ml). The ethyl acetate layer was washed with water (2 x 50 ml), then brine (1 x 25 ml), dried over ($Na_2SO_4$), filtered, and concentrated in vacuo. Chromatography on silica gel (ethyl acetate/petroleum ether 2:3) gave 1.03 g of [2S-(2α,3α,5α,6β)]-3-(azidomethyl)-3-methyl-7-oxo-6-[(phenyl-acetyl)amino]-4-thia-1-azabicyclo [3.2.0]heptane-2-carboxylic acid (4-nitrophenyl)methyl ester.

## Example 7

[2S-(2α,3α,5α,6β)]-3-(Azidomethyl)-3-methyl-7-oxo-6-[(phenylacetyl)amino]-4-thia-1-azabicyclo[3.2.0]-heptane-2-carboxylic acid monosodium salt

To a solution of [2S-(2α,3α,5α,6β)]-3-(azidomethyl)-3-methyl-7-oxo-6-[(phenylacetyl)amino]-4-thia-1-azabicyclo-[3.2.0]heptane-2-carboxylic acid(4-nitrophenyl)methyl ester (0.198g, 0.39 mmol) in ethyl acetate (4 ml) under argon was added 10% Pd/C) (0.175 g). The heterogeneous suspension was evacuated and charged with hydrogen five times, and allowed to stir for 5 hours under a hydrogen atmosphere (delivered via balloon). The reaction mixture was filtered through celite and the celite was washed with tetrahydrofuran. To the filtrate was added sodium bicarbonate (0.049 g, 0.58 mmol) and water (2 ml), and the organic solvents were removed in vacuo. The resultant aqueous solution was lyophilized and purified by reverse phase liquid chromatography(Whatman M9-Partisil 10-ODS-2, linear gradient $CH_3CN/H_2O$), to give [2S-(2α,3α,5α,6β)]-3-(azidomethyl)-3-methyl-7-oxo-6-[(phenyl-acetyl)amino]-4-thia-1-azabicyclo-[3.2.0]heptane-2-carboxylic acid monosodium salt, as a white flocculent powder.

## Example 8

[2S-(2α,3α,5α,6β)]-3-[(Acetylthio)methyl]-3-methyl-7-oxo-6-[(phenylacetyl)-amino]-4-thia-1-azabicyclo[3.2.0]-heptane-2-carboxylic acid(4-nitrophenyl)-methyl ester

To a solution of [2S-(2α,3α,5α,6β)]-3-(bromomethyl)-3-methyl-7-oxo-6-[(phenylacetyl)amino]-1-aza-4-thia-bicyclo[3.2.0]-heptane-2-carboxylic acid (4-nitrophenyl)methyl ester (0.79 g 1.44 mmol), acetone (12 ml) and water (2 ml) was added potassium thioacetate (0.33 g, 2.9 mmol). After stirring at room temperature for 2 hours, the acetone was removed in vacuo and the remaining solution was partition between ethyl acetate and water. The aqueous layer was washed with ethyl acetate, and the ethyl acetate layers were combined, dried over sodium sulfate, filtered, and concentrated in vacuo. The residue was chromatographed on silica gel with ethyl acetate/petroleum ether (30 - 40%) to give [2S-(2α,3α,5α,6β)]-3-[(acetylthio)methyl]-3-methyl-7-oxo-6-[(phenylacetyl)amino]-4-thia-1-azabicyclo[3.2.0]heptane-2-carboxylic acid (4-nitrophenyl)methyl ester, in 35% yield.

## Example 9

[2S-(2α,3α,5α,6β)]-3-[(Acetylthio)methyl]-3-methyl-7-oxo-6-[(phenylacetyl)amino]-4-thia-1-azabicyclo[3.2.0]-heptane-2-carboxylic acid monosodium salt

To a solution of [2S-(2α,3α,5α,6β)]-3-[(acetylthio)methyl]-3-methyl-7-oxo-6-[(phenylacetyl)amino]-4-thia-1-azabicyclo-[3.2.0]heptane-2-carboxylic acid (4-nitrophenyl)methyl ester (0.164 g, 0.3 mmol) in ethyl acetate(1 ml) was added 10% Pd/C (0.154 g). The solution was evacuated and charged with hydrogen five times and allowed to stir under a hydrogen atmosphere, delivered via balloon, for 4 hours. The reaction mixture was filtered through celite and the celite was washed with THF. The solution was concentrated in vacuo and to the residue was added sodium bicarbonate (0.025 g, 0.3 mmol), tetrahydrofuran (0.5 ml) and water(1 ml). The tetrahydrofuran was removed in vacuo, and the remaining aqueous layer was lyophilized. Chromatography on a reverse phase C18 Sep-Pak (Waters Assoc.) with $CH_3CN/H_2O$ (0-20%) gave 53 mg (41%) of [2S-(2α,3α,5α,6β)]-3-[(acetylthio)methyl]-3-methyl-7-oxo-6-[(phenylacetyl)amino]-4-thia-1-azabicyclo[3.2.0]heptane-2-carboxylic acid monosodium salt, as a white flocculent powder.

## Example 10

[2S-(2α,3α,5α,6β)]-3-[[(2,5-Dihydro-3-mercapto-2-methyl-5-oxo-1,2,4-triazine-6-yl)oxy]methyl]-3-methyl-7-oxo-6-[(phenylacetyl)amino]-4-thia-1-azabicyclo-[3.2.0]heptane-2-carboxylic acid monosodium salt

[2S-(2α,3α,5α,6β)]-3-(bromomethyl)-3-methyl-7-oxo-6-[(phenylacetyl)amino]-1-aza-4-thia-bicyclo[3.2.0]-heptane-2-carboxylic acid (4-nitrophenyl) methyl ester was dissolved in DMF (6.55 ml), and tetrahydro-2-methyl-3-thioxo- 1,2,4-triazine-5.6-dione (378.5 mg, 1.191 mmol) was added and stirred at ambient temperature for 16 hours and 50 minutes. The ethyl acetate layer was washed with water (3 x 55 ml) and brine (55 ml), then dried over $Na_2SO_4$, filtered and concentrated to a residue which was purified by flash chromatography using 7% THF in methylene chloride. This gave the desired product (301.8 mg. 0.482 mmol, 25.1% yield) and additional less pure product (148.1 mg 0.236 mmol, 12.3% yield).

This p-nitrobenzyl ester product (289 mg, 0.461 mmol) was dissolved in ethyl acetate (9.58 ml), and 0.1N $NaHCO_3$ (4.61 ml, 0.461 mmol) was added, followed by 10% Pd/C catalyst (292.4 mg). The mixture was stirred at ambient temperature under hydrogen. After 2 hours and 45 minutes, the reaction was filtered through celite and the celite was washed with water and ethyl acetate. After separation, the aqueous layer was passed through a 0.5 mm millipore syringe filter and lyophilized to a crude product (106.4 mg). This product was purified by HPLC using acetonitrile/$H_2O$. A relatively pure product (53.9 mg) was obtained, plus less pure fractions (13.2 mg). They were further purified on an HPLC column, yielding a better product (15.7 mg).

## Example 11

[2S-(2α,3α,5α,6β)]-3-[[(2-Furanylcarbonyl)thio]methyl]-3-methyl-7-oxo-6-[(phenylacetyl)amino]-4-thia-1-azabicyclo-[3.2.0]heptane-2-carboxylic acid (4-nitrophenyl)methyl ester

A mixture of 82 mg (0.641 mmol) of 2-thiofuroic acid, 30 ml acetone,54 mg (0.643 mmol) of sodium bicarbonate, 351 mg (0.641 mmol) of [2S-(2α,3α,5α,6β)]-3-(bromomethyl)-3-methyl-7-oxo-6-[(phenylacetyl)-amino]-4-thia-1-azabicyclo[3.2.0]-heptane-2-carboxylic acid (4-nitrophenyl)methyl ester and water (10 ml) was stirred at room temperature for 1 hour. The reaction mixture was concentrated under reduced pressure until two phases formed and it was partitioned between ethyl acetate and brine. The organic phase was separated, extracted with brine (2x) and dried over anhydrous sodium sulfate. After filtration of the desiccant and evaporation of the solvent under reduced pressure, the residue was chromatographed in silica gel (eluted with 1:1 hexane:ethyl acetate) to give 248 mg of product.

Example 12

[2S-(2α,3α,5α,6β)]-3-[[(2-Furanylcarbonyl)thio]methyl]-7-oxo-6-[(phenylacetyl)amino]-4-thia-1-azabicyclo-[3.2.0]heptane-2-carboxylic acid monosodium salt

A mixture of 250 mg of 10% palladium on carbon, 40 ml of deionized water, 40 mg (0.476 mmol) sodium bicarbonate, 248 mg (0.417 mmol) of [2S-(2α,3α,5α,6β)]-3-[[(2-furanylcarbonyl)thio]methyl]-3-methyl-7-oxo-6-[(phenylacetyl)amino]-4-thia-1-azabicyclo[3.2.0]heptane-2-carboxylic acid (4-nitrophenyl)-methyl ester and 40 ml of ethyl acetate was shaken under an atmosphere of hydrogen (initial pressure 55.5 psi), at ambient temperature, on a Parr apparatus for 2 hours. The catalyst was removed by filtration through a celite pad on a fritted glass disc funnel and the filter cake was washed with cold water. Solid sodium chloride was added to the combined filtrate and washings. The aqueous phase was separated, extracted with ethyl acetate (2X) and lyophilized. The residue was dissolved in 45 ml of deionized water and placed on three C18 Sep Paks connected in series. Following elution with a solvent gradient of water and acetonitrile (100% water to 33% acetonitrile) and lyophilizing, 36 mg of product was obtained.

Example 13

[2S-(2α,3α,5α,6β]-3-(Fluoromethyl)-3-methyl-7-oxo-6-[(phenylacetyl)amino]-4-thia-1-azabicyclo[3.2.0]-heptane-2-carboxylic acid(4-nitrophenyl)methyl ester

A solution of 46 mg (0.362 mmol) of silver fluoride in 3 ml of deionized water was added dropwise at ambient temperature to a stirred solution of 200 mg (0.365 mmol) of [2S-(2α,3α,5α6β)]-3-(bromomethyl)-3-methyl-7-oxo-6-[(phenylacetyl)amino]-4-thia-1-azabicyclo[3.2.0]heptane-2-carboxylic acid (4-nitrophenyl)-methyl ester in 30 ml of acetone (flask was protected from light by covering with aluminum foil). The reaction was stirred at room temperature for 5 1/2 hours and filtered. The filtrate was concentrated under reduced pressure until 2 phases formed and partitioned between ethyl acetate and brine. The organic phase was separated. washed with brine (3x) and dried over anhydrous sodium sulfate. Following removal of the desiccant by filtration and evaporation of the solvent under reduced pressure, the residue was chromatographed on silica gel. Elution with 1:1 hexane to ethylacetate gave 47 mg of product.

Example 14

[2S-(2α,3α,5α,6β)]-3-(Fluoromethyl)-3-methyl-7-oxo-6-[(phenyl-acetyl)amino]-4-thia-1-azabicyclo[3.2.0]-heptane-2-carboxylic acid sodium salt

A mixture of 120 mg of 10% palladium on carbon, 40 ml of deionized water, 25 mg (0.298 mmol) of

sodium bicarbonate, 120 mg (0.246 mmol) of [2S-(2α,3α,5α,6β)]-3-(fluoromethyl)-3-methyl-7-oxo-6-[-(phenylacetyl)amino]-4-thia-1-azabicyclo-[3.2.0]heptane-2-carboxylic acid (4-nitrophenyl)methyl ester and 40 ml of ethyl acetate was shaken under an atmosphere of hydrogen (initial pressure 55.5 psi, 383 kPa), at ambient temperature, on a Parr apparatus for 2 hours. The catalyst was removed by filtration through a celite pad on a fritted disc glass funnel and the filter cake was washed with cold water. Solid sodium chloride (2.0 g) was added to the combined filtrate and washings. The aqueous phase was separated, extracted with ethyl acetate (2x) and lyophilized. The residue was dissolved in 45 ml of deionized water and placed on three C18 Sep Paks connected in series. Following elution with a solvent gradient of water and acetonitrile (100% water to 33% acetonitrile) and lyophilizing, 36 mg of product was obtained.

## Example 15

[2S-(2α,3α,5α,6β)-3-[[[3,4-bis(acetyloxy)benzoyl]thio]methyl]-3-methyl-7-oxo-6-[(phenylacetyl)amino]-4-thia-1-azabicyclo-[3.2.0]heptane-2-carboxylic acid sodium salt

3,4-bis(Acetyloxy)benzene carbothioic acid (113.6 mg, 0.447 mmol) was dissolved in 0.1N $KHCO_3$ (4.24 ml, 0.424 mmol) and 10.6 ml of acetone. [2S-(2α,3α,5α,6β)]-3-(Bromomethyl-3-methyl-7-oxo-6-[(phenylacetyl)amino]-4-thia-1-azabicyclo[3.2.0]heptane-2-carboxylic acid (4-nitrophenyl)methyl ester (231 mg. 0.421 mmol) was added and the reaction was stirred at ambient temperature under nitrogen. After 50 minutes, ethyl acetate (100 ml) and sodium bicarbonate (100 ml) were added. After separating the ethyl acetate layer, it was washed with brine (100 ml), then dried with sodium sulfate, filtered and concentrated to a crude residue (248 mg). This product (156 mg, 0.216 mmol if pure) was dissolved in tetrahydrofuran (10 ml) containing ethanol (0.5 ml). The catalyst, 10%Pd/C (200 mg), was added and the reaction mixture was stirred at ambient temperature under hydrogen. After 2 hours and 25 minutes, the reaction mixture was filtered through celite and concentrated to a residue, which was immediately redissolved in tetrahydrofuran (2 - 3 ml), and enough water(2 ml) was added to make the solution clear. Then, 0.1N $NaHCO_3$ (1.5ml, 0.15 mmol) was added with enough THF to again produce a clear solution (Note: acetonitrile (1 - 2 ml) was added). The THF and acetonitrile were then removed and gave a yellow slurry, which was diluted with water (3 ml) and filtered through celite (very slow). The filtrate was purified by HPLC, using acetonitrile/water. The product weighed 22.8 mg (0.0375 mmol, 17.34% yield).

## Example 16

[2S-(2α,3α,5α,6β)]-3-[[3,4-Dihydroxybenzoyl)oxy]methyl]-3-methyl-7-oxo-6-[(phenylacetyl)amino]-4-thia-1-azabicyclo-[3.2.0]heptane-2-carboxylic acid monosodium salt

A mixture of 200 mg (0.365 mmol) of [2S-(2α,3α,5α,6β)]-3-(bromomethyl)-3-methyl-7-oxo-6-[-(phenylacetyl)amino]-4-thia-1-azabicyclo[3.2.0]heptane-2-carboxylic acid (4-nitrophenyl)methyl ester, 60 mg (0.39 mmol) of 3,4-dihydroxy benzoic acid, 42 mg (0.39 mmol) of sodium bicarbonate, 40 ml of acetone and 10 ml of deionized water was stirred at room temperature for 24 hours and concentrated under reduced pressure until 2 phases formed. The mixture was partition between ethyl acetate and brine. The organic phase was separated, washed with brine (3x) and dried over anhydrous sodium sulfate. After filtration of the desiccant and evaporation of the solvent under reduced pressure, the residue was added to a mixture of 180 mg of 10% palladium in carbon, 40 ml of deionized water, 30 mg (0.357 mmol) of sodium bicarbonate and 40 ml of ethyl acetate. The mixture was hydrogenated (57.5 psi, 396 kPa) at room temperature for 2 hours. The catalyst was filtered off through a celite pad on a fritted disc glass funnel, and the filter pad was washed with water. Solid sodium chloride was added to the combined filtrate and washings. The aqueous phase was separated, extracted with ethyl acetate (2x) and lyophilized overnight. The residue was dissolved in 21 ml of deionized water and placed on three C18 Sep Paks connected in series. Following elution with a solvent gradient of water and acetonitrile (100% water to 33% acetonitrile) and lyophilizing, 21 mg of product was obtained.

## Example 17

[2S-(2α,3α,5α,6β)]-3-[[3-(1H-Imidazol-4-yl)-1-oxo-propoxy)]methyl]-3-methyl-7-oxo-6-[(phenylacetyl)amino]-4-thia-1-azabicyclo[3.2.0]heptane-2-carboxylic acid sodium salt

A mixture of 200 mg (0.365 mmol) of [2S-(2α,3α,5α,6β)]-3-(bromomethyl)-3-methyl-7-oxo-6-[(phenylacetyl)amino]-4-thia-1-azabicyclo[3.2.0]heptane-2-carboxylic acid (4-nitrophenyl)methyl ester, 70 mg (0.5 mmol) of 3-(4-imidazoly) propanoic acid, 42 mg (0.5 mmol) of sodium bicarbonate, 20 ml of acetone and 7 ml of water was stirred at room temperature for 20 hours, concentrated under reduced pressure until 2 phases formed, and partitioned between ethyl acetate and brine. The organic phase was separated, extracted with brine (3x) and dried over anhydrous sodium sulfate. After removing the desiccant by filtration and evaporation of the solvent under reduced pressure, the residue was added to a mixture of 210 mg of 10% palladium on carbon, 50 ml of deionized water, 30 mg (0.45 mmol) of sodium bicarbonate and 50 ml of ethyl acetate. The mixture was hydrogenated (55.0 psi, 379 kPa) on a Parr apparatus at room temperature in 1 1/2 hours. The catalyst was filtered off through a celite pad on a fritted disc glass funnel and the pad was washed with cold water. Solid sodium chloride was added to the combined filtrate and washings. The aqueous phase was extracted with ethyl acetate (2x) and lyophilized overnight. The residue was dissolved in 23 ml of deionized water and placed on three Sep Paks connected in series. Following elution with a solvent gradient of water and acetonitrile (100% water to 50% acetonitrile) and lyophilizing, 17 mg of product was obtained.

## Example 18

[2S-(2α,3α,5α,6β)]-1-[[2-carboxy-3-methyl-7-oxo-6[(phenylacetyl)amino]-4-thia-1-azabicyclo[3.2.0]hept-3-yl]-methylpyridinium hydroxide inner salt

To a dry tared vial containing an argon atmosphere was added $AgBF_4$(0.0453 g, 0.234 mmol). To a separated dry round-bottom flask containing an argon atmosphere was added [2S-(2α,3α,5α,6β)]-3-(bromomethyl)-3-methyl-7-oxo-6-[(phenylacetyl)amino]-4-thia-1-bicyclo[3.2.0]heptane-2-carboxylic acid (4-nitrophenyl) methyl ester (0.16g, 0.21 mmol), dry $CH_2Cl_2$ (3 ml), and dry pyridine (50 mg, 0.64 mmol), along with a magnetic stirring bar. As the solution was stirred rapidly at room temperature, the $AgBF_4$ was added rapidly through a funnel made of glassine paper. The reaction mixture became a cloudy white. The flask was wrapped with aluminum foil. After 3 1/2 hours, the reaction mixture was filtered through a plug of celite, and the celite was rinsed with $CH_2Cl_2$ and subsequently concentrated to a white solid in vacuo. The solid was rinsed with $H_2O$ and subsequently with 5%, 10%, 20% $CH_3CN/H_2O$ solution (a white suspension resulted), applying each rinse-solution (about 10 ml) to a C18 Sep Pak and eluting with $CH_3CN/H_2O$. The desired compound was eluted off the column with 10-40% $CH_3CN/H_2O$. The proper fractions were combined, the organic solvent was removed in vacuo, and the aqueous layer was lyophilized to give [2S-(2α,3α,5α,6β)]-1-[[3-methyl-2-[[(4-nitrophenyl)methoxy]carbonyl]-7-oxo-6-[(phenylacetyl)amino]-4-thia-1-azabicyclo-[3.2.0]hept3-yl]methyl]pyridinium tetrafluoroborate as a white flocculent solid; ($RfSiO_2$) = 0.5[-(EtOAc/AcOH/$H_2O$) 6:2:1)]. A spot grows in at lower Rf = 0.2on TLC ($SiO_2$) when the material obtained above is stored in $H_2O$ at room temperature for 24 - 48 hours. The desired product is soluble (> 1mg/ml) in EtOAc, $CH_2Cl_2$,$CDCl_3$, $CH_3COCH_3$, and slightly soluble in $H_2O$.

To [2S-(2α,3α,5α,6β)]-1-[[3-methyl-2-[[(4-nitrophenyl)methoxy]-carbonyl]-7-oxo-6-[(phenylacetyl)amino]-4-thia-1-azabicyclo-[3.2.0]hept-3-yl]methyl]pyridinium tetrafluoroborate (0.200 g, 0.32 mmol) in THF (10 ml) and $H_2O$ (100 ml) was added 0.2 g of 10% Pd/C and $NaHCO_3$ (0.1 mM, 3.2 ml), as well as and a magnetic stirring bar. The flask containing the rapidly stirring suspension was evacuated and subsequently charged with hydrogen gas three times. After 2 hours, the flask was evacuated and charged with argon and the suspension was filtered through a plug of celite. The THF in the filtrate was removed in vacuo and the remaining aqueous layer was passed through three-C18 Sep Paks connected in series. Acetonitrile/water solutions (0, 2, 5, 10, 20, 100%, 20 ml each) were passed through the C18-column. Collecting the proper fractions (5 - 10% $CH_3CN/H_2O$), removing the $CH_3CN$ in vacuo, and lyophilizing the aqueous layer gave 25 mg of the desired product as a white powder.

Example 19

2S-(2α,3α,5α,6β)]-3-[[[3,4-bis(Acetyloxy)benzoyl]oxy]methyl]-3-methyl-7-oxo-6-[(phenylacetyl)amino]-4-thia-1-azabicyclo-[3.2.0]heptane-2-carboxylic acid (4-nitrophenyl)methyl ester

A suspension of 250 mg (0.73 mmol) of silver diacetyloxy benzoate in 20 ml of dry acetonitrile was added dropwise over 20 minutes to a stirred solution, at 0° C, of 0.4 g (0.73 mmol) of [2S-(2α,3α,5α,6β)-3-(bromomethyl)-3-methyl-7-oxo-6-[(phenylacetyl)amino-4-thia-1- azabicyclo[3.2.0]heptane-2-carboxylic acid (4-nitrophenyl) methyl ester in 20 ml of dry acetone, in a flask wrapped with aluminum foil and present in an ice water bath. The reaction mixture was stirred near 0° C for 5 hours and filtered through a short column of silica gel (wet with acetone). The column was washed with 100 ml of acetone and the solvent was evaporated under reduced pressure from the combined filtrate and washings. The residue was chromatographed on silica gel (230 -400 mesh, eluted with 1:1 hexane:ethyl acetate), to yield 242 mg of product.

Example 20

[2S-(2α,3α,5α,6β)]-3-[[[3,4-bis(Acetyloxy)benzoyl]oxy]methyl]-3-methyl-7-oxo-6-[(phenylacetyl)amino]-4-thia-1-azabicyclo[3.2.0]heptane-2-carboxylic acid monosodium salt

A mixture of 100 mg of 10% palladium on carbon, 50 ml of deionized water, 12 mg (0.143 mmol) of sodium bicarbonate, 100 mg (0.142 mmol) of [2S-(2α,3α,5α,6β)]-3-[[[3,4-bis(acetyloxy)benzoyl]oxy]methyl]-3-methyl-7-oxo-6-[(phenylacetyl)amino]-4-thia-1-azabicyclo[3.2.0]heptane-2-carboxylic acid (4-nitrophenyl)-methyl ester and 50 ml of ethyl acetate was shaken under an atmosphere of hydrogen (initial pressure 55 psi, 379 kPa), at room temperature, on a Parr apparatus for 3 hours. The catalyst was removed by filtration through a celite pad on a fritted disc glass funnel and the filter cake was washed with a small amount of cold water. The combined filtrate and washing were placed in a separator funnel. The aqueous layer was removed and lyophilized. The residue was purified through C18 Sep-Paks (100% $H_2O$ to 40% acetonitrile/water), to give 38 mg of product.

Example 21

2S-(2α,3α,5α,6β)]-3-methyl-7-oxo-6-[(phenylacetyl)amino]-3-[(thiocyanato)methyl-4-thia-1-azabicyclo[3.2.0]-heptane-2-carboxylic acid monosodium salt

To a solution containing [2S-(2α,3α,5α,6β)]-3-(bromomethyl)-3-methyl-7-oxo-6-[(phenylacetyl)amino]-4-thia-1-azabicyclo[3.2.0]heptane-2-carboxylic acid (4-nitrophenyl) methyl ester (0.541 g. 1.06 mmol), acetone (10 ml) and water (10 ml), was added KSCN (0.113 g, 1.17 mmol). After stirring at room temperature for 7 hours, the acetone was removed in vacuo, and EtOAc (50 ml) added to the resultant suspension. The EtOAc layer was washed with water (30 ml), brine (30 ml), dried over $Na_2SO_4$, filtered and concentrated in vacuo. The residue was dissolved in a minimal amount of $CH_2Cl_2$, applied to a flash column (40 g $SiO_2$, 40%EtOAc/pet. ether, 20 ml fractions) and eluted to give 0.28 g of [2S-(2α,3α,5α,6β)]-3-methyl-7-oxo-6-[-(phenylacetyl)amino]-3-[(thiocyanato)methyl]-4-thia-1-azabicyclo[3.2.0]heptane-2-carboxylic acid (4-nitrophenyl)methylester as an off-white amorphous solid.

To a solution of [2S-(2α,3α,5α,6β)-3-methyl-7-oxo-6-[(phenylacetyl)amino]-3-[(thiocyanato)methyl]-4-thia-1-azabicyclo[3.2.0]heptane-2-carboxylic acid (4-nitrophenyl)methy-ester(0.167 g, 0.32 mmol), EtOAc (2 ml), water (3 ml) and $NaHCO_3$(0.04 g) was added 0.17 g of 10% Pd/C and a magnetic stirring bar. The flask containing the rapidly stirring suspension was evacuated and subsequently filled with hydrogen gas three times. After stirring the reaction mixture at room temperature under a hydrogen atmosphere (delivered by balloon) for 3 1/2 hours, the flask was evacuated and filled with argon, and filtered through a celite pad. The EtOAc in the filtrate was removed in vacuo and the resultant aqueous suspension was lyophilized. The

resultant solid was applied to a C18 column consisting of 3-Sep Paks connected in series. The column was eluted with a 0 - 100% $CH_3CN/H_2O$ gradient collecting the proper fractions which were subsequently concentrated in vacuo to remove the $CH_3CN$, and lyophilized to give the desired product as a white flocculent powder (Rf = 0.3 ($SiO_2$), 60:3:1 EtOAc/AcOH/$H_2O$).
A second purification by HPLC, using a C18-Mg Whatman column which was eluted with $CH_3CN/H_2O$, gave the desired product in pure form for testing.

## Example 22

[2S-(2α,3α(E),5α,6β)]-3-[[[3-(1H-Imidazol-4-yl)-1-oxo-2-propenyl]oxy]methyl]-3-methyl-7-oxo-6-[-(phenylacetyl)amino]-4-thia-1-azabicyclo[3.2.0]heptane-2-carboxylic acid monosodium salt

A solution of 200 mg (0.365 mmol) of [2S-(2α,3α,5α,6β)]-3 (bromomethyl)-3-methyl-7-oxo-6-[-(phenylacetyl)amino]-4-thia-1-azabicyclo[3.2.0]heptane-2-carboxylic acid (4-nitrophenyl)methyl ester, 76 mg (0.475 mmol) of sodium urocanate, 20 ml acetone and 7 ml of water was stirred at room temperature for 24 hours. The reaction mixture was concentrated under reduced pressure until 2 phases formed and it was partitioned between ethyl acetate and brine. The organic phase was extracted with brine (2X), dried over anhydrous sodium sulfate, filtered, and the solvent was evaporated under reduced pressure. The residue, 200 mg of 10% palladium on carbon, 40 ml deionized water, 40 ml of ethyl acetate and 24 mg (0.286 mmol) of sodium bicarbonate were shaken at ambient temperature under an atmosphere of hydrogen (42 psi, 290 kPa) on a Parr apparatus for 2 hours. The catalyst was removed by filtration through a celite pad on a fritted disc glass funnel, and the filter cake was washed with cold water. Solid sodium chloride was added to the combined filtrate and washings. The aqueous phase was separated, extracted with ethyl acetate (2x) and lyophilized. The residue was dissolved in 19 ml of deionized water and placed on three C18 Sep Paks in series. Following elution with a solvent gradient of water and acetonitrile (100% water to 50% acetonitrile) and lyophilizing, 55 mg of product was obtained.

## Example 23

[2S-(2α,3α,5α,6β(Z))]-6-[[2-(2-Amino-4-thiazolyl)-2-(methoxyimino)-acetyl]amino]-3-methyl-3-[[(1-methyl-1H-tetrazol-5-yl)thio]methyl]-7-oxo-4-thia-1-azabicyclo[3.2.0]heptane-2-carboxylic acid monosodium salt

To a solution of [2S-(2α,3α,5α,6β)]-3-methyl-3-[[(1-methyl-1H-tetrazol-5-yl)thio]methyl]-7-oxo-6-[-(phenylacetyl)amino]-4-thia-1-azabicyclo[3.2.0]-heptane-2-carboxylic acid (4-nitrophenyl)methyl ester (0.741, 1.27 mmol), pyridine (0.126 g, 1.59 mmol) in $CHCl_3$ (9 ml) and cooled in a cold bath (-15 to -10 °C), was added $PCl_5$ (0.291 g, 1.40 mmol) in one portion as a solid. After stirring at -15 to 10 °C for 1 hour, n-propanol (0.76 g, 12.7 mmol) was added, followed 40 minutes later by the addition of brine (5 ml). The resultant mixture was allowed to stir at about 0 °C for 15 minutes, after which it was transferred to a separatory funnel. EtOAc (50 ml) was added, and the mixture was washed with $NaHCO_3$ (saturated), water and brine, dried over $NaHCO_3$, filtered and concentrated in vacuo. The residue was dissolved in $CH_2Cl_2$ [10 ml] and to the resultant solution was added S-[2-benzothiazolyl]-2-amino-4-thiazologlyoxylate (E)-0-methyloxime. After the reaction was complete as determined by TLC ($SiO_2$, EtOAc), in about 3-4 hours, the reaction mixture was concentrated in vacuo and purified by flash chromatography ($SiO_2$, EtOAc) to give [2S-(2α,3α,5α,6β(z))]-6-[[2-(2-amino-4-thiazolyl)-2-(methoxyimino)acetyl]amino]-3-methyl-3-[[(1-methyl-1H-tetrazole-5-yl)thio]methyl]-7-oxo-4-thia-1-azabicyclo[3.2.0]-heptane-2-carboxylic acid (4-nitrophenyl)methyl ester as a white amorphous solid.

To a solution of [2S-(2α,3α,5α,6β(z))]-6-[[2-(2-amino-4-thiazolyl)-2-(methoxyimino)acetyl]amino]-3-methyl-3-[[(1-methyl-1H-tetrazol-5-yl)thio]methyl]-7-oxo-4-thia-1-azabicyclo[3.2.0]heptane-2-carboxylic acid (4-nitrophenyl)methyl ester (0.058 g, 0.09mmol), EtOAC (2 ml), and aq. $NaHCO_3$ (0.1 M, 0.89 ml), was added 0.06 g of 10% Pd/C and a magnetic stirring bar. The flask containing the rapidly stirring solution was evacuated and filled with hydrogen gas three times, and the contents were allowed to stir at room temperature under an atmosphere of hydrogen (delivered by balloon). After 2 1/2 hours, the reaction

mixture was filtered through a celite pad, the EtOAc was removed in vacuo, and the water was removed by lyophilization. The resultant solid was dissolved in water and purified by C18 chromatography with 3-Sep Paks connected in series and eluting with a $CH_3CN/H_2O$ gradient. After combining the proper fractions, the $CH_2CN$ was removed in vacuo and the resultant solution was lyophilized to give [2S-[2α,3α,5α,6β(z)]]-6-[[2-(2-amino-4-thiazolyl)-2-(methoxyimino)acetyl]amino]-3-methyl-3-[[(1-methyl-1H-tetrazol-5-yl)thio]methyl]-7-oxo-4-thia-1-azabicyclo[3.2.0]heptane-2-carboxylic acid monosodium salt as a white flocculent powder.

Example 24

[2S-(2α,3α,5α,6β(Z))]-6-[[2-(2-Amino-4-thiazolyl)-2-(methoxyimino)acetyl]amino]-3-(azidomethyl)-3-methyl-7-oxo-4-t hia-1-azabicyclo[3.2.0]heptane-2-carboxylic acid monosodium salt

Phosphorus pentachloride (320.4 mg, 1.539 mmol) was dissolved in methylene chloride (5.8 ml), and pyridine (141.8 ml, 1.753 mmol) was added and stirred under argon. This solution was cooled to -10 to 15° C (white precipitate formed as soon as the cooling was started). After 5-10 minutes, [2S-(2α,3α,5α,6β)]-3-(azidomethyl)-3-methyl-7-oxo-6-[(phenylacetyl)amino]-4-thia-1-azabicyclo[3.2.0]heptane-2-carboxylic acid (4-nitrophenyl)methyl ester (716mg, 1.402 mmol) was added with methylene chloride (4.3 ml). After 1 1/2 hours, isobutanol (1.29 ml, 14.02 mmol) was added, and 45 minutes later brine (15 ml) was added and the mixture was stirred for 10 minutes. To this mixture was added saturated sodium bicarbonate (15 ml). After separating, the organic layer was washed with another portion of saturated sodium bicarbonate (15 ml), followed by a brine wash (15 ml). The layer was then dried over sodium sulfate. The solution was transferred to a flask containing S-[2-benzothiazolyl]-2-amino-4-thiazologlyoxylate (E)-O-methyloxime (444.5 mg 1.396) and stirred under argon at ambient temperature for 1 1/2 hours. It was then filtered through celite and purified by flash chromatography using ethyl acetate and petroleum ether (3/1) and ethyl acetate, methanol, and methylene chloride (9/0.25/6, v/v/v). The p-nitrobenzyl ester product weighed 200 mg (0.348 mmol 24.9% yield).

This product (164 mg, 0.285 mmol) was dissolved in tetrahydrofuran (10.8 ml), and 10% Pd/C (165 mg) was added. The mixture was stirred at ambient temperature under hydrogen for 2 hours. It was then filtered through celite along with a THF wash. Sodium bicarbonate (26.6 mg,0.317 mmol) was dissolved in water and added to the THF solution. Enough water and THF was subsequently added to keep the solution clear. The THF was then removed under vacuum producing a yellow slurry which was filtered (some foaming). The filtrate was then passed through a 0.45 mm millipore syringe filter and lyophilized (26.4mg). It was next purified by HPLC using acetonitrile and water to give 17.6 mg (0.0381 mmol, 13.3% yield).

Example 25

[2S-[2α,3α,5α,6β(Z)]]-3-[[[3,4-bis(Acetyloxy)benzoyl]oxy]methyl]-6-[[2-(2-amino-4-thiazolyl)-2-(methoxyimino)acetyl]amino-3-methyl-7-oxo-4-thia-1-azabicyclo[3.2.0]heptane-2-carboxylic acid (4nitrophenyl)methyl ester

Under an atmosphere of dry argon, a stirred mixture of 61.2 mg (0.294 mmole) of phosphorus pentachloride in 4 ml of dry methylene chloride was cooled to -12° C with an ice-salt bath, and 0.06 ml (0.75 mmol) of dry pyridine was added. After 187 mg (0.265 mmol) of [2S-[2α,3α,5α,6β)]-3-[[[3,4-bis-(acetyloxy)benzoyl]oxy]methyl]-3-methyl-7-oxo-6-[(phenylacetyl)amino]-4-thia-1-azabicyclo[3.2.0]heptane-2-carboxylic acid (4-nitrophenyl)methyl ester and 2 ml of dry methylene chloride were added, the temperature of the stirred reaction mixture was maintained near -12° C with an ice-salt bath for 3 1/2 hours. Dry isobutyl alcohol (0.15 ml, 1.63 mmol) was added dropwise and stirring was continued for 1 hour and 15 minutes, with the temperature being held near -11° C (ice-salt bath). Following the dropwise addition of 0.5 ml of saturated brine, the temperature of the stirred reaction mixture was held near -10° C for 1/2 hour, and the reaction mixture was diluted with 6 ml of methylene chloride, 3 ml more of saturated brine and 0.6 ml of saturated sodium bicarbonate. The organic phase was separated, washed by extraction with saturated brine (3x) and dried over anhydrous sodium sulfate. Following removal of the desiccant by filtration, the

methylene chloride solution of [2S-[2α,3α,5α,6β)]-3-[[[3,4-bis-(acetyloxy)-benzoyl]oxy]methyl]-7-oxo-6-amino-4-thia-1-azabicyclo[3.2.0]-heptane-2-carboxylic acid (4-nitrophenyl)methyl ester was added dropwise at ambient temperature to a stirred solution of 70 mg (0.2 mmol) of S-[2-benzothiazolyl]-2-amino-4-thiazologlyoxylate(E)-O-methyloxime in 30 ml of dry methylene chloride. The reaction mixture was stirred at room temperature for 5 1/2 hours, washed by extraction with saturated sodium bicarbonate (1x) followed by saturated brine (3x), and dried over anhydrous sodium sulfate. After removal of the desiccant by filtration and evaporation of the solvent at reduced pressure, the residue was chromatographed on silica gel eluted with 1:4 hexane:ethylacetate, to give 47 mg of product.

## Example 26

[2S-[2α,3α,5α,6β(Z)]]-3-[[[3,4-bis(Acetyloxy)benzoyl]oxy]methyl]-6-[[2-(2-amino-4-thiazolyl)-2-(methoxyimino)acetyl]amino]-3-methyl-7-oxo-4-thia-1-azabicyclo-[3.2.0]heptane-2-carboxylic acid monosodium salt

A mixture of 70 mg of 10% palladium on carbon, 20 ml of deionized water, 10 mg (0.12 mmol) of sodium bicarbonate, 70 mg (0.091 mmol) of [2S-[2α,3α,5α,6β(Z)]]-3-[[[3,4-bis(acetyloxy)benzoyl]oxy]-methyl]-6-[[2-(2-amino-4-thiazolyl)-2-(methoxyimino)acetyl]amino]-3-methyl-7-oxo-4-thia-1-azabicyclo[3.2.0]-heptane2-carboxylic acid (4-nitrophenyl)methyl ester and 30 ml of ethyl acetate was shaken under an atmosphere of hydrogen (initial pressure 56 psi, 386 kPa), at room temperature, on a Parr apparatus for 2 1/2 hours. The catalyst was removed by filtration through a celite pad on a scinter glass funnel, and the filter cake was washed with cold water. Solid sodium chloride was added to the combined filtrate and washings. The organic phase was separated. The aqueous phase was extracted with ethyl acetate (3x) and lyophilized overnight. The residue dissolved in water (15 ml) was placed on two C18 Sep Paks connected in series. Following elution with a solvent gradient of water and acetonitrile (100% water to 33% acetonitrile/water), the product was lyophilized to yield 14 mg of product.

## Example 27

[2S-[2α,3α,5α,6β(Z)]]-6-[[2-(2-Amino-4-thiazolyl)-2-(methoxyimino)acetyl]amino]-3-[[(2,5-dihydro-6-hydroxy-2-methyl-5-oxo-1,2,4-triazine-3-yl)thio]methyl]-3-methyl-7-oxo-4-thia-1-azabicyclo-[3.2.0]heptane-2-carboxylic acid (4-nitrophenyl)methyl ester monosodium salt

Phosphorus pentachloride (205.6 mg, 0.987 mmol) was dissolved in chloroform (3.93 ml, freshly dried through aluminum). Pyridine (90.7 ml, 1.121 mmol) was added (precipitate immediately formed) and the suspension was cooled at -10 °C to -15 °C under nitrogen (dry). After 5 to 10 minutes, [2S-[2α,3α,5α,6β)]-3-(bromomethyl)-3-methyl-7-oxo-6-[(phenylacetyl)amino]-4-thia-1-azabicyclo[3.2.0]heptane-2-carboxylic acid (4-nitrophenyl)methyl ester] (492.4mg, 0.898 mmol) was added. After 45 minutes, n-propanol (0.67 ml, 8.963 mmol) was added. After 1 hour, brine (10 ml) was added and stirred for 5 minutes, then saturated sodium bicarbonate (10 ml) and chloroform (2 ml) were added and stirred for 5 minutes more. The chloroform layer was washed with brine (10 ml) and dried over $Na_2SO_4$, then filtered through celite. S-[2-Benzothiazolyl]-2-amino-4-thiazoleglyoxylate (E)-O-methyloxime (285.8 mg, 0.898 mmol) was added to the filtrate along with a few milliliters of chloroform. Th filtrate was then stirred at ambient temperature under nitrogen (dry). After 2 hours, the solvent was removed and the residue (686.4 mg) was dissolved in methylene chloride and purified by flash chromatography, using 1/1 and 2/1 ethyl acetate/petroleum ether. The resulting product weighed 121 mg.

The above product (119 mg, 0.194 mmol) was dissolved in acetone(2 ml) and water (1.25 ml). The disodium salt of tetrahydro-2-methyl-3-thioxo-1,2,4-triazine-5,6-dione (39.4 mg (0.194 mmol) was added with acetone(4.27 ml) and stirred under nitrogen (dry) for 15 hours. The acetone was then stripped off (foaming occurred). The residue was redissolved in ethyl acetate and water and filtered through celite. A portion of the water layer was purified on a C18 Sep Pak to give 2.8 mg of product.

Example 28

[2S-[2α,3α,5α,6β(Z)]]-6-[[2-(2-Amino-4-thiazolyl)-2-(methoxyimino)acetyl]amino]-3-[[(2,5-dihydro-6-hydroxy-2-methyl-5-oxo-1,2,4-triazine-3-yl)thio]methyl]-3-methyl-7-oxo-4-thia-1-azabicyclo[3.2.0]heptane-2-carboxylic acid disodium salt

[2S-[2α,3α,5α,6β(Z)]]-6-[[2-(2-amino-4-thiazolyl)-2-(methoxyimino)-acetyl]amino]-3-[[(2,5-dihydro-6-hydroxy-2-methyl-5-oxo-1,2,4-triazine-3-yl)thio]methyl]-3-methyl-7-oxo-4-thia-1-azabicyclo[3.2.0]heptane-2-carboxylic acid (4-nitrophenyl)methyl ester monosodium salt (20.4 mg,0.0286 mmol) was dissolved in ethyl acetate (0.6 ml) and 0.1N $NaHCO_3$(0.28 ml, 0.028 mmol) was added, followed by the catalyst, 10% Pd/C-(21.2 mg). The mixture was stirred at ambient temperature under hydrogen. After 2 hours and 45 minutes, ethyl acetate (1 ml) and 0.1N $NaHCO_3$ (1 ml) were added, and the reaction was filtered through celite and the layers were separated. The aqueous layer was passed through two C18 Sep Paks and gave 13.4 mg of product after lyophilizing. This was further purified by HPLC using acetonitrile/water.

Example 29

[2S-[2α,3α,5α,6β(R*))]-6-[[-2-[[4-Ethyl-2,3-dioxo-1-piperazinylcarbonyl]amino]-2-phenylacetyl]amino]-3-methyl-3-[[(1-methyl-1H-tetrazol-5-yl)thio]methyl]-7-oxo-4-thia-1-azabicyclo[3.2.0]heptane-2-carboxylic acid (4-nitrophenyl) methyl ester

(R)-α-[[(4-ethyl-2,3-dioxopiperazinyl)carbonyl]amino]benzene-acetic acid (157.9 mg, 0.495 mmol) and 2-mercaptobenzothiazole (84.1 mg, 0.504 mmol) were dissolved in chloroform (3.15 ml). 1,3-Dicyclohexylcarbodiimide (101.6 mg, 0.493 mmol) was dissolved in chloroform (1.88 ml) and added to the first solution. It was stirred at ambient temperature for 1 hour and 45 minutes. The free amine of [2S-[2α,3α,5α,6β)]-3-methyl-3-[[(1-methyl-1H-tetrazol-5-yl)thio]methyl]-7-oxo-6-amino-4-thia-1-azabicyclo[3.2.0] heptane-2-carboxylic acid (4-nitrophenyl)methyl ester was simultaneously prepared as follows: phosphorus pentachloride (90 mg, 0.432 mmol) was dissolved in 2 ml chloroform (passed through alumina) and pyridine (39.7 ml, 0.491 mmol) was added (a precipitate immediately formed). The mixture was cooled to -15 to -20 °C for 5 to 10 minutes, then [2S-[2α,3α,5α,6β)]-3-methyl-3-[[(1-methyl-1H-tetrazole-3-yl)thio]methyl]-7-oxo-6-[-(phenylacetyl)amino]-4-thia-1-azabicyclo[3.2.0]heptane-2-carboxylic acid (4-nitrophenyl)methyl ester was added. After 1 hour and 15 minutes, n-propanol (305 ml, 4.080 mmol) was added. After 1 hour and 40 minutes, brine (3 ml) was stirred in for 10 minutes, then the layers were separated and saturated sodium bicarbonate (4 ml) and chloroform (2 ml) were added. The aqueous layers were extracted with chloroform (2 ml). All chloroform layers were combined and dried over $Na_2SO_4$, then filtered and the filtrate added to the reaction solution prepared in the first part.

After 2 hours, the reaction mixture was extracted with saturated sodium bicarbonate and the organic phase was dried over $Na_2SO_4$, then filtered and purified by flash chromatography using 9/0.25/6 ethylacetate/methanol/methylene chloride as an eluant. The partially purified product (103 mg) was further purified by HPLC, using ethyl acetate/methylene chloride, to give 61.6 mg of product (0.0803 mmol, 20.3% yield).

Example 30

[2S-[2α,3α,5α,6β(R*)]]-6-[[-2-[[(4-Ethyl-2,3-dioxo-1-piperazinyl)carbonyl]amino]-2-phenylacetyl]amino]-3-methyl-3-[[(1-methyl-1H-tetrazol-5-yl)thio]methyl]-7-oxo-4-thia-1-azabicyclo[3.2.0]heptane-2-carboxylic acid monosodium salt sesquihydrate

[2S-[2α,3α,5α,6β(R*)]]-6-[[-2-[[(4-Ethyl-2,3-dioxo-1-piperazinyl)carbonyl]amino]-2-phenylacetyl]amino]-3-methyl-3-[[(1-methyl-1H-tetrazol-5-yl)thio]methyl]-7-oxo-4-thia-1-azabicyclo[3.2.0]heptane-2-carboxylic acid

(4-nitrophenyl)methyl ester (38.5 mg, 0.0502 mmol) was dissolved in ethyl acetate (1.74 ml), and 10% Pd/C (39.2 mg) was added. Water (2.6 ml) was then added, followed by 0.1 N $NaHCO_3$ (0.6 ml, 0.06mmol). The mixture was stirred at ambient temperature under hydrogen. After 1 hour and 55 minutes, the mixture was filtered through celite and the celite was washed with ethyl acetate and water. This was then filtered through a 0.22 mm millipore syringe filter and allowed to separate for 1 hour. The aqueous layer was purified by HPLC using acetonitrile/water, and the product 15.0 mg (0.0229 mmol, 45.7% yield) was recovered by stripping off the acetonitrile and lyophilizing the water layer.

## Example 31

[2S-[2α,3α,5α,6β(R*)]]-3-[[[3,4-bis(Acetyloxy)benzoyl]oxy]methyl-6-[[-2-[[(4-ethyl-2,3-dioxo-1-piperazinyl)-carbonyl]amino]-2-phenylacetyl]amino]-3-methyl-7-oxo-4-thia-1-azabicyclo[3.2.0]heptane-2-carboxylic acid (4-nitrophenyl)methyl ester

A stirred mixture of 91. mg (0.437 mmol) of phosphorous pentachloride in 5 ml of dry methylene chloride was cooled to -13° C by means of an ice-salt bath, and 0.07 ml (0.87 mmol) of dry pyridine was added. After 280 mg (0.4 mmol) of [2S-[2α,3α,5α,6β]]-3-[[[3,4-bis(acetyloxy)benzoyl]oxy]methyl]-6-[-(phenylacetyl)amino]-3-methyl-7-oxo-4-thia-1-azabicyclo[3.2.0]heptane-2-carboxylic acid (4-nitrophenyl)-methyl ester was added, the reaction was stirred near -15° C for 3 hours, and 0.2 ml (2.18 mmol) of dry isobutyl alcohol was added dropwise. The reaction mixture was stirred an additional hour with the temperature held near -12° C, and 0.6 ml of saturated brine was added. Stirring was continued for 15 minutes after the last addition, and the stirred mixture was diluted with 20 ml of methylene chloride. The organic phase was separated and extracted with saturated sodium bicarbonate (1x), followed by brine (3x). After drying over anhydrous sodium sulfate, the desiccant was filtered off and the filtrate was added to a stirred tetrahydrofuran solution of previously prepared 109.5 mg (0.251 mmol) of(R)-N[2-(1H-benzotriazol-1-yl-oxy)-2-oxo-1-phenylethyl]-4-ethyl-2,3-dioxo-1-piperazine carboxamide (a mixture of 80 mg (0.251 mmol) of (R)-alpha-[[(4-ethyl-2,3-dioxopiperazinyl)carbonyl]amino]phenylacetic acid, 34 mg (0.25 mmol) of 1-hydroxybenzotriazole, 52 mg (0.25 mmol) N,N-dicyclohexylcarbodiimide and 10 ml of dry tetrahydrofuran was stirred at room temperature for 2 hours). The reaction mixture was stirred overnight at room temperature and concentrated to dryness under reduced pressure. The residue was triturated with ethyl acetate (4x) and filtered. The filtrate was extracted with dilute sodium bicarbonate (1x), brine (3x), and then was dried over anhydrous sodium sulfate. Following filtration of the desiccant and evaporation of the solvent, the residue was chromatographed on silica gel (eluted with ethyl acetate, neat), to give 41 mg of product.

## Example 32

[2S-[2α,3α,5α,6β(R*)]]-3-[[[3,4-bis(Acetyloxy)benzoyl] oxy]methyl]-6-[[-2-[[(4-ethyl-2,3-dioxo-1-piperazinyl)-carbonyl]amino]-2-phenylacetyl]amino]-3-methyl-7-oxo-4-thia-1-azabicyclo[3.2.0]heptane-2-carboxylic acid monosodium salt

A mixture of 60 mg of 10% palladium on carbon, 30 ml of deionized water and 14 mg (17 mmol) of sodium bicarbonate, 56 mg (0.063 mmol) of [2S-[2α,3α,5α,6β(R*)]]-3-[[[3,4-bis(acetyloxy)benzoyl]oxy]-methyl]-6-[[-2-[[(4-ethyl-2,3-dioxo-1-piperazinyl)carbonyl]amino]-2-phenylacetyl]amino]-3-methyl-7-oxo-4-thia-1-azabicyclo[3.2.0]heptane-2-carboxylic acid (4-nitrophenyl)methyl ester and 30 ml of ethyl acetate was shaken under an atmosphere of hydrogen (initial pressure 53.5 psi, 369 kPa) at room temperature on a Parr apparatus for 2 hours. The catalyst was removed by filtration through a celite pad on a fritted disc glass funnel and the filter cake was washed with cold water. Solid sodium chloride was added to the combined filtrate and washings. The aqueous phase was extracted with ethyl acetate (3x) and lyophilized overnight. The residue, dissolved in 35 ml of deionized water, was placed on two C18 Sep Paks connected in series. Following elution with a solvent gradient of water and acetonitrile(100% water to 50% acetonitrile/water), 16 mg of product was obtained.

Example 33

[2S-[2α,3α,5α,6β(R*)]]-6-[[-2-[[(4-Ethyl-2,3-dioxo-1-piperazinyl)-carbonyl]amino]-2-phenylacetyl]amino]-3-[[-(3,4-dihydroxybenzoyl)oxy]methyl]-3-methyl-7-oxo-4-thia-1-azabicyclo[3.2.0]heptane-2-carboxylic acid monosodium salt

A solution of 250 mg (0.34 mmol) of [2S-(2α,3α,5α,6β)]-3-(bromomethyl)-3-methyl-6-[[-2-[[(4-ethyl-2,3-dioxo-1-piperazinyl)-carbonyl]amino]-2-phenylacetylamino]-7-oxo-4-thia-1-azabicyclo-[3.2.0]heptane-2-carboxylic acid (4-nitrophenyl)methyl ester, 60 mg (0.39 mmol) of 3,4-dihydroxybenzoic acid, 42 mg (0.5 mmol) of sodium bicarbonate, 40 ml of acetone and 10 ml of deionized water was stored at room temperature for 19 hours. The reaction mixture was concentrated under reduced pressure until two phases formed and partitioned between ethyl acetate and brine. The organic phase was separated, washed by extraction with brine (3x) and dried over anhydrous sodium sulfate. After removal of the desiccant by filtration and evaporation of the solvent under reduced pressure,the residue was added to a suspension of 200 mg of 10% palladium on carbon, 40 ml of deionized water, 25 mg (0.298 mmol) of sodium bicarbonate and 40 ml of ethyl acetate. The mixture was hydrogenated (55.0 psi, 379 kPa) on a Parr apparatus for 2 hours. The catalyst was removed by filtration through a celite pad on a fritted disc glass funnel, and the filter pad was washed with cold water. Solid sodium chloride (2.0 g) was added to the combined filtrate and washings. The aqueous phase was separated, extracted with ethyl acetate (2 x) and lyophilized overnight. The residue, dissolved in 18 ml of deionized water, was placed on three C18 Sep Paks connected in series. Following elution with a solvent gradient of water and acetonitrile (100% water to 50% acetonitrile/water), 21.3 mg of product was obtained.

Example 34

[2S[2α,3α,5α,6β(R*)]]-6-[[-2-[[(4-Ethyl-2,3-dioxo-1-piperazinyl)-carbonyl]amino]-2-phenylacetyl]amino]-3-[[(2-furanylcarbonyl)thio]methyl]-3-methyl-7-oxo-4-thia-1-azabicyclo[3.2.0]heptane-2-carboxylic acid sodium salt

A solution of 50 mg (0.39 mmol) of 2-thiofuroic acid, 60 ml of acetone, 34 mg (0.41 mmol) of sodium bicarbonate, 197 mg (0.27 mmol) of [2S[2α,3α,5α,6β(R*)]]-3-(bromomethyl)-6-[[2-[[(4-ethyl-2,3-dioxo-1-piperazinyl)carbonyl]amino]-2-phenylacetyl]amino]-3-methyl-7-oxo-4-thia-1-azabicyclo[3.2.0]heptane-2-carboxylic acid (4-nitrophenyl)methyl ester and 20 ml of deionized water was stirred for 1 for hour at room temperature. The reaction mixture was concentrated under reduced pressure until 2 phases formed, then partitioned between ethyl acetate and brine. The organic phase was separated, washed by extraction with brine (3x) and dried over anhydrous sodium sulfate. Following the removal of the desiccant by filtration and evaporation of the solvent under reduced pressure, the residue was added to a mixture of 170 mg of 10% palladium on carbon, 21 mg (0.25 mmol) of sodium bicarbonate, 50 ml of deionized water, and 50 ml of ethyl acetate. The mixture was shaken under an atmosphere of hydrogen (initial pressure 56 psi, 386 kPa), at ambient temperature, on a Parr apparatus for 1 1/2 hour. The catalyst was filtered off through a celite pad on a fritted disc glass funnel, and the filter cake was washed with cold water. Solid sodium chloride was added to the combined filtrate and washings. The aqueous phase was separated, extracted with ethyl acetate (2x) and lyophilized. The residue was dissolved in 25 ml of deionized water and placed on three C18 Sep Paks in series. Following elution with a solvent gradient of water and acetonitrile (100% water to 50% acetonitrile/water) and lyophilizing, 23 mg of product was obtained.

Example 35

[2S-(2α,3α,5α,6β(R*))]-6-[[2-[[(4-Ethyl-2,3-dioxo-1-piperazinyl)carbonyl]amino]-2-phenylacetyl]amino]-3-[[(2,5-dihydro-6-hydroxy-2-methyl-5-oxo-1,2,4-triazine-3-yl)thio]methyl]-3-methyl-7-oxo-4-thia-1-azabicyclo[3.2.0]-heptane-2-carboxylic acid (4-nitrophenyl)methyl ester monosodium salt

(R)-α-[[(4-ethyl-2,3-dioxopiperazinyl)carbonyl]amino]phenylacetic acid (263.5 mg, 0.826 mmol) and 2-mercaptobenzothiazole (138.3 mg, 0.827 mmol) were dissolved in methylene chloride (11.7 ml). 1,3-Dicyclohexylcarbodiimide (171.3 mg, 0.830 mmol) was dissolved in methylene chloride (2.5 ml) and added to the first solution, which was then stirred at ambient temperature under nitrogen (dry). The free amine of [2S-(2α,3α,5α,6β)]-3-(bromomethyl)-3-methyl-7-oxo-6-amino-4-thia-1-azabicyclo[3.2.0]-heptane-2-carboxylic acid (4-nitrophenyl)methyl ester was simultaneously prepared according to the following procedure:

Phosphorus pentachloride (192.7 mg, 0.925 mmol) was dissolved in methylene chloride (3.3 ml). Pyridine (83.5 ml, 1.032 mmol) was then added (precipitate immediately formed) and the suspension was stirred and cooled at -15° C. After 5 to 10 minutes, [2S-(2α,3α,5α,6β)]-3-(bromomethyl)-3-methyl-7-oxo-6-[-(phenylacetyl)amino]-4-thia-1-azabicyclo[3.2.0]heptane-2-carboxylic acid (4-nitrophenyl)methyl ester (452.3 mg, 0.825 mmol) was added and stirred under nitrogen (dry). After 2 hours, the reaction mixture was transferred by pipet into a saturated sodium bicarbonate (12 ml) solution. The methylene chloride layer was then washed with more saturated sodium bicarbonate (7 ml) and finally to brine (15 ml). It was then dried over $Na_2SO_4$ in a test tube.

The solution was then transferred to the activated ester being prepared in the first part. Prior to the addition, the volume of the activated ester solution was reduced to 5 to 10 ml. The solution was stirred at ambient temperature under nitrogen. After 2 hours and 25 minutes, the volume was reduced to 5 to 10 ml and was filtered through celite and purified by flash chromatography using ethyl acetate in petroleum ether. Two fractions (84 mg about 80% pure and 115 mg about 33% pure by NMR) of [2S-[2α,3α,5α,6β(R*)]]-3-(bromomethyl)-6-[[-2-[[(4-ethyl-2,3-dioxo-1-piperazinyl)carbonyl]amino]-2-phenylacetyl]amino]-3-methyl-7-oxo-4-thia-1-azabicyclo[3.2.0]heptane-2-carboxylic acid (4-nitrophenyl)-methyl ester were obtained.

The above intermediate (80 mg, 0.109 mmol) was dissolved in acetone(1.3 ml), and water (0.63 ml) was then added. To this milky solution was added tetrahydro-2-methyl-3-thioxo-1,2,4-triazine-5,6-dione disodium salt, then acetone (1.74 ml). The clear yellow solution was stirred at ambient temperature under nitrogen. After 3 hours and 30 minutes, water (5 ml) was added and the acetone was evaporated (foaming occurred). The product was partly purified on C18 Sep Paks followed by HPLC using acetonitrile/water and a C18 column. Two fractions were collected, which weighed 26.9 mg and 3.9 mg, respectively.

## Example 36

[2S-(2α,3α,5α,6β(R*))]-6-[[2-[[(4-Ethyl-2,3-dioxo-1-piperazinyl)carbonyl]amino]-2-phenylacetyl]amino]-3-[[(2,5-dihydro-6-hydroxy-2-methyl-5-oxo-1,2,4-triazine-3-yl)thio]methyl]-3-methyl-7-oxo-4-thia-1-azabicyclo[3.2.0]-heptane-2-carboxylic acid disodium salt

[2S-(2α,3α,5α,6β(R*))]-6-[[2-[[(4-Ethyl-2,3-dioxo-1-piperazinyl)carbonyl]amino]-2-phenylacetyl]amino]-3-[-[(2,5-dihydro-6-hydroxy-2-methyl-5-oxo-1,2,4-triazine-3-yl)thio]methyl]-3-methyl-7-oxo-4-thia-1-azabicyclo-[3.2.0]heptane-2-carboxylic acid (4-nitrophenyl)methyl ester monosodium salt (40 mg, 0.0481 mmol) was dissolved in 0.1 N $NaHCO_3$ (0.46 ml, 0.046 mmol), and water (0.46 ml) was added, then ethyl acetate (1 ml) and 10% Pd/C(40 mg). The mixture was stirred at ambient temperature under hydrogen. After 2 hours, water (1 ml) and ethyl acetate (1 ml) were added and the suspension was filtered through celite. The water layer was removed and briefly stripped and it was then lyophilized to crude product (30.9 mg), which was purified by HPLC using acetonitrile/water, yielding two fractions (18.9 mg and 6.5 mg).

## Example 37

[2S-[2α,3α,5α,6β(R*)]]-3-[(Acetyl)thio)methyl]-3-methyl-6-[[2-[[4-ethyl-2,3-dioxo-1-piperazinyl)carbonyl]-amino]-2-phenylacetyl]amino]-7-oxo-4-thia-1-azabicyclo[3.2.0]heptane-2-carboxylic acid monosodium salt

To a dry flask containing an argon atmosphere was added [2S-(2α,3α,5α,6β)]-3-(bromomethyl)-3-methyl-7-oxo-6-[(phenylacetyl)amino]-4-thia-1-azabicyclo[3.2.0]heptane-2-carboxylic acid (4-nitrophenyl)-methyl ester (6.73 g, 12.27 mmol), $CH_2Cl_2$ (41 ml), and the mixture was cooled to -5° to -10° C. To this cooled solution was added pyridine (1.21 g, 15.35 mmol, 1.24 ml), followed 5 minutes later by the addition

of $PCl_5$ (2.808 g, 13.5 mmol) as a solid in one portion. After stirring at this temperature for 30 minutes, the reaction mixture was cooled to -15 to 20° C, and isobutanol (5.45 g,73.69 mmol, 6.78 ml) was added slowly along the inside wall of the flask over 5 minutes. After stirring for an additional 15 minutes, a saturated solution of NaBr (20 ml) at 0° C was added, and the reaction flask placed in a 0° C ice bath. After 5 minutes, $Et_2O$ (41 ml) was added slowly, causing the desired compound to precipitate from solution. The reaction mixture was allowed to stir rapidly for an additional 5 minutes, after which time the reaction mixture was filtered in a Buchner funnel and the solid was washed with $Et_2O$ until the product became granular in appearance and an odor of phosphorus halides was not detected any longer. The solid was transferred to a watch glass and dried in a desiccator containing $CaSO_4$ under vacuum. The solid obtained (4.77 g) contained about 50% of the desired 6-amine hydrobromide derivative, the major impurity being NaBr.

To a flask containing [2S-(2α,3α,5α,6β)]-6-amino-3-(bromomethyl)-3-methyl-7-oxo-4-thia-1-azabicyclo-[3.2.0]heptane-2-carboxylic acid (4-nitrophenyl)methyl ester, obtained from the amine hydrobromide derivative by treatment with $NaHCO_3$, acetone (12 ml) and water (4 ml), was added potassium thioacetate (0.34 g, 2.98 mmol). The reaction mixture was allowed to stir for 7 1/2 hours at room temperature, after which EtOAc (100 ml) was added and the mixture was transferred to a separatory funnel and washed with $H_2O$ (50 ml), $NaHCO_3$ (50 ml), $H_2O$ (30 ml) and brine, dried with $Na_2SO_4$, and concentrated to a volume of 1-2 ml. This solution was added to the reaction mixture described below.

To a dry flask containing an argon atmosphere was added (R)-α-[[(4-ethyl-2,3-dioxopiperazinyl)-carbonyl]amino]phenyl acetic acid (0.475 g, 1.49 mmol), DMF (5 ml) and 1-hydroxy-benzotriazole (0.201 g, 1.49 mmol), and the solution was cooled in a 0° C ice bath. After 5 minutes, dicyclohexylcarbodiimide (0.337 g 1.64 mmol) was added and the reaction was allowed to stir for an additional 10 minutes. At this point, the solution described in the preceding paragraph was added along with $CH_2Cl_2$ (2-3 ml). The resulting solution was allowed to stir overnight at room temperature. EtOAc was added (100 ml) and washed in a separatory funnel with $H_2O$ (3 x 100 ml), $NaHCO_3$ (50 ml) and brine (50 ml), and then dried over $Na_2SO_4$, filtered and concentrated in vacuo. The residue was purified by flash chromatography ($SiO_2$ (51 g), EtOAc), to give 0.375 g (35%) of [2S-[2α,3α,5α,6β(R*)]]-3-[(acetyl)thio)methyl]-3-methyl-6-[[2-[[4-ethyl-2,3-dioxo-1-piperazinyl)carbonyl]amino]-2-phenylacetyl]amino]-7-oxo-4-thia-1-azabicyclo[3.2.0]heptane-2-carboxylic acid (4-nitrophenyl)methyl ester as an off-white amorphous solid.

To a solution of [2S-[2α,3α,5α,6β(R*)]]-3-[(acetyl)thio)-methyl]-3-methyl-6-[[2-[[4-ethyl-2,3-dioxo-1-piperazinyl)-carbonyl]amino]-2-phenylacetyl]amino]-7-oxo-4-thia-1-azabicyclo[3.2.0]heptane-2-carboxylic acid (4-nitrophenyl)methyl ester (0.202 g, 0.278 mmol), EtOAc (6 ml) and aq. $NaHCO_3$ (0.1 M, 5.56 ml) was added 0.20 g of 10% Pd (C) and a magnetic stirring bar. The flask containing the rapidly stirring suspension was evacuated and filled with hydrogen gas three times. After stirring the reaction mixture for 2 hours at room temperature under a hydrogen atmosphere (delivered by balloon), the reaction mixture was filtered through a pad of celite, the EtOAc was removed in vacuo, and the resultant aqueous suspension was lyophilized. The solid obtained after lyophilization was purified by C18 chromatography with three Sep Paks connected in series and was eluted with a gradient of $CH_3CN/H_2O$ (0 - 100%). The proper fractions were combined (10 - 20% $CH_3CN/H_2O$), the $CH_3CN$ was removed in vacuo, and the resulting aqueous solution was lyophilized to give 0.063 g of the desired product as a white flocculent powder. This solid was purified by HPLC with a WHATMAN-M9 C18 column which was eluted with $CH_3CN/H_2O$ (15% to 50% gradient) and gave, after removal of the $CH_3CN$ and water, 0.034 g of [2S-[2α,3α,5α,6β(R*)]]-3-[(acetyl)thio)methyl]-3-methyl-6-[[2-[[4-ethyl-2,3-dioxo-1-piperazinyl)carbonyl]amino]-2-phenylacetyl]amino]-7-oxo-4-thia-1-azabicyclo[3.2.0]heptane-2-carboxylic acid monosodium salt.

## Example 38

**[2S-[2α,3α,5α,6β(R*)]]-3-(Fluoromethyl)-6-[[2-[[(4-ethyl-2,3-dioxo-1-piperazinyl)carbonyl]amino]-2-phenylacetyl]amino]-3-methyl-7-oxo-4-thia-1-azabicyclo[3.2.0]heptane-2-carboxylic acid monosodium salt**

A mixture of 137 mg (0.19 mmol) of [2S-[2α,3α,5α,6β(R*)]]-3-(bromomethyl)-6-[[2-[[(4-ethyl-2,3-dioxo-1-piperazinyl)carbonyl]amino]-2-phenylacetyl]amino]-3-methyl-7-oxo-4-thia-1-azabicyclo[3.2.0]heptane-2-carboxylic acid (4-nitrophenyl)methyl ester (reaction flask covered with aluminum foil), 30 ml of acetone and 24 mg (0.19 mmol) of silver fluoride in 3 ml of deionized water was stirred at room temperature for 5 1/2 hours. Following filtration through a millipore filter, most of the acetone was removed from the filtrate under reduced pressure. The residue was partitioned between ethyl acetate and brine. The organic phase was

separated, washed with brine (3x) and dried over sodium sulfate. After removing the desiccant by filtration and evaporation of the ethyl acetate under reduced pressure, the residue was added to a mixture of 40 mg of 10% palladium on carbon, 30 ml of deionized water, 10 mg (0.12 mmol) of sodium bicarbonate and 20 ml of ethyl acetate. The mixture was shaken under an atmosphere of hydrogen (initial pressure 56 psi, 386 kPa) at ambient temperature on a Parr apparatus for 3 hours. The catalyst was filtered off through a celite pad on a fritted disc glass funnel and the filter cake was washed with cold water. Solid sodium chloride (521.7 mg) was added to the combined filtrate and washings. The aqueous phase was separated, extracted with ethyl acetate and lyophilized. The residue was dissolved in 15 ml of deionized water and placed on three C18 Sep Paks connected in series. Following elution with a solvent gradient of water and acetonitrile (100% water to 50% acetonitrile/water) and lyophilizing, 10 mg of product was obtained.

## Example 39

[2S-[2α,3α,5α,6β(R*)]]-3-[[[3,4-bis(Acetyloxy)benzoyl]thio]-methyl]-6-[[2-[[(4-ethyl-2,3-dioxo-1-piperazinyl)-carbonyl]amino]-2-phenylacetyl]amino]-3-methyl-7-oxo-4-thia-1-azabicyclo[3.2.0]heptane-2-carboxylic acid monosodium salt

Phosphorus pentachloride (423.2 mg, 2.032 mmol) was dissolved in methylene chloride (5.73 ml), and pyridine (187 ml, 2.312 mmol) was then added (white precipitate formed). The mixture was cooled in an acetone/ice bath (-10° C to -15° C) under argon and, after 5 minutes, [2S-(2α,3α,5α,6β)]-3-(bromomethyl)-3-methyl-7-oxo-6-[(phenylacetyl)amino]-1-aza-4-thia-bicyclo[3.2.0]heptane-2-carboxylic acid (4-nitrophenyl)-methyl ester (1.0156 g, 1.852 mmol) was added. After 45 minutes, isobutanol (1.70 ml, 18.42 mmol) was added and the stirring was continued in the acetone/ice bath. After 40 minutes, sodium bromide solution (13 ml of solution of 70 g NaBr in 100 ml $H_2O$ solution) was added and stirred at ambient temperature. After five minutes, diethyl ether (6 ml) was added and vigorously stirred for a few minutes. The resulting mixture was filtered into a coarse scintered glass funnel and washed with sodium bromide solution (some cold and some ambient temperature). This was followed by four diethyl ether washes of 12.5 ml each. The solid was air dried on the funnel and redissolved in methylene chloride (5 ml) and saturated sodium bicarbonate (5 ml). The methylene chloride layer was dried over sodium sulfate and was then added to the activated ester which was simultaneously being prepared as follows:

2-Mercaptobenzothiazole (311.5 mg, 1.862 mmol) and (R)-α-[[4-ethyl-2,3-dioxopiperazinyl)carbonyl]-amino]phenylacetic acid (580.4 mg, 1.819 mmol) was dissolved in methylene chloride (24 ml). 1,3-Dicyclohexylcarbodiimide (385.6 mg, 1.869 mmol) was dissolved in methylene chloride (7 ml) and added to the first solution. The solution was stirred under argon at ambient temperature for 2 1/3 hours. The volume was reduced to one-quarter of the original and the free amine solution prepared as described above was added and stirred at ambient temperature under argon. After 2 hours and 10 minutes, the solution was filtered through celite and purified by flash chromatography using ethyl acetate in petroleum ether. Two fractions (72 mg and 11 mg) were obtained.

The purer fraction (72 mg, 0.098 mmol, 5.3% yield based on [2S-(2α,3α,5α,6β)]-3-(bromomethyl)-3-methyl-7-oxo-6-[[2-[[(4-ethyl-2,3-dioxo-1-piperazinyl)carbonyl]amino]-2-phenylacetyl]amino]-4-thia-1-azabicyclo[3.2.0]heptane-2-carboxylic acid (4-nitrophenyl)methyl ester and 3,4 bis (acetyloxy)-benzenecarbothioic acid (26.7 mg, 0.105 mmol) were dissolved in acetone and 0.1 N potassium bicarbonate (1 ml, 0.1 mmol). The mixture was stirred at ambient temperature under argon. After 30 minutes, ethyl acetate (20 ml) and saturated sodium bicarbonate (20 ml) were added. The ethyl acetate layer was dried over sodium sulfate (anhydrous) and then filtered and concentrated to a residue.

The residue was dissolved in tetrahydrofuran (5 ml) and 10% Pd on carbon (72.1 mg) was added. The reaction mixture was stirred at ambient temperature and pressure under hydrogen. After two hours, it was filtered through celite and 0.1 N sodium bicarbonate (0.93 ml, 0.093 mmol), and water (2 ml) was then added along with enough tetrahydrofuran to produce a clear solution. The tetrahydrofuran was stripped off and the aqueous portion was filtered through celite. The filtrate was passed through two C18 Sep Pak cartridges (in series) and eluted with water and mixtures of 5%, 10%, 20%, and 40% acetonitrile in water (10 ml of each, except 20% which was 15 ml and 5 ml fractions collected). Two sets of fractions were recovered (4.1 mg and 2.2 mg, total 0.0080 mmol, 0.43% from[2S-(2α,3α,5α,6β)]-3-(bromomethyl)-3-methyl-7-oxo-6-[(phenylacetyl)amino]-1-aza-4-thia-bicyclo[3.2.0]heptane-2-carboxylic acid (4-nitrophenyl)methyl ester. The product was further purified by HPLC using acetonitrile in water.

Example 40

[2S-[2α,3α,5α,6β(R*)]]-3-(Bromomethyl)-6-[[2-[[(4-ethyl-2,3-dioxo-1-piperazinyl)carbonyl]amino]-2-phenylacetyl]amino]-3-methyl-7-oxo-4-thia-1-azabicyclo[3.2.0]heptane-2-carboxylic acid (4-nitrophenyl)-methyl ester

A solution of 158 mg (0.496 mmol) of (R)-α-[[(4-ethyl-2,3-dioxopiperazinyl)carbonyl]amino]phenyl acetic acid, 83 mg (0.496 mmol) 2-mercaptobenzothiazole, 103 mg (0.5 mmol) of N, N′-dicyclohexylcarbodiimide and 25 ml of dry tetrahydrofuran was stirred at room temperature for 2 hours. A solution of 175 mg (0.41 mmol) of [2S-(2α,3α,5α,6β)]-6-amino-3-(bromomethyl)-3-methyl-7-oxo-4-thia-1-azabicyclo[3.2.0]heptane-2-carboxylic acid (4-nitrophenyl) methyl ester in 16 ml of dry methylene chloride was added dropwise at ambient temperature. The reaction mixture was stirred at room temperature an additional 2 hours and concentrated to dryness under reduced pressure. The residue was partitioned between ethyl acetate and dilute sodium bicarbonate. The organic phase was separated and, in turn, extracted with water (2x) and brine (1 x), then dried over anhydrous sodium sulfate. After removal of the desiccant by filtration and evaporation of the solvent under reduced pressure, the residue was chromatographed on silica gel (eluted with ethylacetate, neat), to give 250 mg of product.

Example 41

[2S-(2α,3α,5α,6β)]-6-[[[3,4-bis(Acetyloxy)benzoylamino]phenylacetyl]amino]-3-[[3,4-bis(acetyloxy)-benzoyloxy]methyl]-3-methyl-7-oxo-4-thia-1-azabicyclo-[3.2.0]heptane-2-carboxylic acid monosodium salt

A mixture of (3,4-bisacetoxybenzoylamino)-phenylacetic acid (0.055 g, 0.15 mmol), 1-hydroxy benzotriazole (0.025 g, 0.16 mmol) and N,N-dicyclohexylcarbodiimide (0.034 g, 0.16 mmol) in dry methylene chloride (5 ml) was stirred for 30 min. To the above solution was added [2S[2α,3α,5α,6β]]-3-[[[3,4-bis-(acetyloxy)benzoyl]oxymethyl]-6-amino-3-methyl-7-oxo-4-thia-1-azabicyclo [3.2.0] heptane-2-carboxylic acid (4-nitrophenyl)methyl ester (0.112 g, 0.18 mmol, as prepared in Example 31) and the reaction was stirred at room temperature for 3 h. A similar work-up and purification as described in Example 31 gave [2S-(2α,3α,5α,6β)]-6-[[[3,4-bis (acetyloxy)benzoylamino]phenyl-acetyl]amino]-3-[[3,4-bis (acetyloxy)benzoyloxy]-methyl]-3-methyl-7-oxo-4-thia-1-azabicyclo[3.2.0]heptane-2-carboxylic acid (4-nitrophenyl) methyl ester (33 mg, 26.6%).

The above ester (33 mg, 0.04 mmol) and 10% pd on charcoal (35 mg) in THF-$H_2O$ (1:1, 24 ml) was hydrogeneated with $H_2$ (45 psi, 310 kPa) at room temperature for 2 h. After converting to the sodium salt with sodium bicarbonate, the crude product was purified on $C_{18}$-column and the proper fractions were lyophilized to give the title compound (18.4 mg, 5.6%).

Example 42

[2S-[2α,3α,5α,6β(R*)]]-4-[[[2-Carboxy-6-[[2-[[(4-ethyl-2,3-dioxo-1-piperazinyl)carbonyl]amino]-2-phenylacetyl]amino]-3-methyl-7-oxo-4-thia-1-azabicyclo[3.2.0]hept-3-yl]thiol]methyl]-1-ethylpyridinium hydroxide inner salt

To a solution of [2S-[2α,3α,5α,6β(R*)]]-3-bromomethyl-6-[[2-[[(4-ethyl-2,3-dioxo-1-piperazinyl)carbonyl]-amino]-2-phenylacetyl]amino]-3-methyl-7-oxo-4-thia-1-azabicyclo[3.2.0]heptane-2-carboxylic acid (4-nitrophenyl)methyl ester (0.26 g, 0.35 mmol, Example 40) in DMF (13 ml) was added N-ethyl-4-thiopyridone (0.064 g, 0.46 mmol) at room temperature and the mixture was stirred at 45 °C for 8 hr. The solvent was removed and the residue was triturated with ethyl acetate. The precipitant was purified on silica gel column eluted with methylene chloride and then methylene chloride-methanol (9:1) containing 2% acetic acid to give [2S-[2α,3α,5α,6β(R*)]]-4-[[[6-[[2-[[(4-Ethyl-2,3-dioxo-1-piperazinyl)carbonyl]amino]-2-phenylacetyl]-

amino]-3-methyl-2-[[(4-nitrophenyl)methoxy]carbonyl]-7-oxo-4-thia-1-azabicyclo[3.2.0]hept-3-yl]methyl]thio]-1-ethyl pyridinium bromide (49 mg, 17%).

The above ester (15 mg, 0.019 mmol) and 10% pd on charcoal (15 mg) in THF-$H_2O$ (1:1, 10 ml) was hydrogenated with hydrogen (45 psi, 310 kPa) at room temperature for 2 h. Pruification through the $C_{18}$-column and lyophilizing the proper fractions gave the title compound (6.7 mg, 55.8%).

## Example 43

[2S-[2α,3α,5α,6β)]-4-[[[2-Carboxy-3-methyl-7-oxo-6-[(phenylacetyl)amino]-4-thia-1-azabicyclo[3.2.0]hept-3-yl]methyl]thio]-1-ethylpyridinium hydroxide inner salt

To a solution of [2S-[2α,3α,5α,6β)]-3- (bromomethyl)-3-methyl-7-oxo-6-[(phenylacetyl)amino]-4-thia-1-azabicyclo [3.2.0]heptane-2-carboxylic acid (4-nitrophenyl)methyl ester (0.274 g, 0.5 mmol) in DMF (4.8 ml) was added N-ethyl-4-thiopyridine (0.082 g, 0.58 mmol) at room temperature and the mixture was stirred overnight. The solvent was removed in vacuo and the residue was purified on preparative silica plates eluted with methylene chrloride-methanol (92:8) to give [2S-[2α,3α,5α,6β]]-4-[[[2-[[4-nitrophenylmethoxy]-carbonyl]-6-[(phenylacetyl)amino]-3-methyl-7-oxo-4-thia-1-azabicyclo [3.2.0]hept-3-yl] methyl]thio]-1-ethyl pyridinium bromide (0.108 g, 31%).

The above ester (0.144 g, 0.20 mmol) and 10% pd on charcoal (160 mg) in THF-$H_2O$ (1:1, 10 ml) was hydrogenated with $H_2$ (45 psi, 310 kPa) at room temperature for 2 h. Purification through the $C_{18}$-column and lyophilizing the proper fractions gave the title compound (36.7 mg, 37.5%).

## Example 44

[2S-[2α,3α,5α,6β)]-3-[[[3,4-bis(Acetyloxy)benzoyl]oxy]methyl]-6-[[(hexahydro-1H-azepin-1-yl)methylene]-amino]-7-oxo-4-thia-1-azabicyclo[3.2.0]heptane-2-carboxylic acid

To a mixture of p-toluenesulfonic acid (48.2 mg, 0.25 mmol) and N,N-diisopropyl-ethylamine (0.044 ml, 0.25 mmol) in methylene chloride (5.7 ml) was added [2S-[2α,3α,5α,6β]]-3-[[[3,4-bis(acetyloxy)benzoyl]oxy]-methyl]-6-amino-3-methyl-7-oxo-4-thia-1-azabicyclo[3.2.0]heptane-2-carboxylic acid (4-nitrophenyl)methyl ester (284 mg, 0.465 mmol, as prepared in Example 37) and stirred at room temperature under argon. Subsequently, N-formylhexamethyl-eneimine dimethyl acetal (0.32 ml, 1.83 mmol) was added to the above mixture and the reaction was stirred at same temperature for 1 hr. The reaction mixture was poured into ethyl acetate and washed with water, saturated sodium bicarbonate and brine. Solvent was removed ant the residue which was dissolved in THF-$H_2O$ (5.7 ml: 5.7 ml) was hydrogenated with 10% pd on charcoal (560 mg) and $H_2$ (1 atm) for 2 h. Catalyst was removed by filtration and the crude product was purified on $C_{18}$-column to give the title compound (33 mg, 13.5%).

## Example 45

[2S-[2α,3α,5α,6β]-3-[[[3,4-bis(Acetyloxy)benzoyl]oxy]methyl]-6-[[[(difluoromethyl)thio]acetyl]amino]-3-methyl-7-oxo-4-thia-1-azabicyclo[3.2.0]heptane-2-carboxylic acid monosodium salt

To a solution of [2S-[2α,3α,5α,6β]]-3-[[[3,4-bis (acetyloxy)benzoyl]oxy]-methyl]-6-amino-3-methyl-7-oxo-4-thia-1-azabicyclo[3.2.0]heptane-2-carboxylic acid (4-nitrophenyl)methyl ester (360 mg, 0.59 mmol, as prepared in Example 31) and pyridine (0.066 ml, 0.823 mmol) in methylene chloride (10 ml) was added difluoromethylthioacetyl chloride (117.3 mg, 0.73 mmol) and the resulting mixture were stirred for 5 h at room temperature. After the reaction, the solution was washed with dil. HCl, dil. $NaHCO_3$ solution and brine. The crude product was purified on silica gel column eluted with ethyl acetate-pet. ether (1:1) to give [2S-[2α,3α,5α,6β)]-3-[[[3.4-bis(acetyloxy)-benzoyl]oxy]methyl]-6-[[[(difluoromethyl)thio]acetyl]amino]-3-methyl-7-oxo-4-thia-1-azabicyclo[3.2.0]heptane-2-carboxylic acid (4-nitrophenyl)methyl ester (103 mg, 25.7%).

The above ester (87.3 mg, 0.128 mmol) was hydrogenated with 10% Pd on charcoal (92 mg) in THF:$H_2O$ (3.2 ml:2.2 ml) at 1 atm. of hydrogen. Product was purified on $C_{18}$-column to give the title compound (39 mg, 51%).

## Example 46

[2S-[2α,3α,5α,6β(R*)]]-3-[[[3,4-bis(Acetyloxy)benzoyl]oxy]methyl-6-[amino-(phenylacetyl)amino]-3-methyl-7-oxo-4-thia-1-azabicyclo[3.2.0]heptane-2-carboxylic acid monosodium salt

A mixture of (R)-N-(4-nitrobenzyloxy)carbonyl-phenyl glycine (147 mg, 0.445 mmol), 1-hydroxybenzotriazole (60 mg, 0.44 mmol) and N,N-dicyclohexylcarbodiimide (98.5 mg, 0.478 mmol) in dry methylene chloride (5 ml) was stirred for 2.5 h at room temperature. To the above solution was added [2S-[2α,3α,5α,6β]]-3-[[[3,4-bis(acetyloxy)benzoyl]oxy]methyl]-6-amino-3-methyl-7-oxo-4-thia-1-azabicyclo[3.2.0]-heptane-2-carboxylic acid (4-nitrophenyl) methyl ester (262 mg, 0.46 mmol, as prepared in Example 31) and the reaction was stirred at room temperature for 2 h. A similar work-up and purification as described in Example 31 gave [2S-[2α,3α,5α,6β(R*)]]-3-[[[3,4-bis-(acetyloxy)benzoyl]oxy]methyl]-6-[[(4-nitrobenzyloxy)-carbonyl]amino(phenylacetyl)amino]-3-methyl-7-oxo-4-thia-1-azabicyclo[3.2.0] heptane-2-carboxylic acid (4-nitrophenyl)methyl ester (146 mg, 37.6%).

The above ester (107.7 mg, 0.122 mmol) and 10% Pd on charcoal (100 mg) in THF-$H_2O$ (3 ml:3 ml) was hydrogenated with 1 atm. of hydrogen for 3 h. After converting to sodium salt with NaHCO3, the crude product was purified on $C_{18}$-column and the proper factions were lyophilized to give the title compound (25.6 mg, 33.6%).

## Example 47

[2S-[2S-[2α,3α,5α,6β(Z)]]-1-[6-[[[(2-Amino-4-thiazolyl)(methoxyimino)acetyl]-amino]-2-carboxy-3-methyl-7-oxo-4-thia-1-azabicyclo[3.2.0]hept-3-yl]methyl]-pyridinium hydroxide inner salt

To a solution of [2S-[2α,3α,5α,6β(Z)]]-6-[[(2-amino-4-thiazolyl)(methoxy-imino)acetyl]amino]-3-bromomethyl-3-methyl-7-oxo-4-thia-1-azabicyclo[3.2.0]-heptane-2-carboxylic acid (4-nitrophenyl)methyl ester (300 mg, 0.49 mmol, as prepared in Example 27) and pyridine (0.115 ml, 1.42 mmol) was added $AgBF_4$ (119 mg, 0.61 mmol) and the reaction was stirred for 4 h at room temperature. The reaction mixture was filtered and filtrate, after concentration in vacuo, was purified on $C_{18}$-column. The proper fractions were lyophilized to give [2S-[2α,3α,5α,6β(Z)]]-1-[6-[[[(2-amino-4-thiazolyl)(methoxyimino)acetyl]amino]-2-(4-nitrobenzyloxycarbonyl)-3-methyl-7-oxo-4-thia-1-azabicyclo[3.2.0]hept-3-yl]methyl]pyridinium tetrafluoroborate (87 mg, 25.4 %).

The above tetrafluoroborate (87 mg, 0.124 mmol) was dissolved in acetonitrile -$H_2O$ (4 ml:5 ml) and $NaHCO_3$ (0.1 N, 2.48 ml, 0.248 mmol) was added. Hydrogenation of the resulting mixture with 10% Pd on charcoal (96 mg) at 1 atm. of hydrogen for 2h yielded, after purification on $C_{18}$-column, the title compound (11.2 mg, 19.2%).

## Example 48

[2S-[2α,3α,5α,6β(R*)]]-1-[[2-Carboxy-6-[[[[(4-ethyl-2,3-dioxo-1-piperazinyl)-carbonyl]amino]phenylacetyl]-amino]-3-methyl-7-oxo-4-thia-1-azabicyclo[3.2.0]-hept-3-yl]methyl] pyridinium hydroxide inner salt

To a solution of [2S-[2α,3α,5α,6β (R*)]]-3-bromomethyl-6-[[[[(4-ethyl-2,3-dioxo-1-piperazinyl)carbonyl]-

amino]phenylacetyl]amino]-3-methyl-7-oxo-4-thia-1-azabicyclo[3.2.0]heptane-2-carboxylic acid (4-nitrophenyl)methyl ester (443 mg, 0.60 mmol, Example 40) and pyridine (0.149 ml, 1.84 mmol) in methylene chloride (8.7 ml) was added $AgBF_4$ (144 mg, 0.74 mmol) and the reaction was stirred for 3 h. Solvent was removed and the residue was purified on $C_{18}$-column eluted with a gradient of $CH_3CN$-$H_2O$ to give [2S-[2α,3α,5α,6β(R)]]-1-[[2-(4-nitrobenzyloxy carbonyl)-6-[[[[(4-ethyl-2,3-dioxo-1-piperazinyl)carbonyl]-amino]phenylacetyl]amino]-3-methyl-7-oxo-4-thia-1-azabicyclo[3.2.0]hept-3-yl]methyl] pyridinium tetrafluoroborate (113.6 mg 23%).

The above tetrafluorobonate (80 mg, 0.098 mmol) was dissolved in acetonitrile -$H_2O$ (3 ml:8.7 ml) and $NaHCO_3$ (0.1 N, 0.98 ml, 0.098 mmol) was added. Hydrogenation of the resulting mixture with 10% Pd on charcoal (80 mg) at atm of hydrogen for 3 h yielded, after purification on $C_{18}$-column, the title compound (21.3 mg, 36.7%).

Using procedures similar to those in Examples 31 and 32 above, but substituting the appropriate phenylacetic acid, the following compounds named in Examples 49-53 were prepared.

## Example 49

[2S-[2α,3α,5α,6β(S*)]]-3-[[[3,4-bis(acetyloxy)benzoyl]oxy]methyl]-6-[[[[(4-hydroxy-6-methyl-3-pyridinyl)-carbonyl]amino](4-hydroxy-phenyl)acetyl]amino-3-methyl-7-oxo-4-thia-1-azabicyclo[3.2.0]-heptane-2-carboxylic acid monosodium salt

## Example 50

[2S-[2α,3α,5α,6β(R*)]]-3-[[[3,4-bis(acetyloxy)benzoyl]oxy]methyl]-6-[[[[(4-hydroxy-6-methyl-3-pyridinyl)-carbonyl] amino](4-hydroxyphenyl)acetyl]amino-3-methyl-7-oxo-4-thia-1-azabicyclo[3.2.0]-heptane-2-carboxylic acid monosodium salt

## Example 51

[2S-[2α,3α,5α,6β(R*)]]-3-[[[3,4-bis(acetyloxy)benzoyl]oxy]methyl]-6-[[[[(4-ethyl-2,3-dioxo-1-piperazinyl)-carbonyl]amino](4-hydroxyphenyl)acetyl]amino-3-methyl-7-oxo-4-thia-1-azabicyclo[3.2.0]-heptane-2-carboxylic acid monosodium salt

## Example 52

[2S-[2α,3α,5α,6β(S*)]]-3-[[[3,4-bis(acetyloxy)benzoyl]oxy]methyl]-6-[[[[(4-ethyl-2,3-dioxo-1-piperazinyl) carbonyl]amino](4-hydroxyphenyl)acetyl]amino-3-methyl-7-oxo-4-thia-1-azabicyclo[3.2.0]-heptane-2-carboxylic acid monosodium salt

## Example 53

[2S-[2α,3α,5α,6β(R,S)]]-3-[[[3,4-bis(acetyloxy)benzoyl]oxy]methyl]-6-[[[3,4-bis(acetyloxy)phenyl][[(4-ethyl-2,3-dioxo-1-piperazinyl)carbonyl]amino]acetyl]amino]-3-methyl-7-oxo-4-thia-1-azabicyclo[3.2.0]-heptane-2-carboxylic acid monosodium salt

## Claims

1. A compound of the general formula

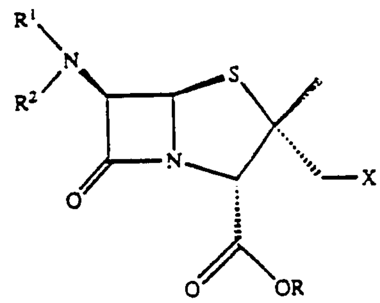

I

in which R is hydrogen, a readily hydrolyzable ester group or a protecting group, $R^1$ is hydrogen and $R^2$ is hydrogen or an acyl group, or $R^1$ and $R^2$ taken together are the group

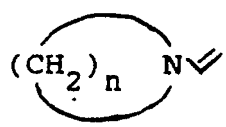

in which n is 5, 6 or 7,
X is fluoro, thiocyanato, azido or the group $R^{3a}CO_2$-

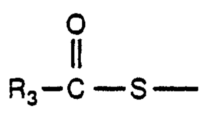 , 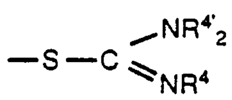 , $-SR^3$ , $-OR^3$

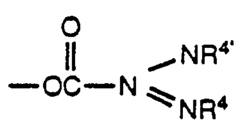 , 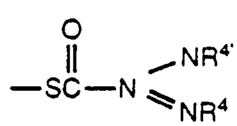 , 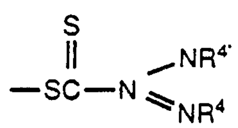 ,

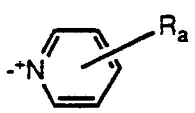 or 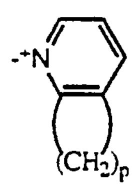 ,

$R^{3a}$ is $C_2$- to $C_{16}$-alkyl, aryl, a heterocycle, $-(CH_2)_m$-aryl, $-(CH_2)_m$-heterocycle, a heterocycle-lower alkenyl or aryl-lower alkenyl, $R^3$ is $C_1$- to $C_{16}$-alkyl, aryl, a heterocycle, $-(CH_2)_m$-aryl, a $-(CH_2)_m$-heterocycle, a heterocycle-lower alkenyl or aryl-lower alkenyl, $R^4$ and $R^{4'}$ independently are hydrogen, lower alkyl, lower alkenyl or lower alkynyl, $R_a$ is hydrogen, halogen, amino, amino-lower alkyl, carboxy, carbamoyl, carboxy-lower alkyl, carbamoyl-lower alkyl or lower carbalkoxy-lower alkyl, m is from 1 to 4 and p is 3, 4 or 5, provided that X is other than fluoro when R is a protecting group, a hydrate, a readily hydrolyzable ester or salt thereof, or a hydrate of the ester or the salt, when R is hydrogen.

2. A compound according to claim 1, in which R is a protecting group.
3. A compound according to claim 1, in which $R^1$ and $R^2$ are hydrogen.
4. A compound according to claim 1, in which $R^1$ is hydrogen and $R^2$ is acyl.
5. A compound according to claim 1, in which $R^1$ and $R^2$ taken together are the group

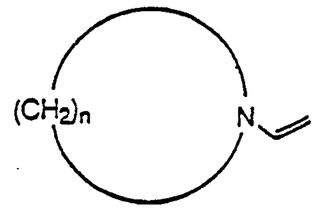

wherein n is 5, 6 or 7.

6. A compound according to claim 4 wherein the acyl group $R^2$ is an aliphatic acyl group of the formula

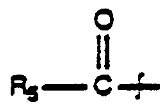

wherein $R_5$ is hydrogen, lower alkyl, lower cycloalkyl, lower alkoxy, lower alkenyl, lower cycloalkenyl, cyclohexadienyl or lower alkyl or lower alkenyl substituted with one or more halogen, cyano, nitro, amino, mercapto, alkylthio or cyanomethylthio groups.

7. A compound according to claim 4 wherein the acyl group $R^2$ is an aromatic acyl group selected from the group consisting of

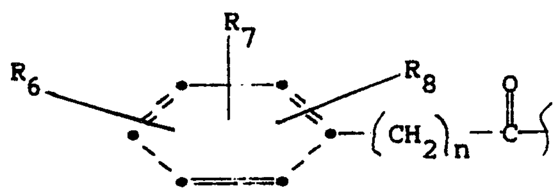

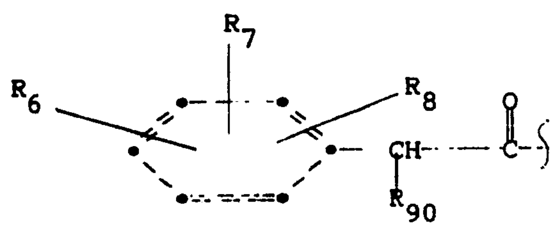

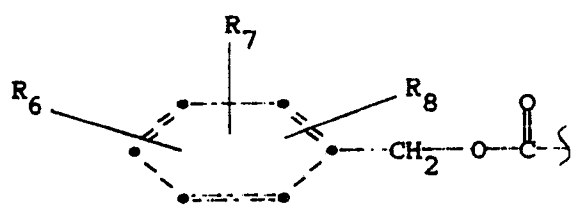

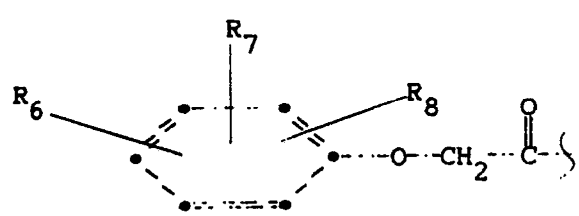

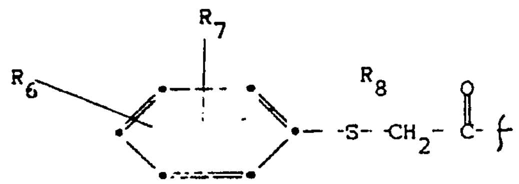

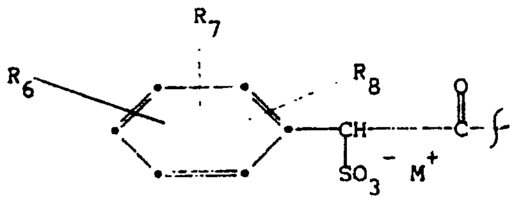

and

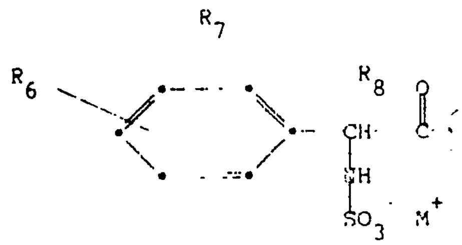

wherein n is 0, 1, 2 or 3, $R_6$, $R_7$ and $R_8$ are independently hydrogen, halogen, hydroxy, nitro, amino, cyano, trifluoromethyl, alkyl of 1 to 4 carbon atoms, alkoxy of 1 to 4 carbon atoms or aminomethyl and $R_{90}$ is amino, acylamino, hydroxy, a carboxy or sulfo salt, protected carboxy, formyloxy or azido.

8. A compound according to claim 4 wherein the acyl group $R^2$ is a heteroaromatic acyl group selected from the group consisting of

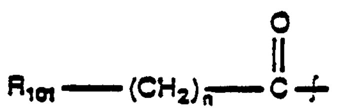

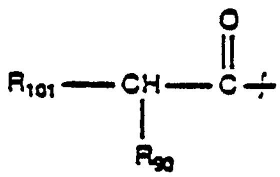

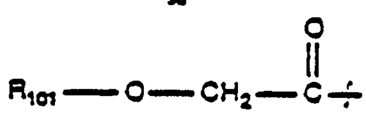

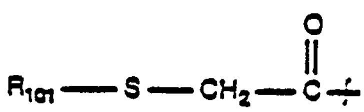

and

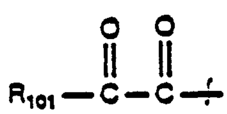

wherein n is 0, 1, 2 or 3, $R_{90}$ is as defined in claim 7 and $R^{101}$ is a substituted or unsubstituted 5-, 6- or 7-membered heterocyclic ring containing 1, 2, 3 or 4 hetero atoms selected from the group consisting of nitrogen, oxygen and sulfur.

9. A compound according to claim 4 wherein the acyl group $R^2$ is a [[(4-substituted-2,3-dioxo-1-piperazinyl) carbonyl]amino]acetyl group of the formula

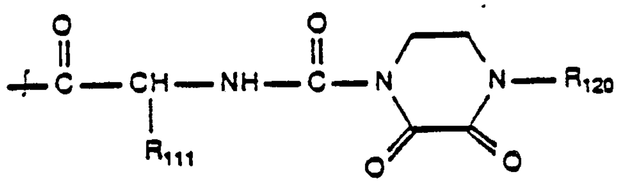

wherein $R_{111}$ is lower alkyl, lower hydroxyalkyl, an aromatic group of the formula

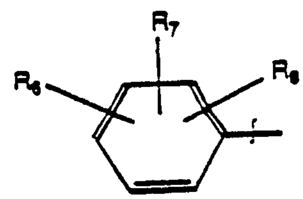

wherein $R_6$, $R_7$ and $R_8$ are as defined in claim 7, or a substituted or unsubstituted 5-, 6- or 7-membered heterocyclic ring containing 1, 2, 3 or 4 hetero atoms selected from the group consisting of nitrogen, oxygen and sulfur, and $R_{120}$ is lower alkyl or substituted lower alkyl (wherein the lower alkyl group is substituted with one or more halogen, cyano, nitro, amino or mercapto groups).

10. A compound according to claim 4 wherein the acyl group $R^2$ is a (acylamino) acetyl group having the formula

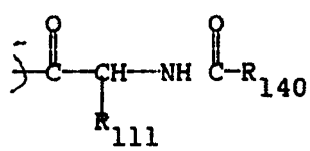

wherein $R_{111}$ is as defined in claim 9, and $R_{140}$ is an unsaturated heterocycle or a group of the formula

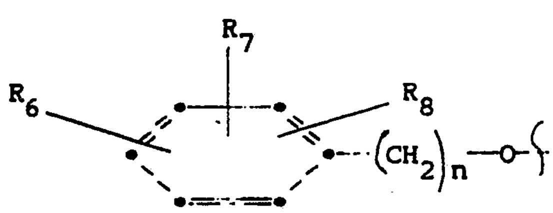

or

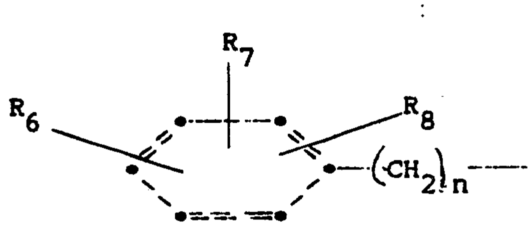

(wherein $R_6$, $R_7$ and $R_8$ are as defined in claim 7 and n is 0, 1, 2 or 3), hydrogen, lower alkyl, substituted lower alkyl, amino, lower alkylamino, di(loweralkyl)amino, (lower cyanoalkyl)amino, hydrazino, lower alkyl-hydrazino, aryl-hydrazino or acyl-hydrazino.

11. A compound according to claim 4 wherein the acyl group $R^2$ is a (substituted oxyimino) acetyl group having the formula

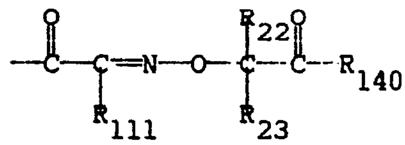

wherein $R_{111}$ is as defined in claim 9, $R_{140}$ is as defined in claim 10 and $R_{22}$ and $R_{23}$ are independently selected from the group consisting of hydrogen and lower alkyl, or $R_{22}$ and $R_{23}$ taken together with the carbon atom to which they are attached form a $C_3$-$C_7$-carbocyclic ring.

12. A compound according to claim 4 wherein the acyl group $R^2$ is a [[[3-substituted-2-oxo-1-imidazolidinyl]carbonyl]amino]acetyl group of the formula

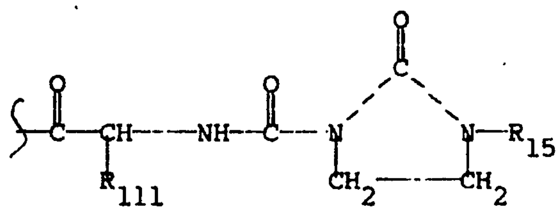

wherein $R_{111}$ is as defined in claim 9 and $R_{15}$ is hydrogen, lower alkylsulfonyl, arylmethyleneamino (i.e., -N=CHR$_{111}$ wherein $R_{111}$ is as defined in claim 9), -COR$_{16}$ (wherein $R_{16}$ is hydrogen, lower alkyl or halogen substituted lower alkyl), an aromatic group (as defined by $R_{111}$ in claim 9), lower alkyl or substituted lower alkyl (wherein the lower alkyl group is substituted with one or more halogen, cyano, nitro, amino or mercapto groups).

13. A compound according to claim 4 wherein the acyl group $R^2$ is an acyl group of the formula

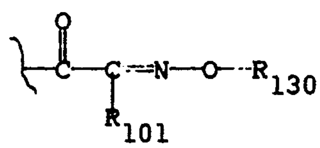

wherein $R_{101}$ is as defined in claim 8 and $R_{130}$ is hydrogen, lower alkyl, $C_3$-$C_7$-cycloalkyl or substituted lower alkyl [wherein the lower alkyl group is substituted with one or more halogen, cyano, nitro, amino, mercapto, lower alkylthio, aromatic groups (as defined by $R^{111}$ in claim 9), carboxy (including salts thereof), amido, carbamoyl, lower alkoxycarbonyl, phenylmethoxycarbonyl, diphenylmethoxycarbonyl, hydroxyalkoxyphosphinyl, dihydroxyphosphinyl, hydroxy(phenylmethoxy)phosphinyl or di(lower alkoxy)phosphinyl] or carboxy-$C_3$-$C_7$-cycloalkyl.

14. A compound according to any one of claims 1-13, wherein X is $R^{3a}CO_2$-, $R^3$-COS-, -SR$^3$ or -OR$^3$ and $R^{3a}$ and $R^3$ independently are aryl or a heterocycle.

15. [2S-(2a,3a,5a,6β)]-3-Methyl-3-[[(1-methyl-1H-tetrazol-5-yl)thio]methyl-7-oxo-6-[(phenylacetyl)amino]-4-thia-1-azabicyclo[3.2.0]heptane-2-carboxylic acid monosodium salt.

16. [2S-(2a,3a,5a,6β)]-3-[[(2,5-Dihydro-6-hydroxy-2-methyl-5-oxo-1,2,4-triazine-3-yl)thio]-methyl]-3-methyl-7-oxo-6-[(phenylacetyl)amino]-4-thia-1-azabicyclo[3.2.0]heptane-2-carboxylic acid disodium salt.

17. [2S-(2a,3a,5a,6β)]-3-[(Acetylthio)methyl]-3-methyl-7-oxo-6-[(phenylacetyl)amino]-4-thia-1-azabicyclo-[3.2.0]heptane-2-carboxylic acid monosodium salt.

18. [2S-(2α,3α,5α,6β)]-3-[[(2,5-Dihydro-3-mercapto-2-methyl-5-oxo-1,2,4-triazine-6-yl)oxy]methyl]-3-methyl-7-oxo-6-[(phenylacetyl)amino]-4-thia-1-azabicyclo-[3.2.0]heptane-2-carboxylic acid monosodium salt.

19. [2S-(2α,3α,5α,6β)]-3-[[(2-Furanylcarbonyl)thio]methyl]-7-oxo-6-[(phenylacetyl)amino]-4-thia-1-azabicyclo[3.2.0]-heptane-2-carboxylic acid monosodium salt.

20. [2S-(2α,3α,5α,6β)]-3-(Fluoromethyl)-3-methyl-7-oxo-6-[(phenyl-acetyl)amino]-4-thia-1-azabicyclo-[3.2.0]heptane-2-carboxylic acid sodium salt.

21. [2S-(2α,3α,5α,6β)]-3-[[[3,4-bis(Acetyloxy)benzoyl]thio]methyl]-3-methyl-7-oxo-6-[(phenylacetyl)-amino]-4-thia-1-azabicyclo-[3.2.0]heptane-2-carboxylic acid sodium salt.

22. [2S-(2α,3α,5α,6β)]-3-[[3,4-Dihydroxybenzoyl)oxy]methyl]-3-methyl-7-oxo-6-[(phenylacetyl)amino]-4-thia-1-azabicyclo-[3.2.0]heptane-2-carboxylic acid monosodium salt.

23. [2S-(2α,3α,5α,6β)]-3-[[3-(1H-Imidazol-4-yl)-1-oxopropoxy)]-methyl]-3-methyl-7-oxo-6-[(phenylacetyl)-

amino]-4-thia-1-azabicyclo[3.2.0]heptane-2-carboxylic acid sodium salt.

24. [2S-(2α,3α,5α,6β)]-1-[[2-Carboxy-3-methyl-7-oxo-6[(phenylacetyl)amino]-4-thia-1-azabicyclo[3.2.0]-hept-3-yl]methyl pyridinium hydroxide inner salt.

25. [2S-(2α,3α,5α,6β)]-3-[[[3,4-bis(Acetyloxy)benzoyl]oxy]methyl]-3-methyl-7-oxo-6-[(phenylacetyl)-amino]-4-thia-1-azabicyclo[3.2.0]heptane-2-carboxylic acid monosodium salt.

26. [2S-(2α,3α(E),5α,6β)]-3-[[[3-(1H-Imidazol-4-yl)-1-oxo-2-propenyl]oxy]methyl]-3-methyl-7-oxo-6-[-(phenylacetyl)amino]-4-thia-1-azabicyclo[3.2.0]heptane-2-carboxylic acid monosodium salt.

27. [2S-[2α,3α,5α,6β(Z)]]-6-[[(2-Amino-4-thiazolyl)(methoxyimino)-acetyl]amino]-3-methyl-3-[[(1-methyl-1H-tetrazol-5-yl)thio]methyl]-7-oxo-4-thia-1-azabicyclo[3.2.0]heptane-2-carboxylic acid monosodium salt.

28. [2S-[2α,3α,5α,6β(Z)]]-6-[[(2-Amino-4-thiazolyl)(methoxyimino)-acetyl]amino]-3-(azidomethyl)-3-methyl-7-oxo-4-thia-1-azabicyclo[3.2.0]heptane-2-carboxylic acid monosodium salt.

29. [2S-[2α,3α,5α,6β(Z)]]-3-[[[3,4-bis(Acetyloxy)benzoyl]-oxy]methyl]-6-[[(2-amino-4-thiazolyl)-(methoxyimino)acetyl]-amino]-3-methyl-7-oxo-4-thia-1-azabicyclo-[3.2.0]heptane-2-carboxylic acid monosodium salt.

30. [2S-[2α,3α,5α,6β(Z)]]-6-[[(2-Amino-4-thiazolyl)(methoxyimino)acetyl]amino]-3-[[(2,5-dihydro-6-hydroxy-2-methyl-5-oxo-1,2,4-triazine-3-yl)thio]methyl]-3-methyl-7-oxo-4-thia-1-azabicyclo[3.2.0]heptane-2-carboxylic acid disodium salt.

31. [2S-[2α,3α,5α,6β(R*)]]-6-[[[[(4-Ethyl-2,3-dioxo-1-piperazinyl)carbonyl]amino]phenylacetyl]amino]-3-methyl-3-[[(1-methyl-1H-tetrazole-5-yl)thio]methyl]-7-oxo-4-thia-1-azabicyclo[3.2.0]heptane-2-carboxylic acid monosodium salt sesquihydrate.

32. [2S-[2α,3α,5α,6β(R*)]]-3-[[[3,4-bis(Acetyloxy)benzoyl]oxy]methyl]-6-[[[[(4-ethyl-2,3-dioxo-1-piperazinyl)carbonyl]-amino]phenylacetyl]amino]-3-methyl-7-oxo-4-thia-1-azabicyclo-[3.2.0]-heptane-2-carboxylic acid monosodium salt.

33. [2S-[2α,3α,5α,6β(R*)]]-6-[[[[(4-Ethyl-2,3-dioxo-1-piperazinyl)-carbonyl]amino]-phenylacetyl]amino]-3-[[(3,4- dihydroxybenzoyl)oxy]methyl]-3-methyl-7-oxo-4-thia-1-azabicyclo[3.2.0]heptane-2-carboxylic acid monosodium salt.

34. [2S[2α,3α,5α,6β(R*)]]-6-[[[[(4-Ethyl-2,3-dioxo-1-piperazinyl)-carbonyl]amino]-phenylacetylamino]-3-[[-(2-furanylcarbonyl)thio]methyl]-3-methyl-7-oxo-4-thia-1-azabicyclo[3.2.0]heptane-2-carboxylic acid sodium salt.

35. [2S-[(2α,3α,5α,6β(R*))]-6-[[[[(4-Ethyl-2,3-dioxo-1-piperazinyl)carbonyl]amino]phenylacetyl[amino]-3-[[-(2,5-dihydro-6-hydroxy-2-methyl-5-oxo-1,2,4-triazine-3-yl)thio]methyl]-3-methyl-7-oxo-1-aza-4-thiabicyclo-[3.2.0]heptane-2-carboxylic acid disodium salt.

36. [2S-[2α,3α,5α,6β(R*)]]-3-(Acetyl)thio)methyl]-3-methyl-6-[[[[4-ethyl-2,3-dioxo-1-piperazinyl)carbonyl]-amino]phenylacetyl]amino]-7-oxo-4-thia-1-azabicyclo[3.2.0]heptane-2-carboxylic acid monosodium salt.

37. [2S-[2α,3α,5α,6β(R*)]]-3-(Fluoromethyl)-6[[[[(4-ethyl-2,3-dioxo-1-piperazinyl)carbonyl]amino]-phenylacetyl]amino]-3-methyl-7-oxo-4-thia-1-azabicyclo[3.2.0]-heptane-2-carboxylic acid monosodium salt.

38. [2S-[2α,3α,5α,6β(R*)]]-3-[[[3,4-bis(Acetyloxy)benzoyl]thio]methyl]-6-[[[[(4-ethyl-2,3-dioxo-1-piperazinyl)carbonyl]-amino]phenylacetyl]amino]-3-methyl-7-oxo-4-thia-1-azabicyclo-[3.2.0]-heptane-2-carboxylic acid monosodium salt.

39. [2S-[2α,3α,5α,6β(R*)]]-3-[[[3,4-bis(Acetyloxlybenzoyl]oxy]methyl-6-[amino(phenylacetyl)amino]-3-methyl-7-oxo-4-thia-1-azabicyclo[3.2.0]heptane-2-carboxylic acid monosodium salt.

40. [2S-[2α,3α,5α,6β(R*)]]-4-[[[2-Carboxy-6-[[[[(4-ethyl-2,3-dioxo-1-piperazinyl)carbonyl]amino]-phenylacetyl] amino-3-methyl-7-oxo-4-thia-1-azabicyclo[3.2.0]hept-3-yl]-thiol]methyl]-1-ethylpyridinium hydroxide inner salt.

41. [2S-[2α,3α,5α,6β)]-4-[[[2-Carboxy-3-methyl-7-oxo-6-[(phenylacetyl)amino]-4-thia-1-azabicyclo[3.2.0]-hept-3-yl]methyl]thio]-1-ethylpyridinium hydroxide inner salt.

42. [2S-[2α,3α,5α,6β)]-3-[[[3,4-bis(Acetyloxy)benzoyl]oxy]methyl]-6-[[(hexahydro-1H-azepin-1-yl)-methylene]amino]-7-oxo-4-thia-1-azabicyclo[3.2.0]heptane-2-carboxylic acid.

43. [2S-[2α,3α,5α,6β]-3-[[[3,4-bis(Acetyloxy)benzoyl]oxy]-methyl]-6-[[[(difluoromethyl)thio]acetyl]amino]-3-methyl-7-oxo-4-thia-1-azabicyclo[3.2.0]heptane-2-carboxylic acid monosodium salt.

44. [2S-[(2α,3α,5α,6β)]-6-[[[3,4-bis(Acetyloxy)benzoylamino]phenylacetyl]amino]-3-[[3,4-bis(acetyloxy)-benzoyloxy]methyl]-3-methyl-7-oxo-4-thia-1-azabicyclo-[3.2.0]heptane-2-carboxylic acid monosodium salt.

45. [2S-[2α,3α,5α,6β(Z)]]-1-[6-[[[(2-Amino-4-thiazolyl)-(methoxyimino)acetyl]-amino]-2-carboxy-3-methyl-7-oxo-4-thia-1-azabicyclo[3.2.0]hept-3-yl]methyl]pyridinium hydroxide inner salt.

46. [2S-[2α,3α,5α,6β(R*)]]-1-[[2-Carboxy-6-[[[[(4-ethyl-2,3-dioxo-1-piperazinyl)-carbonyl]amino]phenyl-acetyl]amino]-3-methyl-7-oxo-4-thia-1-azabicyclo[3.2.0]-hept-3-yl]methyl]-pyridinium hydroxide inner salt.

47. [2S-[2α,3α,5α,6β(S*)]]-3-[[[3,4-bis(acetyloxy)benzoyl]oxy]methyl]-6-[[[[(4-hydroxy-6-methyl-3-pyridinyl)carbonyl]-amino](4-hydroxy-phenyl)acetyl]amino-3-methyl-7-oxo-4-thia-1-azabicyclo[3.2.0]-heptane-2-carboxylic acid monosodium salt.

48. [2S-[2α,3α,5α,6β(R*)]]-3-[[[3,4-bis(acetyloxy)benzoyl]oxy]methyl]-6-[[[[(4-hydroxy-6-methyl-3-pyridinyl)carbonyl]amino](4-hydroxyphenyl)acetyl]amino-3-methyl-7-oxo-4-thia-1-azabicyclo[3.2.0]-heptane-2-carboxylic acid monosodium salt.

49. [2S-[2α,3α,5α,6β(R*)]]-3-[[[3,4-bis(acetyloxy)benzoyl]oxy]methyl]-6-[[[[(4-ethyl-2,3-dioxo-1-piperazinyl)carbonyl]amino](4-hydroxyphenyl)acetyl]amino-3-methyl-7-oxo-4-thia-1-azabicyclo[3.2.0]-heptane-2-carboxylic acid monosodium salt.

50. [2S-[2α,3α,5α,6β(S*)]]-3-[[[3,4-bis(acetyloxy)benzoyl]oxy]methyl]-6-[[[[(4-ethyl-2,3-dioxo-1-piperazinyl) carbonyl]amino](4-hydroxyphenyl)acetyl]amino-3-methyl-7-oxo-4-thia-1-azabicyclo[3.2.0]-heptane-2-carboxylic acid monosodium salt.

51. [2S-[2α,3α,5α,6β(R,S)]]-3-[[[3,4-bis(acetyloxy)benzoyl]oxy]methyl]-6-[[[3,4-bis(acetyloxy)phenyl][[(4-ethyl-2,3-dioxo-1-piperazinyl)carbonyl]amino]acetyl]amino]-3-methyl-7-oxo-4-thia-1-azabicyclo[3.2.0]-heptane-2-carboxylic acid monosodium salt

52. Compounds according to any one of claims 1-51 for use as therapeutically active substances.

53. Compounds according to any one of claims 1-51 for use as antibiotics.

54. A process for the manufacture of compounds according to any one of claims 1-51, which process comprises

a) reacting a compound of the general formula

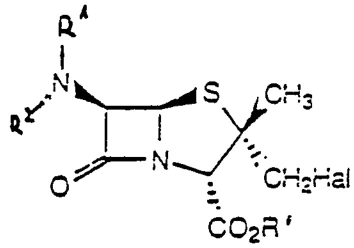

X

wherein Hal is halogen and R′ is a protecting group and $R^1$ and $R^2$ are as defined in claim 1, with a salt of a compound of the general formula HX, wherein X is as defined in claim 1, or

b) reacting a compound of the general formula

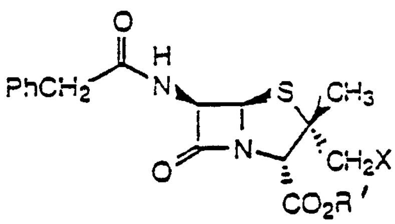

IV

wherein R′ is as defined above and X is as defined in claim 1, with $PCl_5$ or $PBr_5$ and pyridine or lutidine, or

c) reacting a compound of the general formula

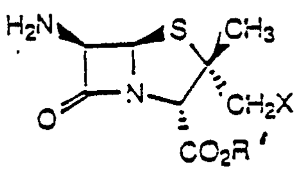

VI

wherein R′ is as defined above and X is as defined in claim 1, with a compound of the general formula

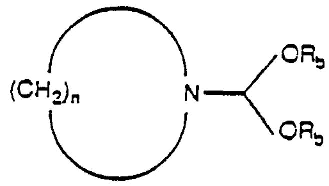

or

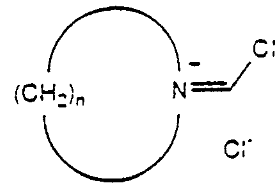

wherein n is as defined in claim 1 and Rb is lower alkyl, or

d) acylating a compound of the above general formula VI with a carboxylic acid or a reactive derivative thereof, and, if desired,

e) removing the ester protecting group in a compound obtained, wherein R is an ester protecting group, converting a compound obtained, wherein R is hydrogen, into a readily hydrolysable ester or a salt and/or converting a compound obtained, wherein R is hydrogen, or a readily hydrolysable ester or salt thereof into a hydrate.

55. A pharmaceutical composition containing a compound according to any one of claims 1-51 and a therapeutically inert carrier material.

56. An antibiotically active pharmaceutical composition containing a compound according to any one of claims 1-51 and a therapeutically inert carrier material.

57. The use of compounds according to any one of claims 1-51 in the control or prevention of illnesses.

58. The use of compounds according to any one of claims 1-51 in the control or prevention of infectious diseases.

59. The use of compounds according to any one of claims 1-51 for the manufacture of antibiotically active pharmaceutical compositions.

Claims for the following Contracting State: GR

1. A process for the manufacture of a compound of the general formula

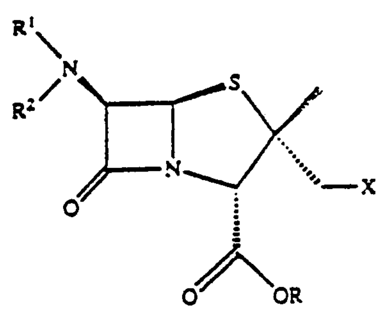

I

in which R is hydrogen, a readily hydrolyzable ester group or a protecting group, $R^1$ is hydrogen and $R^2$ is hydrogen or an acyl group, or $R^1$ and $R^2$ taken together are the group

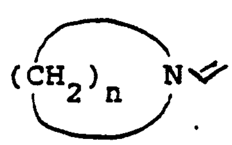

in which n is 5, 6 or 7,

X is fluoro, thiocyanato, azido or the group $R^{3a}CO_2$-

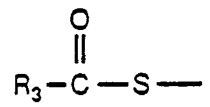 , 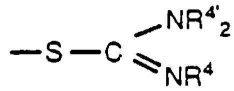 , $-SR^3$ , $-OR^3$

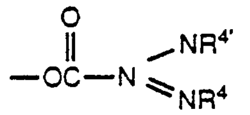 , 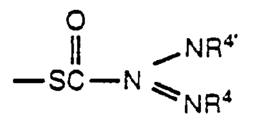 , 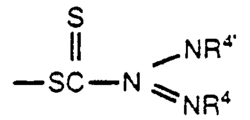 ,

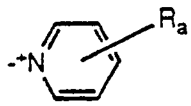 or 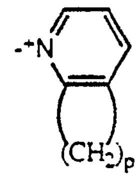 ,

$R^{3a}$ is $C_2$- to $C_{16}$-alkyl, aryl, a heterocycle, $-(CH_2)_m$-aryl, $-(CH_2)_m$-heterocycle, a heterocycle-lower alkenyl or aryl-lower alkenyl, $R^3$ is $C_1$- to $C_{16}$-alkyl, aryl, a heterocycle, $-(CH_2)_m$-aryl, a $-(CH_2)_m$-heterocycle, a heterocycle-lower alkenyl or aryl-lower alkenyl, $R^4$ and $R^{4'}$ independently are hydrogen, lower alkyl, lower alkenyl or lower alkynyl, $R_a$ is hydrogen, halogen, amino, amino-lower alkyl, carboxy, carbamoyl, carboxy-lower alkyl, carbamoyl-lower alkyl or lower carbalkoxy-lower alkyl, m is from 1 to 4 and p is 3, 4 or 5, provided that X is other than fluoro when R is a protecting group, a hydrate, a readily hydrolyzable ester or salt thereof, or a hydrate of the ester or the salt, when R is hydrogen, which process comprises,

a) reacting a compound of the general formula

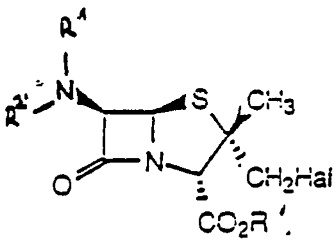

X

wherein Hal is halogen and $R'$ is a protecting group and $R^1$ and $R^2$ are as defined in claim 1, with a salt of a compound of the general formula HX, wherein X is as defined in claim 1, or

b) reacting a compound of the general formula

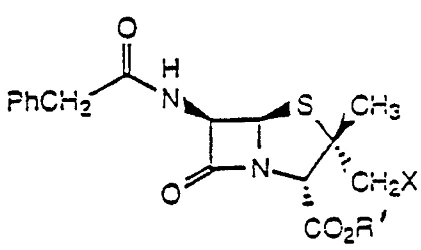

IV

wherein $R'$ is as defined above and X is as defined in claim 1, with $PCl_5$ or $PBr_5$ and pyridine or lutidine, or

c) reacting a compound of the general formula

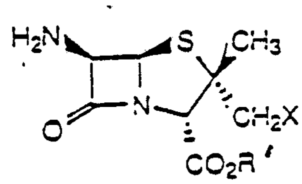

VI

wherein $R'$ is as defined above and X is as defined in claim 1, with a compound of the general formula

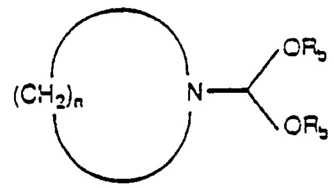

or

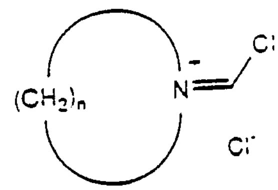

wherein n is as defined in claim 1 and Rb is lower alkyl, or

d) acylating a compound of the above general formula VI with a carboxylic acid or a reactive derivative thereof, and, if desired,

e) removing the ester protecting group in a compound obtained, wherein R is an ester protecting group, converting a compound obtained, wherein R is hydrogen, into a readily hydrolysable ester or a salt and/or converting a compound obtained, wherein R is hydrogen, or a readily hydrolysable ester or salt thereof into a hydrate.

2. A process according to claim 1, in which R is a protecting group.

3. A process according to claim 1, in which $R^1$ and $R^2$ are hydrogen.

4. A process according to claim 1, in which $R^1$ is hydrogen and $R^2$ is acyl.

5. A process according to claim 1, in which $R^1$ and $R^2$ taken together are the group

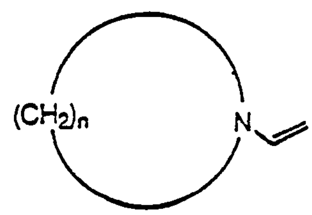

wherein n is 5, 6 or 7.

6. A process according to claim 4 wherein the acyl group $R^2$ is an aliphatic acyl group of the formula

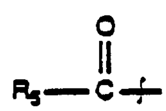

wherein $R_5$ is hydrogen, lower alkyl, lower cycloalkyl, lower alkoxy, lower alkenyl, lower cycloalkenyl, cyclohexadienyl or lower alkyl or lower alkenyl substituted with one or more halogen, cyano, nitro, amino, mercapto, alkylthio or cyanomethylthio groups.

7. A process according to claim 4 wherein the acyl group $R^2$ is an aromatic acyl group selected from the group consisting of

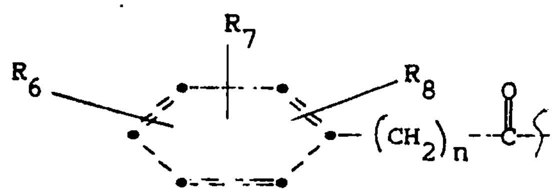

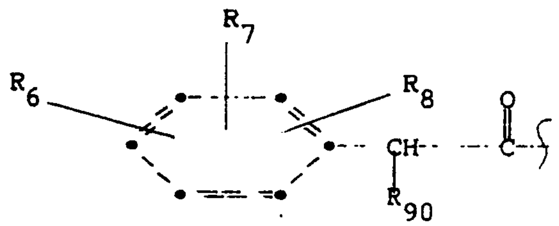

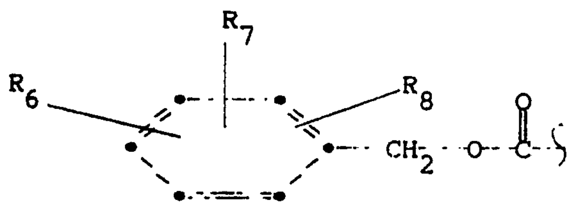

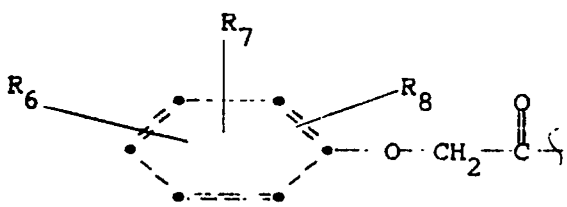

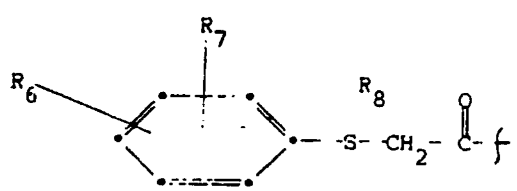

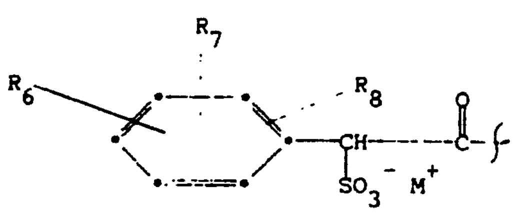 and 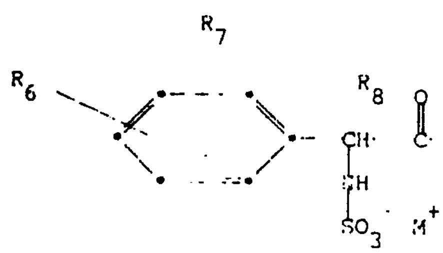

wherein n is 0, 1, 2 or 3, $R_6$, $R_7$ and $R_8$ are independently hydrogen, halogen, hydroxy, nitro, amino, cyano, trifluoromethyl, alkyl of 1 to 4 carbon atoms, alkoxy of 1 to 4 carbon atoms or aminomethyl and $R_{90}$ is amino, acylamino, hydroxy, a carboxy or sulfo salt, protected carboxy, formyloxy or azido.

8. A process according to claim 4 wherein the acyl group $R^2$ is a heteroaromatic acyl group selected from the group consisting of

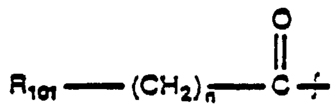

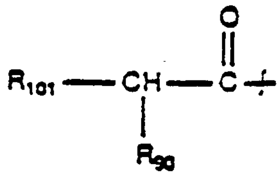

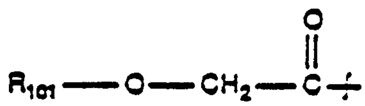

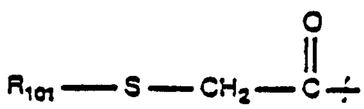

and

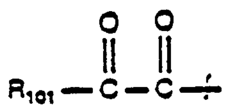

wherein n is 0, 1, 2 or 3, $R_{90}$ is as defined in claim 7 and $R^{101}$ is a substituted or unsubstituted 5-, 6- or 7-membered heterocyclic ring containing 1, 2, 3 or 4 hetero atoms selected from the group consisting of nitrogen, oxygen and sulfur.

9. A process according to claim 4 wherein the acyl group $R^2$ is a [[(4-substituted-2, 3-dioxo-1-piperazinyl) carbonyl]amino]acetyl group of the formula

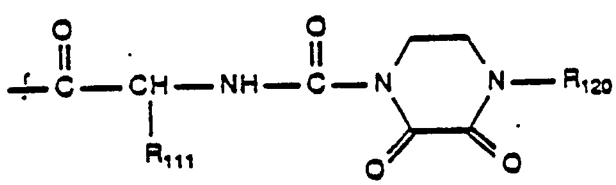

wherein $R_{111}$ is lower alkyl, lower hydroxyalkyl, an aromatic group of the formula

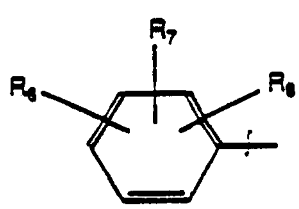

wherein $R_6$, $R_7$ and $R_8$ are as defined in claim 7, or a substituted or unsubstituted 5-, 6- or 7-membered heterocyclic ring containing 1, 2, 3 or 4 hetero atoms selected from the group consisting of nitrogen, oxygen and sulfur, and $R_{120}$ is lower alkyl or substituted lower alkyl (wherein the lower alkyl group is substituted with one or more halogen, cyano, nitro, amino or mercapto groups).

10. A process according to claim 4 wherein the acyl group $R^2$ is a (acylamino) acetyl group having the formula

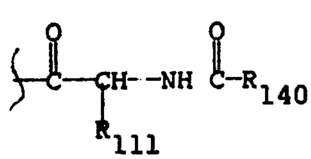

wherein $R_{111}$ is as defined in claim 9, and $R_{140}$ is an unsaturated heterocycle or a group of the formula

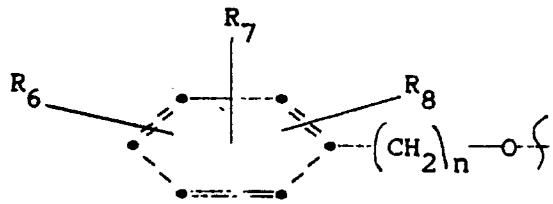

or

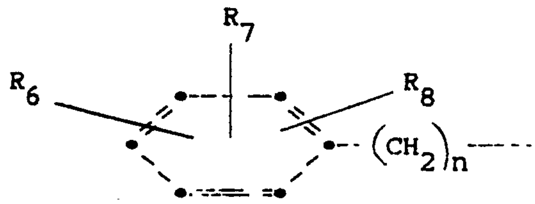

(wherein $R_6$, $R_7$ and $R_8$ are as defined in claim 7 and n is 0, 1, 2 or 3), hydrogen, lower alkyl, substituted lower alkyl, amino, lower alkylamino, di(loweralkyl)amino, (lower cyanoalkyl)amino, hydrazino, lower alkyl-hydrazino, aryl-hydrazino or acyl-hydrazino.

11. A process according to claim 4 wherein the acyl group $R^2$ is a (substituted oxyimino) acetyl group having the formula

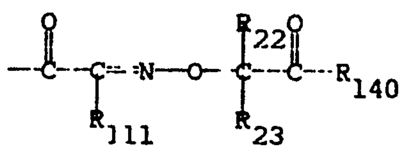

wherein $R_{111}$ is as defined in claim 9, $R_{140}$ is as defined in claim 10 and $R_{22}$ and $R_{23}$ are independently selected from the group consisting of hydrogen and lower alkyl, or $R_{22}$ and $R_{23}$ taken together with the carbon atom to which they are attached form a $C_3$-$C_7$-carbocyclic ring.

12. A process according to claim 4 wherein the acyl group $R^2$ is a [[[3-substituted-2-oxo-1-imidazolidinyl]carbonyl]amino]acetyl group of the formula

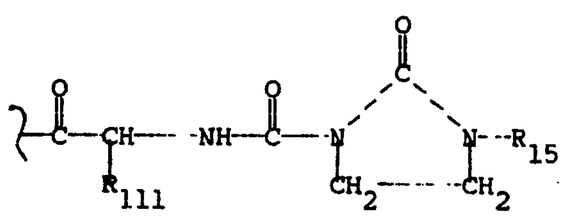

wherein $R_{111}$ is as defined in claim 9 and $R_{15}$ is hydrogen, lower alkylsulfonyl, arylmethyleneamino (i.e., $-N=CHR_{111}$ wherein $R_{111}$ is as defined in claim 9), $-COR_{16}$ (wherein $R_{16}$ is hydrogen, lower alkyl or halogen substituted lower alkyl), an aromatic group (as defined by $R_{111}$ in claim 9), lower alkyl or substituted lower alkyl (wherein the lower alkyl group is substituted with one or more halogen, cyano, nitro, amino or mercapto groups).

13. A process according to claim 4 wherein the acyl group $R^2$ is an acyl group of the formula

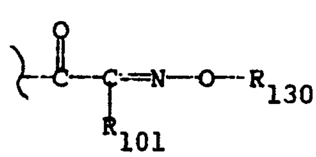

wherein $R_{101}$ is as defined in claim 8 and $R_{130}$ is hydrogen, lower alkyl, $C_3$-$C_7$-cycloalkyl or substituted lower alkyl [wherein the lower alkyl group is substituted with one or more halogen, cyano, nitro, amino, mercapto, lower alkylthio, aromatic groups (as defined by $R^{111}$ in claim 9), carboxy (including salts thereof), amido, carbamoyl, lower alkoxycarbonyl, phenylmethoxycarbonyl, diphenylmethoxycarbonyl, hydroxyalkoxyphosphinyl, dihydroxyphosphinyl, hydroxy(phenylmethoxy)phosphinyl or di(lower alkoxy)phosphinyl] or carboxy-$C_3$-$C_7$-cycloalkyl.

14. A process according to any one of claims 1-13, wherein X is $R^{3a}CO_2$-, $R^3$-COS-, -$SR^3$ or $OR^3$ and $R^{3a}$ and $R^3$ independently are aryl or a heterocycle.

15. A process in accordance with claim 1 wherein the following compound is prepared: [2S-(2a,3a,5a,6β)]-3-Methyl-3-[[(1-methyl-1H-tetrazol-5-yl)thio]methyl-7-oxo-6-[(phenylacetyl)amino]-4-thia-1-azabicyclo[3.2.0]heptane-2-carboxylic acid monosodium salt.

16. A process in accordance with claim 1 wherein the following compound is prepared: [2S-(2a,3a,5a,6β)]-3-[[(2,5-Dihydro-6-hydroxy-2-methyl-5-oxo-1,2,4-triazine-3-yl)thio]-methyl]-3-methyl-7-oxo-6-[(phenylacetyl)amino]-4-thia-1-azabicyclo[3.2.0]heptane-2- carboxylic acid disodium salt.

17. A process in accordance with claim 1 wherein the following compound is prepared: [2S-(2α,3α,5α,6β)]-3-[(Acetylthio)methyl]-3-methyl-7-oxo-6-[(phenylacetyl)amino]-4-thia-1-azabicyclo[3.2.0]-heptane-2-carboxylic acid monosodium salt.

18. A process in accordance with claim 1 wherein the following compound is prepared: [2S-(2α,3α,5α,6β)]-3-[[(2,5-Dihydro-3-mercapto-2-methyl-5-oxo-1,2,4-triazine-6-yl)oxy]methyl]-3-methyl-7-oxo-6-[(phenylacetyl)amino]-4-thia-1-azabicyclo-[3.2.0]heptane-2-carboxylic acid monosodium salt.

19. A process in accordance with claim 1 wherein the following compound is prepared: [2S-(2α,3α,5α,6β)]-3-[[(2-Furanylcarbonyl)thio]methyl]-7-oxo-6-[(phenylacetyl)amino]-4-thia-1-azabicyclo-[3.2.0]-heptane-2-carboxylic acid monosodium salt.

20. A process in accordance with claim 1 wherein the following compound is prepared: [2S-(2α,3α,5α,6β)]-3-(Fluoromethyl)-3-methyl-7-oxo-6-[(phenyl-acetyl)amino]-4-thia-1-azabicyclo[3.2.0]-heptane-2-carboxylic acid sodium salt.

21. A process in accordance with claim 1 wherein the following compound is prepared: [2S-(2α,3α,5α,6β)]-3-[[[3,4-bis(Acetyloxy)benzoyl]thio]methyl]-3-methyl-7-oxo-6-[(phenylacetyl)amino]-4-thia-1-azabicyclo-[3.2.0]heptane-2-carboxylic acid sodium salt.

22. A process in accordance with claim 1 wherein the following compound is prepared: [2S-(2α,3α,5α,6β)]-3-[[3,4-Dihydroxybenzoyl)oxy]methyl]-3-methyl-7-oxo-6-[(phenylacetyl)amino]-4-thia-1-azabicyclo-[3.2.0]heptane-2-carboxylic acid monosodium salt.

23. A process in accordance with claim 1 wherein the following compound is prepared: [2S-(2α,3α,5α,6β)]-3-[[3-(1H-Imidazol-4-yl)-1-oxopropoxy)]-methyl]-3-methyl-7-oxo-6-[(phenylacetyl)amino]-4-thia-1-azabicyclo[3.2.0]heptane-2-carboxylic acid sodium salt.

24. A process in accordance with claim 1 wherein the following compound is prepared: [2S-(2α,3α,5α,6β)]-1-[[2-Carboxy-3-methyl-7-oxo-6[(phenylacetyl)amino]-4-thia-1-azabicyclo[3.2.0]hept-3-yl]-methyl pyridinium hydroxide inner salt.

25. A process in accordance with claim 1 wherein the following compound is prepared: [2S-(2α,3α,5α,6β)]-3-[[[3,4-bis(Acetyloxy)benzoyl]oxy]methyl]-3-methyl-7-oxo-6-[(phenylacetyl)amino]-4-thia-1-azabicyclo[3.2.0]heptane-2-carboxylic acid monosodium salt.

26. A process in accordance with claim 1 wherein the following compound is prepared: [2S-(2α,3α(E),5α,6β)]-3-[[[3-(1H-Imidazol-4-yl)-1-oxo-2-propenyl]oxy]methyl]-3-methyl-7-oxo-6-[-(phenylacetyl)amino]-4-thia-1-azabicyclo[3.2.0]heptane-2-carboxylic acid monosodium salt.

27. A process in accordance with claim 1 wherein the following compound is prepared: [2S-(2α,3α,5α,6β(Z)]-6-[[(2-Amino-4-thiazolyl)(methoxyimino)-acetyl]amino]-3-methyl-3-[[(1-methyl-1H-tetrazol-5-yl)thio]methyl]-7-oxo-4-thia-1-azabicyclo[3.2.0]heptane-2-carboxylic acid monosodium salt.

28. A process in accordance with claim 1 wherein the following compound is prepared: [2S-[2α,3α,5α,6β(Z)]]-6-[[(2-Amino-4-thiazolyl)(methoxyimino)-acetyl]amino]-3-(azidomethyl)-3-methyl-7-oxo-4-thia-1-azabicyclo[3.2.0]heptane-2-carboxylic acid monosodium salt.

29. A process in accordance with claim 1 wherein the following compound is prepared: [2S-[2α,3α,5α,6β(Z)]]-3-[[[3,4-bis(Acetyloxy)benzoyl]-oxy]methyl]-6-[[(2-amino-4-thiazolyl)(methoxyimino)-acetyl]-amino]-3-methyl-7-oxo-4-thia-1-azabicyclo-[3.2.0]heptane-2-carboxylic acid monosodium salt.

30. A process in accordance with claim 1 wherein the following compound is prepared: [2S-[2α,3α,5α,6β(Z)]]-6-[[(2-Amino-4-thiazolyl)(methoxyimino)acetyl]amino]-3-[[(2,5-dihydro-6-hydroxy-2-methyl-5-oxo-1,2,4-triazine-3-yl)thio]methyl]-3-methyl-7-oxo-4-thia-1-azabicyclo[3.2.0]heptane-2-carboxylic acid disodium salt.

31. A process in accordance with claim 1 wherein the following compound is prepared:

[2S-[2α,3α,5α,6β(R*)]]-6-[[[[(4-Ethyl-2,3-dioxo-1-piperazinyl)carbonyl]amino]phenylacetyl]amino]-3-methyl-3-[[(1-methyl-1H-tetrazole-5-yl)thio]methyl]-7-oxo-4-thia-1-azabicyclo[3.2.0]heptane-2-carboxylic acid monosodium salt sesquihydrate.

32. A process in accordance with claim 1 wherein the following compound is prepared: [2S-[2α,3α,5α,6β(R*)]]-3-[[[3,4-bis(Acetyloxy)benzoyl]oxy]methyl]-6-[[[[(4-ethyl-2,3-dioxo-1-piperazinyl)-carbonyl]-amino]phenylacetyl]amino]-3-methyl-7-oxo-4-thia-1-azabicyclo-[3.2.0]-heptane-2-carboxylic acid monosodium salt.

33. A process in accordance with claim 1 wherein the following compound is prepared: [2S-[2α,3α,5α,6β(R*)]]-6-[[[[(4-Ethyl-2,3-dioxo-1-piperazinyl)-carbonyl]amino]-phenylacetyl]amino]-3-[[(3,4-dihydroxybenzoyl)oxy]methyl]-3-methyl-7-oxo-4-thia-1-azabicyclo[3.2.0]heptane-2-carboxylic acid monosodium salt.

34. A process in accordance with claim 1 wherein the following compound is prepared: [2S[2α,3α,5α,6β(R*)]]-6-[[[[(4-Ethyl-2,3-dioxo-1-piperazinyl)-carbonyl]amino]-phenylacetylamino]-3-[[(2-furanylcarbonyl)thio]methyl]-3-methyl-7-oxo-4-thia-1-azabicyclo[3.2.0]heptane-2-carboxylic acid sodium salt.

35. A process in accordance with claim 1 wherein the following compound is prepared: [2S-(2α,3α,5α,6β(R*)]]-6-[[[[(4-Ethyl-2,3-dioxo-1-piperazinyl)carbonyl]amino]phenylacetyl[amino]-3-[[(2,5-dihydro-6-hydroxy-2-methyl-5-oxo-1,2,4-triazine-3-yl)thio]methyl]-3-methyl-7-oxo-1-aza-4-thiabicyclo[3.2.0]-heptane-2-carboxylic acid disodium salt.

36. A process in accordance with claim 1 wherein the following compound is prepared: [2S-[2α,3α,5α,6β(R*)]]-3-[(Acetyl)thio)methyl]-3-methyl-6-[[[[4-ethyl-2,3-dioxo-1-piperazinyl)carbonyl]amino]-phenylacetyl]amino]-7-oxo-4-thia-1-azabicyclo[3.2.0]heptane-2-carboxylic acid monosodium salt.

37. A process in accordance with claim 1 wherein the following compound is prepared: [2S-[2α,3α,5α,6β(R*)]]-3-(Fluoromethyl)-6[[[[(4-ethyl-2,3-dioxo-1-piperazinyl)carbonyl]amino]phenylacetyl]-amino]-3-methyl-7-oxo-4-thia-1-azabicyclo[3.2.0]-heptane-2-carboxylic acid monosodium salt.

38. A process in accordance with claim 1 wherein the following compound is prepared: [2S-[2α,3α,5α,6β(R*)]]-3-[[[3,4-bis(Acetyloxy)benzoyl]thio]methyl]-6-[[[[(4-ethyl-2,3-dioxo-1-piperazinyl)-carbonyl]-amino]phenylacetyl]amino]-3-methyl-7-oxo-4-thia-1-azabicyclo-[3.2.0]-heptane-2-carboxylic acid monosodium salt.

39. A process in accordance with claim 1 wherein the following compound is prepared: [2S-[2α,3α,5α,6β(R*)]]-3-[[[3,4-bis(Acetyloxy)benzoyl]oxy]methyl-6-[amino(phenylacetyl)amino]-3-methyl-7-oxo-4-thia-1-azabicyclo[3.2.0]heptane-2-carboxylic acid monosodium salt.

40. A process in accordance with claim 1 wherein the following compound is prepared: [2S-[2α,3α,5α,6β(R*)]]-4-[[[2-Carboxy-6-[[[[(4-ethyl-2,3-dioxo-1-piperazinyl)carbonyl]amino]-phenylacetyl]-amino]-3-methyl-7-oxo-4-thia-1-azabicyclo[3.2.0]hept-3-yl]-thiol]methyl]-1-ethylpyridinium hydroxide inner salt.

41. A process in accordance with claim 1 wherein the following compound is prepared: [2S-[2α,3α,5α,6β)]-4-[[[2-Carboxy-3-methyl-7-oxo-6-[(phenylacetyl)amino]-4-thia-1-azabicyclo[3.2.0]hept-3-yl]methyl]thio]-1-ethylpyridinium hydroxide inner salt.

42. A process in accordance with claim 1 wherein the following compound is prepared: [2S-[2α,3α,5α,6β)]-3-[[[3,4-bis(Acetyloxy)benzoyl]oxy]methyl]-6-[[(hexahydro-1H-azepin-1-yl)-methylene]-amino]-7-oxo-4-thia-1-azabicyclo[3.2.0]heptane-2-carboxylic acid.

43. A process in accordance with claim 1 wherein the following compound is prepared: [2S-[2α,3α,5α,6β]-3-[[[3,4-bis(Acetyloxy)benzoyl]oxy]-methyl]-6-[[[(difluoromethyl)thio]acetyl]amino]-3-methyl-7-oxo-4-thia-1-azabicyclo[3.2.0]heptane-2-carboxylic acid monosodium salt.

44. A process in accordance with claim 1 wherein the following compound is prepared: [2S-(2α,3α,5α,6β)]-6-[[[3,4-bis(Acetyloxy)benzoylamino]phenylacetyl]amino]-3-[[3,4-bis(acetyloxy)-benzoyloxy]methyl]-3-methyl-7-oxo-4-thia-1-azabicyclo-[3.2.0]heptane-2-carboxylic acid monosodium salt.

45. A process in accordance with claim 1 wherein the following compound is prepared: [2S-[2α,3α,5α,6β(Z)]]-1-[6-[[[(2-Amino-4-thiazolyl)-(methoxyimino)acetyl]-amino]-2-carboxy-3-methyl-7-oxo-4-thia-1-azabicyclo[3.2.0]hept-3-yl]methyl]pyridinium hydroxide inner salt.

46. A process in accordance with claim 1 wherein the following compound is prepared: [2S-[2α,3α,5α,6β(R*)]]-1-[[2-Carboxy-6-[[[[(4-ethyl-2,3-dioxo-1-piperazinyl)-carbonyl]amino]phenyl-acetyl]-amino]-3-methyl-7-oxo-4-thia-1-azabicyclo[3.2.0]-hept-3-yl]methyl]-pyridinium hydroxide inner salt.

47. A process in accordance with claim 1 wherein the following compound is prepared: [2S-[2α,3α,5α,6β(S*)]]-3-[[[3,4-bis(acetyloxy)benzoyl]oxy]methyl]-6[[[[(4-hydroxy-6-methyl-3-pyridinyl)-carbonyl]-amino](4-hydroxy-phenyl)acetyl]amino-3-methyl-7-oxo-4-thia-1-azabicyclo[3.2.0]-heptane-2-carboxylic acid monosodium salt.

48. A process in accordance with claim 1 wherein the following compound is prepared:

[2S-[2α,3α,5α,6β(R*)]]-3-[[[3,4-bis(acetyloxy) benzoyl]oxy]methyl]-6-[[[[(4-hydroxy-6-methyl-3-pyridinyl)-carbonyl]amino](4-hydroxyphenyl)acetyl]amino-3-methyl-7-oxo-4-thia-1-azabicyclo[3.2.0]-heptane-2-carboxylic acid monosodium salt.

49. A process in accordance with claim 1 wherein the following compound is prepared: [2S-[2α,3α,5α,6β(R*)]]-3-[[[3,4-bis(acetyloxy)benzoyl]oxy]methyl]-6-[[[[(4-ethyl-2,3-dioxo-1-piperazinyl)-carbonyl]amino](4-hydroxyphenyl)acetyl]amino-3-methyl-7-oxo-4-thia-1-azabicyclo[3.2.0]-heptane-2-carboxylic acid monosodium salt.

50. A process in accordance with claim 1 wherein the following compound is prepared: [2S-[2α,3α,5α,6β(S*)]]-3-[[[3,4-bis(acetyloxy)benzoyl]oxy]methyl]-6-[[[[(4-ethyl-2,3-dioxo-1-piperazinyl) carbonyl]amino](4-hydroxyphenyl)acetyl]amino-3-methyl-7-oxo-4-thia-1-azabicyclo[3.2.0]-heptane-2-carboxylic acid monosodium salt.

51. A process in accordance with claim 1 wherein the following compound is prepared: [2S-[2α,3α,5α,6β(R,S)]]-3-[[[3,4-bis(acetyloxy)benzoyl]oxy]methyl]-6-[[[3,4-bis(acetyloxy)phenyl][[(4-ethyl-2,3-dioxo-1-piperazinyl)carbonyl]amino]acetyl]amino]-3-methyl-7-oxo-4-thia-1-azabicyclo[3.2.0]-heptane-2-carboxylic acid monosodium salt

52. A process for the manufacture of a pharmaceutical composition, particularly to be used in the control or prevention of infectious diseases, which comprises bringing a compound of formula I as defined in claim 1, a hydrate, a readily hydrolysable ester or salt thereof, or a hydrate of the ester or the salt, when R is hydrogen, into a galenical administration form together with a therapeutically inert carrier material.

53. The use of a compound of formula I as defined in claim 1, a hydrate, a readily hydrolysable ester or salt thereof, or a hydrate of the ester or the salt, when R is hydrogen for the manufacture of antibiotically active pharmaceutical compositions.

Claims for the following Contracting State: ES

1. A process for the manufacture of a compound of the general formula

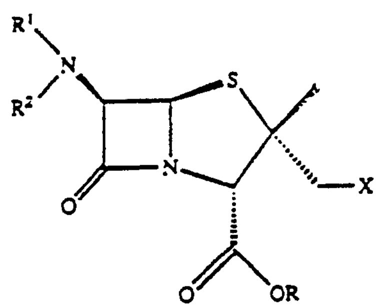

I

in which R is hydrogen, a readily hydrolyzable ester group or a protecting group, $R^1$ is hydrogen and $R^2$ is hydrogen or an acyl group, or $R^1$ and $R^2$ taken together are the group

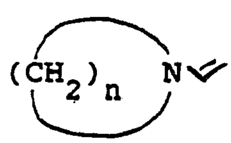

in which n is 5, 6 or 7,
X is fluoro, thiocyanato, azido or the group $R^{3a}CO_2$-

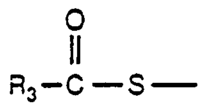 , 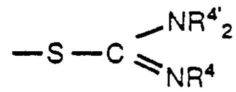 , $-SR^3$ , $-OR^3$

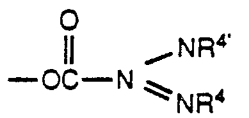 , 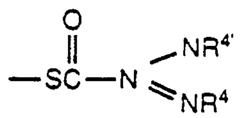 , 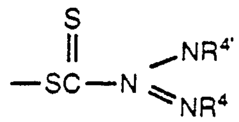 ,

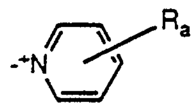 or 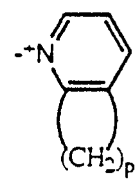 ,

$R^{3a}$ is $C_2$- to $C_{16}$-alkyl, aryl, a heterocycle, $-(CH_2)_m$-aryl, $-(CH_2)_m$-heterocycle, a heterocycle-lower alkenyl or aryl-lower alkenyl, $R^3$ is $C_1$- to $C_{16}$-alkyl, aryl, a heterocycle, $-(CH_2)_m$-aryl, a $-(CH_2)_m$-heterocycle, a heterocycle-lower alkenyl or aryl-lower alkenyl, $R^4$ and $R^{4'}$ independently are hydrogen, lower alkyl, lower alkenyl or lower alkynyl, $R_a$ is hydrogen, halogen, amino, amino-lower alkyl, carboxy, carbamoyl, carboxy-lower alkyl, carbamoyl-lower alkyl or lower carbalkoxy-lower alkyl, m is from 1 to 4 and p is 3, 4 or 5, provided that X is other than fluoro when R is a protecting group, a hydrate, a readily hydrolyzable ester or salt thereof, or a hydrate of the ester or the salt, when R is hydrogen, which process comprises,

a) reacting a compound of the general formula

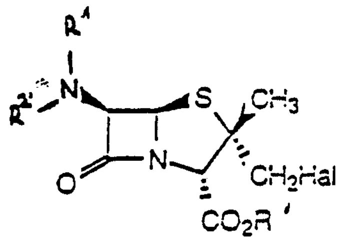

X

wherein Hal is halogen and $R'$ is a protecting group and $R^1$ and $R^2$ are as defined in claim 1, with a salt of a compound of the general formula HX, wherein X is as defined in claim 1, or

b) reacting a compound of the general formula

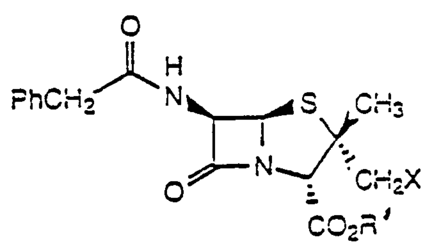

IV

wherein $R'$ is as defined above and X is as defined in claim 1, with $PCl_5$ or $PBr_5$ and pyridine or lutidine, or

c) reacting a compound of the general formula

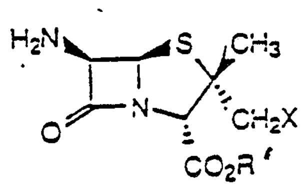

VI

wherein R′ is as defined above and X is as defined in claim 1, with a compound of the general formula

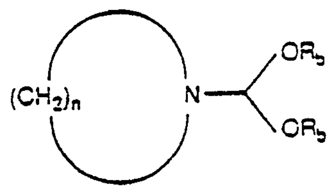

or

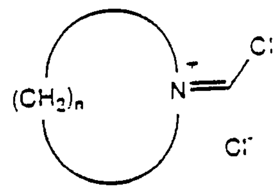

wherein n is as defined in claim 1 and Rb is lower alkyl, or

d) acylating a compound of the above general formula VI with a carboxylic acid or a reactive derivative thereof, and, if desired,

e) removing the ester protecting group in a compound obtained, wherein R is an ester protecting group, converting a compound obtained, wherein R is hydrogen, into a readily hydrolysable ester or a salt and/or converting a compound obtained, wherein R is hydrogen, or a readily hydrolysable ester or salt thereof into a hydrate.

2. A process according to claim 1, in which R is a protecting group.

3. A process according to claim 1, in which $R^1$ and $R^2$ are hydrogen.

4. A process according to claim 1, in which $R^1$ is hydrogen and $R^2$ is acyl.

5. A process according to claim 1, in which $R^1$ and $R^2$ taken together are the group

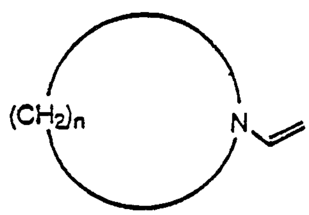

wherein n is 5, 6 or 7.

6. A process according to claim 4 wherein the acyl group $R^2$ is an aliphatic acyl group of the formula

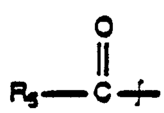

wherein $R_5$ is hydrogen, lower alkyl, lower cycloalkyl, lower alkoxy, lower alkenyl, lower cycloalkenyl, cyclohexadienyl or lower alkyl or lower alkenyl substituted with one or more halogen, cyano, nitro, amino, mercapto, alkylthio or cyanomethylthio groups.

7. A process according to claim 4 wherein the acyl group $R^2$ is an aromatic acyl group selected from the group consisting of

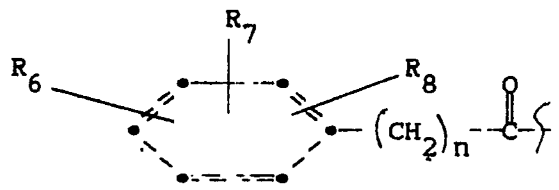

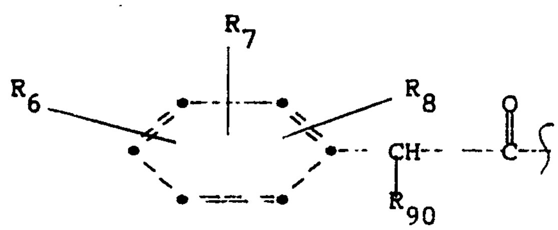

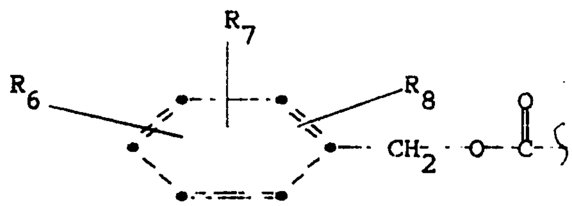

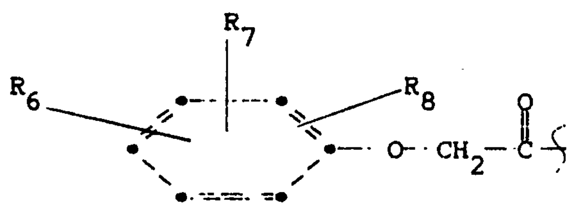

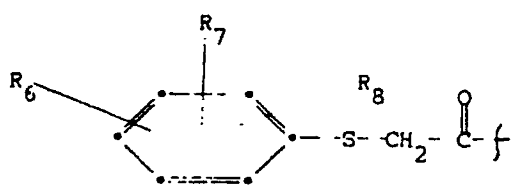

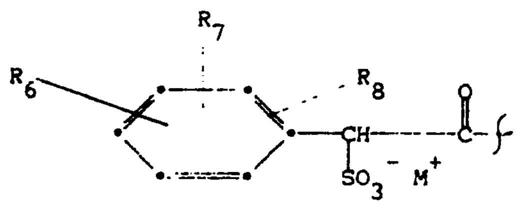

and

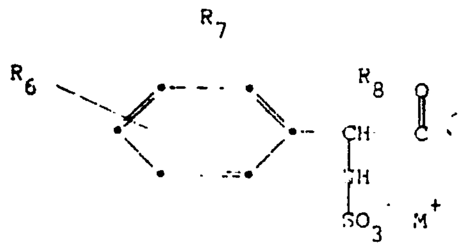

wherein n is 0, 1, 2 or 3, $R_6$, $R_7$ and $R_8$ are independently hydrogen, halogen, hydroxy, nitro, amino, cyano, trifluoromethyl, alkyl of 1 to 4 carbon atoms, alkoxy of 1 to 4 carbon atoms or aminomethyl and $R_{90}$ is amino, acylamino, hydroxy, a carboxy or sulfo salt, protected carboxy, formyloxy or azido.

8. A process according to claim 4 wherein the acyl group $R^2$ is a heteroaromatic acyl group selected from the group consisting of

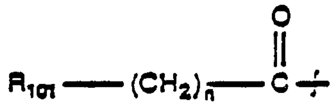

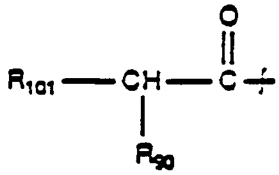

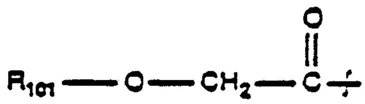

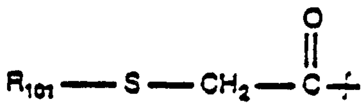

and

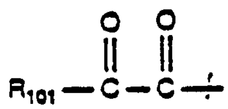

wherein n is 0, 1, 2 or 3, $R_{90}$ is as defined in claim 7 and $R^{101}$ is a substituted or unsubstituted 5-, 6- or 7-membered heterocyclic ring containing 1, 2, 3 or 4 hetero atoms selected from the group consisting of nitrogen, oxygen and sulfur.

9. A process according to claim 4 wherein the acyl group $R^2$ is a [[(4-substituted-2, 3-dioxo-1-piperazinyl) carbonyl]amino]acetyl group of the formula

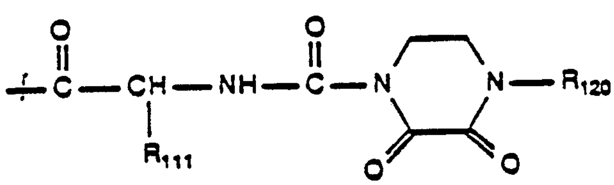

wherein $R_{111}$ is lower alkyl, lower hydroxyalkyl, an aromatic group of the formula

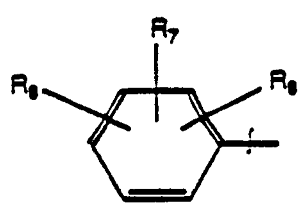

wherein $R_6$, $R_7$ and $R_8$ are as defined in claim 7, or a substituted or unsubstituted 5-, 6- or 7-membered heterocyclic ring containing 1, 2, 3 or 4 hetero atoms selected from the group consisting of nitrogen, oxygen and sulfur, and $R_{120}$ is lower alkyl or substituted lower alkyl (wherein the lower alkyl group is substituted with one or more halogen, cyano, nitro, amino or mercapto groups).

10. A process according to claim 4 wherein the acyl group $R^2$ is a (acylamino) acetyl group having the formula

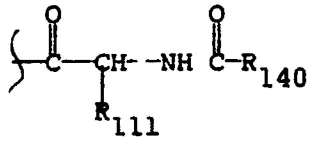

wherein $R_{111}$ is as defined in claim 9, and $R_{140}$ is an unsaturated heterocycle or a group of the formula

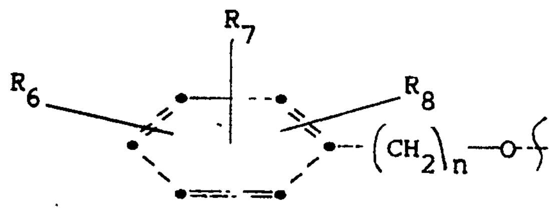

or

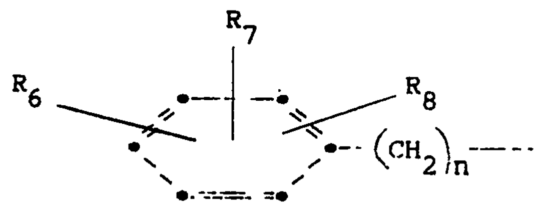

(wherein $R_6$, $R_7$ and $R_8$ are as defined in claim 7 and n is 0, 1, 2 or 3), hydrogen, lower alkyl, substituted lower alkyl, amino, lower alkylamino, di(loweralkyl)amino, (lower cyanoalkyl)amino, hydrazino, lower alkyl-hydrazino, aryl-hydrazino or acyl-hydrazino.

11. A process according to claim 4 wherein the acyl group $R^2$ is a (substituted oxyimino) acetyl group having the formula

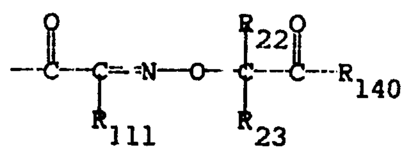

wherein $R_{111}$ is as defined in claim 9, $R_{140}$ is as defined in claim 10 and $R_{22}$ and $R_{23}$ are independently selected from the group consisting of hydrogen and lower alkyl, or $R_{22}$ and $R_{23}$ taken together with the carbon atom to which they are attached form a $C_3$-$C_7$-carbocyclic ring.

12. A process according to claim 4 wherein the acyl group $R^2$ is a [[[3-substituted-2-oxo-1-imidazolidinyl]carbonyl]amino]acetyl group of the formula

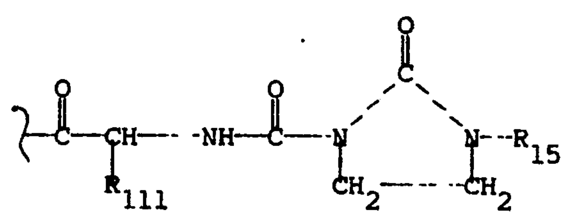

wherein $R_{111}$ is as defined in claim 9 and $R_{15}$ is hydrogen, lower alkylsulfonyl, arylmethyleneamino (i.e., $-N=CHR_{111}$ wherein $R_{111}$ is as defined in claim 9), $-COR_{16}$ (wherein $R_{16}$ is hydrogen, lower alkyl or halogen substituted lower alkyl), an aromatic group (as defined by $R_{111}$ in claim 9), lower alkyl or substituted lower alkyl (wherein the lower alkyl group is substituted with one or more halogen, cyano, nitro, amino or mercapto groups).

13. A process according to claim 4 wherein the acyl group $R^2$ is an acyl group of the formula

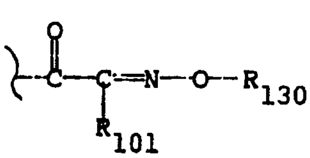

wherein $R_{101}$ is as defined in claim 8 and $R_{130}$ is hydrogen, lower alkyl, $C_3$-$C_7$-cycloalkyl or substituted lower alkyl [wherein the lower alkyl group is substituted with one or more halogen, cyano, nitro, amino, mercapto, lower alkylthio, aromatic groups (as defined by $R^{111}$ in claim 9), carboxy (including salts thereof), amido, carbamoyl, lower alkoxycarbonyl, phenylmethoxycarbonyl, diphenylmethoxycarbonyl, hydroxyalkoxyphosphinyl, dihydroxyphosphinyl, hydroxy(phenylmethoxy)phosphinyl or di(lower alkoxy)phosphinyl] or carboxy-$C_3$-$C_7$-cycloalkyl.

14. A process according to any one of claims 1-13, wherein X is $R^{3a}CO_2$-, $R^3$-COS-, -$SR^3$ or -$OR^3$ and $R^{3a}$ and $R^3$ independently are aryl or a heterocycle.

15. A process in accordance with claim 1 wherein the following compound is prepared: [2S-(2a,3a,5a,6β)]-3-Methyl-3-[[(1-methyl-1H-tetrazol-5-yl)thio]methyl-7-oxo-6-[(phenylacetyl)amino]-4-thia-1-azabicyclo[3.2.0]heptane-2-carboxylic acid monosodium salt.

16. A process in accordance with claim 1 wherein the following compound is prepared: [2S-(2a,3a,5a,6β)]-3-[[(2,5-Dihydro-6-hydroxy-2-methyl-5-oxo-1,2,4-triazine-3-yl)thio]-methyl]-3-methyl-7-oxo-6-[(phenylacetyl)amino]-4-thia-1-azabicyclo[3.2.0]heptane-2- carboxylic acid disodium salt.

17. A process in accordance with claim 1 wherein the following compound is prepared: [2S-(2α,3α,5α,6β)]-3-[(Acetylthio)methyl]-3-methyl-7-oxo-6-[(phenylacetyl)amino]-4-thia-1-azabicyclo[3.2.0]-heptane-2-carboxylic acid monosodium salt.

18. A process in accordance with claim 1 wherein the following compound is prepared: [2S-(2α,3α,5α,6β)]-3-[[(2,5-Dihydro-3-mercapto-2-methyl-5-oxo-1,2,4-triazine-6-yl)oxy]methyl]-3-methyl-7-oxo-6-[(phenylacetyl)amino]-4-thia-1-azabicyclo-[3.2.0]heptane-2-carboxylic acid monosodium salt.

19. A process in accordance with claim 1 wherein the following compound is prepared: [2S-(2α,3α,5α,6β)]-3-[[(2-Furanylcarbonyl)thio]methyl]-7-oxo-6-[(phenylacetyl)amino]-4-thia-1-azabicyclo-[3.2.0]-heptane-2-carboxylic acid monosodium salt.

20. A process in accordance with claim 1 wherein the following compound is prepared: [2S-(2α,3α,5α,6β)]-3-(Fluoromethyl)-3-methyl-7-oxo-6-[(phenyl-acetyl)amino]-4-thia-1-azabicyclo[3.2.0]-heptane-2-carboxylic acid sodium salt.

21. A process in accordance with claim 1 wherein the following compound is prepared: [2S-(2α,3α,5α,6β)]-3-[[[3,4-bis(Acetyloxy)benzoyl]thio]methyl]-3-methyl-7-oxo-6-[(phenylacetyl)amino]-4-thia-1-azabicyclo-[3.2-0]heptane-2-carboxylic acid sodium salt.

22. A process in accordance with claim 1 wherein the following compound is prepared: [2S-(2α,3α,5α,6β)]-3-[[3,4-Dihydroxybenzoyl)oxy]methyl]-3-methyl-7-oxo-6-[(phenylacetyl)amino]-4-thia-1-azabicyclo-[3.2.0]heptane-2-carboxylic acid monosodium salt.

23. A process in accordance with claim 1 wherein the following compound is prepared: [2S-(2α,3α,5α,6β)]-3-[[3-(1H-Imidazol-4-yl)-1-oxopropoxy)]-methyl]-3-methyl-7-oxo-6-[(phenylacetyl)amino]-4-thia-1-azabicyclo[3.2.0]heptane-2-carboxylic acid sodium salt.

24. A process in accordance with claim 1 wherein the following compound is prepared: [2S-(2α,3α,5α,6β)]-1-[[2-Carboxy-3-methyl-7-oxo-6[(phenylacetyl)amino]-4-thia-1-azabicyclo[3.2.0]hept-3-yl]-methyl pyridinium hydroxide inner salt.

25. A process in accordance with claim 1 wherein the following compound is prepared: [2S-(2α,3α,5α,6β)]-3-[[[3,4-bis(Acetyloxy)benzoyl]oxy]methyl]-3-methyl-7-oxo-6-[(phenylacetyl)amino]-4-thia-1-azabicyclo[3.2.0]heptane-2-carboxylic acid monosodium salt.

26. A process in accordance with claim 1 wherein the following compound is prepared: [2S-(2α,3α(E),5α,6β)]-3-[[[3-(1H-Imidazol-4-yl)-1-oxo-2-propenyl]oxy]methyl]-3-methyl-7-oxo-6-[-(phenylacetyl)amino]-4-thia-1-azabicyclo[3.2.0]heptane-2-carboxylic acid monosodium salt.

27. A process in accordance with claim 1 wherein the following compound is prepared: [2S-[2α,3α,5α,6β(Z)]]-6-[[(2-Amino-4-thiazolyl)(methoxyimino)-acetyl]amino]-3-methyl-3-[[(1-methyl-1H-tetrazol-5-yl)thio]methyl]-7-oxo-4-thia-1-azabicyclo[3.2.0]heptane-2-carboxylic acid monosodium salt.

28. A process in accordance with claim 1 wherein the following compound is prepared: [2S-[2α,3α,5α,6β(Z)]]-6-[[(2-Amino-4-thiazolyl)(methoxyimino)-acetyl]amino]-3-(azidomethyl)-3-methyl-7-oxo-4-thia-1-azabicyclo[3.2.0]heptane-2-carboxylic acid monosodium salt.

29. A process in accordance with claim 1 wherein the following compound is prepared: [2S-[2α,3α,5α,6β(Z)]]-3-[[[3,4-bis(Acetyloxy)benzoyl]-oxy]methyl]-6-[[(2-amino-4-thiazolyl)(methoxyimino)-acetyl]-amino]-3-methyl-7-oxo-4-thia-1-azabicyclo-[3.2.0]heptane-2-carboxylic acid monosodium salt.

30. A process in accordance with claim 1 wherein the following compound is prepared: [2S-[2α,3α,5α,6β(Z)]]-6-[[(2-Amino-4-thiazolyl)(methoxyimino)acetyl]amino]-3-[[(2,5-dihydro-6-hydroxy-2-methyl-5-oxo-1,2,4-triazine-3-yl)thio]methyl]-3-methyl-7-oxo-4-thia-1-azabicyclo[3.2.0]heptane-2-carboxylic acid disodium salt.

31. A process in accordance with claim 1 wherein the following compound is prepared:

[2S-[2α,3α,5α,6β(R*)]]-6-[[[[(4-Ethyl-2,3-dioxo-1-piperazinyl)carbonyl]amino]phenylacetyl]amino]-3-methyl-3-[[(1-methyl-1H-tetrazole-5-yl)thio]methyl]-7-oxo-4-thia-1-azabicyclo[3.2.0]heptane-2-carboxylic acid monosodium salt sesquihydrate.

32. A process in accordance with claim 1 wherein the following compound is prepared: [2S-[2α,3α,5α,6β(R*)]]-3-[[[3,4-bis(Acetyloxy)benzoyl]oxy]methyl]-6-[[[[(4-ethyl-2,3-dioxo-1-piperazinyl)-carbonyl]-amino]phenylacetyl]amino]-3-methyl-7-oxo-4-thia-1-azabicyclo-[3.2.0]-heptane-2-carboxylic acid monosodium salt.

33. A process in accordance with claim 1 wherein the following compound is prepared: [2S-[2α,3α,5α,6β(R*)]]-6-[[[[(4-Ethyl-2,3-dioxo-1-piperazinyl)-carbonyl]amino]-phenylacetyl]amino]-3-[[(3,4-dihydroxybenzoyl)oxy]methyl]-3-methyl-7-oxo-4-thia-1-azabicyclo[3.2.0]heptane-2-carboxylic acid monosodium salt.

34. A process in accordance with claim 1 wherein the following compound is prepared: [2S[2α,3α,5α,6β(R*)]]-6-[[[[(4-Ethyl-2,3-dioxo-1-piperazinyl)-carbonyl]amino]-phenylacetylamino]-3-[[(2-furanylcarbonyl)thio]methyl]-3-methyl-7-oxo-4-thia-1-azabicyclo[3.2.0]heptane-2-carboxylic acid sodium salt.

35. A process in accordance with claim 1 wherein the following compound is prepared: [2S-(2α,3α,5α,6β(R*)]]-6-[[[[(4-Ethyl-2,3-dioxo-1-piperazinyl)carbonyl]amino]phenylacetyl[amino]-3-[[(2,5-dihydro-6-hydroxy-2-methyl-5-oxo-1,2,4-triazine-3-yl)thio]methyl]-3-methyl-7-oxo-1-aza-4-thiabicyclo[3.2.0]-heptane-2-carboxylic acid disodium salt.

36. A process in accordance with claim 1 wherein the following compound is prepared: [2S-[2α,3α,5α,6β(R*)]]-3-[(Acetyl)thio)methyl]-3-methyl-6-[[[[4-ethyl-2,3-dioxo-1-piperazinyl)carbonyl]amino]-phenylacetyl]amino]-7-oxo-4-thia-1-azabicyclo[3.2.0]heptane-2-carboxylic acid monosodium salt.

37. A process in accordance with claim 1 wherein the following compound is prepared: [2S-[2α,3α,5α,6β(R*)]]-3-(Fluoromethyl)-6[[[[(4-ethyl-2,3-dioxo-1-piperazinyl)carbonyl]amino]phenylacetyl]-amino]-3-methyl-7-oxo-4-thia-1-azabicyclo[3.2.0]-heptane-2-carboxylic acid monosodium salt.

38. A process in accordance with claim 1 wherein the following compound is prepared: [2S-[2α,3α,5α,6β(R*)]]-3-[[[3,4-bis(Acetyloxy)benzoyl]thio]methyl]-6-[[[[(4-ethyl-2,3-dioxo-1-piperazinyl)-carbonyl]-amino]phenylacetyl]amino]-3-methyl-7-oxo-4-thia-1-azabicyclo-[3.2.0]-heptane-2-carboxylic acid monosodium salt.

39. A process in accordance with claim 1 wherein the following compound is prepared: [2S-[2α,3α,5α,6β(R*)]]-3-[[[3,4-bis(Acetyloxy)benzoyl]oxy]methyl-6-[amino(phenylacetyl)amino]-3-methyl-7-oxo-4-thia-1-azabicyclo[3.2.0]heptane-2-carboxylic acid monosodium salt.

40. A process in accordance with claim 1 wherein the following compound is prepared: [2S-[2α,3α,5α,6β(R*)]]-4-[[[2-Carboxy-6-[[[[(4-ethyl-2,3-dioxo-1-piperazinyl)carbonyl]amino]-phenylacetyl]-amino]-3-methyl-7-oxo-4-thia-1-azabicyclo[3.2.0]hept-3-yl]-thiol]methyl]-1-ethylpyridinium hydroxide inner salt.

41. A process in accordance with claim 1 wherein the following compound is prepared: [2S-[2α,3α,5α,6β)]-4-[[[2-Carboxy-3-methyl-7-oxo-6-[(phenylacetyl)amino]-4-thia-1-azabicyclo[3.2.0]hept-3-yl]methyl]thio]-1-ethylpyridinium hydroxide inner salt.

42. A process in accordance with claim 1 wherein the following compound is prepared: [2S-[2α,3α,5α,6β)]-3-[[[3,4-bis(Acetyloxy)benzoyl]oxy]methyl]-6-[[(hexahydro-1H-azepin-1-yl)methylene]-amino]-7-oxo-4-thia-1-azabicyclo[3.2.0]heptane-2-carboxylic acid.

43. A process in accordance with claim 1 wherein the following compound is prepared: [2S-[2α,3α,5α,6β)]-3-[[[3,4-bis(Acetyloxy)benzoyl]oxy]-methyl]-6-[[[(difluoromethyl)thio]acetyl]amino]-3-methyl7-oxo-4-thia-1-azabicyclo[3.2.0]heptane-2-carboxylic acid monosodium salt.

44. A process in accordance with claim 1 wherein the following compound is prepared: [2S-(2α,3α,5α,6β)]-6-[[[3,4-bis(Acetyloxy)benzoylamino]phenylacetyl]amino]-3-[[3,4-bis(acetyloxy)-benzoyloxy]methyl]-3-methyl-7-oxo-4-thia-1-azabicyclo-[3.2.0]heptane-2-carboxylic acid monosodium salt.

45. A process in accordance with claim 1 wherein the following compound is prepared: [2S-[2α,3α,5α,6β(Z)]]-1-[6-[[[(2-Amino-4-thiazolyl)-(methoxyimino)acetyl]-amino]-2-carboxy-3-methyl-7-oxo-4-thia-1-azabicyclo[3.2.0]hept-3-yl]methyl]pyridinium hydroxide inner salt.

46. A process in accordance with claim 1 wherein the following compound is prepared: [2S-[2α,3α,5α,6β(R*)]]-1-[[2-Carboxy-6-[[[[(4-ethyl-2,3-dioxo-1-piperazinyl)-carbonyl]amino]phenylacetyl]-amino]-3-methyl-7-oxo-4-thia-1-azabicyclo[3.2.0]-hept-3-yl]methyl]-pyridinium hydroxide inner salt.

47. A process in accordance with claim 1 wherein the following compound is prepared: [2S-[2α,3α,5α,6β(S*)]]-3-[[[3,4-bis(acetyloxy)benzoyl]oxy]methyl]-6[[[[(4-hydroxy-6-methyl-3-pyridinyl)-carbonyl]-amino](4-hydroxy-phenyl)acetyl]amino-3-methyl-7-oxo-4-thia-1-azabicyclo[3.2.0]-heptane-2-carboxylic acid monosodium salt.

48. A process in accordance with claim 1 wherein the following compound is prepared:

[2S-[2α,3α,5α,6β(R*)]]-3-[[[3,4-bis(acetyloxy) benzoyl]oxy]methyl]-6-[[[[(4-hydroxy-6-methyl-3-pyridinyl)-carbonyl]amino](4-hydroxyphenyl)acetyl]amino-3-methyl-7-oxo-4-thia-1-azabicyclo[3.2.0]-heptane-2-carboxylic acid monosodium salt.

49. A process in accordance with claim 1 wherein the following compound is prepared: [2S-[2α,3α,5α,6β(R*)]]-3-[[[3,4-bis(acetyloxy)benzoyl]oxy]methyl]-6-[[[[(4-ethyl-2,3-dioxo-1-piperazinyl)-carbonyl]amino](4-hydroxyphenyl)acetyl]amino-3-methyl-7-oxo4-thia-1-azabicyclo[3.2.0]-heptane-2-carboxylic acid monosodium salt.

50. A process in accordance with claim 1 wherein the following compound is prepared: [2S-[2α,3α,5α,6β(S*)]]-3-[[[3,4-bis(acetyloxy)benzoyl]oxy]methyl]-6-[[[[(4-ethyl-2,3-dioxo-1-piperazinyl) carbonyl]amino](4-hydroxyphenyl)acetyl]amino-3-methyl-7-oxo-4-thia-1-azabicyclo[3.2.0]-heptane-2-carboxylic acid monosodium salt.

51. A process in accordance with claim 1 wherein the following compound is prepared: [2S-[2α,3α,5α,6β(R,S)]]-3[[[3,4-bis(acetyloxy)benzoyl]oxy]methyl]-6-[[[3,4-bis(acetyloxy)phenyl][[(4-ethyl2,3-dioxo-1-piperazinyl)carbonyl]amino]acetyl]amino]3-methyl-7-oxo-4-thia-1-azabicyclo[3.2.0]-heptane-2-carboxylic acid monosodium salt

52. The use of a compound of formula I as defined in claim 1, a hydrate, a readily hydrolysable ester or salt thereof, or a hydrate of the ester or the salt, when R is hydrogen for the manufacture of antibiotically active pharmaceutical compositions.

European Patent Office

# EUROPEAN SEARCH REPORT

Application Number

EP 89 11 9941

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 287 734 (BEECHAM)<br>* Claims *<br>--- | 1-59 | C 07 D 499/84<br>A 61 K 31/43 |
| X | FR-A-2 217 340 (FUJISAWA)<br>* Claims, especially claims 17-20 *<br>--- | 1-51 | |
| X | US-A-4 183 850 (DARBY et al.)<br>* Whole document *<br>--- | 1-51 | |
| X | EP-A-0 206 000 (HOFFMANN-LA ROCHE)<br>* Claims 31-35 *<br>--- | 1-14 | |
| X | US-A-4 081 443 (SHOICHIRO UYEO)<br>* Whole document *<br>--- | 1-14 | |
| X | US-A-3 466 275 (R.B. MORIN)<br>* Whole document *<br>--- | 1-59 | |
| X | CHEMICAL ABSTRACTS, vol. 100, no. 11, 12th March 1984, page 526, abstract no. 85504y, Columbus, Ohio, US; & JP-A-58 154 587 (SAGAMI CHEMICAL RESEARCH CENTER) 14-09-1983<br>* Abstract *<br>--- | 1-51 | TECHNICAL FIELDS SEARCHED (Int. Cl.5)<br>C 07 D 499/00<br>A 61 K 31/00 |
| X | US-A-3 989 685 (TANIDA et al.)<br>* Column 27, example 18; column 35, example 9; claims *<br>--- | 1-14 | |
| A | US-A-4 464 237 (TOAII et al.)<br>* Whole document *<br>--- | 1 | |
| A | GB-A-2 017 705 (BRISTOL-MYERS)<br>* Claims *<br>----- | 1 | |

The present search report has been drawn up for all claims

| Place of search | Date of completion of the search | Examiner |
|---|---|---|
| THE HAGUE | 29-01-1990 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)